# EUROPEAN PATENT APPLICATION

(11) **EP 4 574 168 A1**
(43) Date of publication of application: **25.06.2025**
(21) Application number: 23220177.2
(22) Date of filing: 22.12.2023
(51) Int. Cl.: A61K 39/145, A61P 31/16, C12N 15/86, C12N 7/04

(54) **PRIME-BOOST IMMUNIZATION AGAINST INFLUENZA**

(71) Applicant: Institut für Virologie und Immunologie (IVI), 3147 Mittelhäusern (CH); Universität Bern, 3012 Bern (CH)
(72) Inventor: SUMMERFIELD, Artur, 3147 Mittelhäusern, (CH); AVANTHAY, Robin, 3147 Mittelhäusern (CH); ZIMMER, Gert, 3147 Mittelhäusern (CH)
(74) Representative: HGF

(57) **Abstract**

The invention relates to compositions and methods for immunisation against influenza virus.

## Description

### Field of Invention

The invention relates to compositions and methods for immunisation against influenza virus.

### Background to the Invention

In humans, influenza A virus (IAV) causes acute respiratory infections that are normally associated with sudden high fever, muscle pain, headache, coughing, and fatigue (França and Brown, 2014; Janke, 2014; Thangavel and Bouvier, 2014). These symptoms begin from one to four days after exposure to the virus and may last for about 2-8 days. However, IAV can cause life-threatening infections, in particular if the virus disseminates to the lower respiratory tract which could lead to viral pneumonia and acute respiratory distress syndrome (ARDS) (Herold et al., 2015; Kalil and Thomas, 2019; Rello and Pop-Vicas, 2009). Persons at high risk to suffer from severe influenza include the very young (< 2 years of age), the elderly (>65 years of age) or persons with underlying diseases such as diabetes, asthma, and cardiovascular diseases. IAV infections occur as seasonal epidemics which - in the Northern hemisphere - start in November and last until March. The cold season is associated with staying indoors, breathing dry air and having closer contacts, all factors that promote aerosol transmission which boost infection rates (Landier et al., 2021).

The most used human influenza vaccines to control seasonal epidemics represent inactivated viruses that are standardized according to their hemagglutinin (HA) content (Schotsaert and Garcia-Sastre, 2017; Wong and Webby, 2013). These vaccines are administered to persons prior to the influenza season by intramuscular injection. The immunization triggers the production of serum antibodies that predominantly bind to the HA globular head domain and exhibit virus-neutralizing activity (Lee et al., 2019; Wu and Wilson, 2020). A minor fraction of these virus-specific serum IgG is secreted into the lower respiratory tract where they provide protection from severe influenza pneumonia and ARDS (Epstein et al., 1997; Loosli et al., 1953). However, secretion of serum IgG into the mucosal tissues of the upper respiratory tract is not efficient (Barber and Small, 1978; Kris et al., 1988; Schmidt et al., 2001). Consequently, intramuscular immunization does not efficiently interfere with IAV replication in these tissues with the consequence that infectious virus is shed from the upper respiratory tract and potentially transmitted to other individuals.

Not only are those inactivated vaccines unable to interrupt the infectious chain during seasonal epidemics, they may also drive antigenic drift by selecting for viral escape mutants typically with mutations in the HA globular head domain (Archetti and Horsfall, 1950; Fonville et al., 2014; Smith et al., 2004). To compete with the constant antigenic drift of HA, influenza vaccines must be updated every year. However, as the corresponding seed viruses are usually selected six months in advance, there is a considerable risk of antigenic mismatch with the circulating IAV strains (Fiore et al., 2009; Trombetta et al., 2022). For some seasons, the efficacy of influenza vaccines can be relatively low, in particular in the elderly population (McLean and Belongia, 2021; Smetana et al., 2017).

In addition to inactivated vaccines, live-attenuated influenza vaccines (LAIV) have been approved (Mossad, 2003; Subbarao, 2021). These vaccines are based on cold-adapted viruses which harbor temperature-sensitive mutations in the genes that encode the viral RNA polymerase complex. Following nasal administration, these cold-adapted LAIV only replicate in the upper respiratory tract without disseminating to the lung. The high attenuation level of these vaccines limits LAIV transmission to contacts, but it also negatively impacts their immunogenicity (Subbarao, 2021). In addition, this vaccine is recommended for use in persons between 2 and 49 years of age by the Centers of Disease Control and Prevention (CDC). Thus, the elderly population is excluded from vaccination with these LAIV, although this population is particularly affected by severe influenza.

LAIV vaccines based on A/swine/Texas/4199-2/98 (H3N2) (sTX98) encoding a truncated NS1 protein were shown to protect pigs against challenge with the homologous virus (Richt et al., 2006; Vincent et al., 2007) and to even provide protection against certain H1N2 virus (Genzow et al., 2018; Kaiser et al., 2019; Sharma et al., 2020). Such a vaccine was approved for use in piglets of one day of age in the US in 2017 (Ingelvac Provenza, Boehringer Ingelheim) but had to be withdrawn from the market, following discovery of reassortant viruses with gene segments of the vaccine and field strains (Mancera Gracia et al., 2020; Sharma et al., 2020). A recent study showed that NS1-truncated sTX98 replicated for a similar time than wild-type virus (Vandoorn et al., 2022). These findings suggest that NS1-truncated LAIV are not sufficiently attenuated.

In our previous work, we generated recombinant LAIV candidates that were based on the pandemic A/Hamburg/4/2009 (H1N1) strain (pH1N1/09) (Avanthay et al., 2023). We compared a LAIV candidate encoding a C-terminally truncated NS1 protein (pH1N1/09-NS1 (1-126)) with the same virus that had additional mutations in the PA gene that prevented PA-X expression (pH1N1/09-NS1(1-126)-ΔPAX). The latter virus further enhanced innate immune responses, replicated less efficiently, and induced less apoptotic cell death in a swine bronchiolar epithelial cell. Based on these characteristics, we proposed that this virus could be a sufficiently attenuated LAIV candidate (Avanthay et al., 2023).

In the present study, these LAIVs were characterized and further developed as efficacious vaccines using pigs as animal model. While both LAIV were highly immunogenetic, they appeared to be insufficiently attenuated with respect to the height and duration of vaccine virus shedding. We therefore developed a novel heterologous prime/boost vaccination protocol, which was based on a first intramuscular immunization with a propagation-defective vesicular stomatitis virus (VSV) vector encoding the pH1N1/09 HA antigen and a secondary immunization using the LAIV candidates via the intranasal route. The selection of the VSV vector was based on our previous work demonstrating its efficacy in the induction of antibody and T cell responses against HA that mediate partial protection against heterologous IAV challenge of pigs in absence of neutralizing antibodies (Ricklin et al., 2016). Our results identified a highly efficient heterologous prime/boost vaccine inducing strong systemic and mucosal immune responses, resulting in sterilizing immunity in pigs.

### Summary of Invention

In accordance with a first aspect of the invention there is provided a combination of a priming composition and a boosting composition for priming and boosting an immune response in a subject comprising (1) a priming composition comprised of a propagation-defective RNA virus vector or self-replicating RNA or non-self-replicating RNA, and (2) a boosting composition comprising a Live Attenuated Influenza Vaccine and, whereby the immune response resulting from administration of the priming composition to the subject is capable of being boosted.

In a second aspect, the present invention provides a method for inhibiting a viral infection in a subject, comprising administering a prime-boost vaccination to the subject, wherein the prime-boost vaccination comprises administering a primer vaccine and a booster vaccine to the subject, wherein: the primer vaccine comprises a propagation-defective RNA virus vector or self-replicating RNA or non-self-replicating RNA and the booster vaccine comprises a Live Attenuated Influenza Vaccine; thereby inhibiting the viral infection in the subject.

In a third aspect, the present invention provides a method of generating an immune response in a subject, comprising (a) parenterally administering a first immunogenic composition comprising a propagation-defective RNA virus vector or self-replicating RNA or non-self-replicating RNA and; (b) administering a second immunogenic composition by the respiratory route comprising a Live Attenuated Influenza Vaccine, thereby inducing an immune response in the subject.

In a forth aspect, the present invention provides a method for vaccinating a subject comprising a first step of administering an effective amount of a propagation-defective RNA virus vector or self-replicating RNA or non-self-replicating RNA and a second step of administering an effective amount of a Live Attenuated Influenza Vaccine to the subject.

In a fifth aspect, the present invention provides the use of a prime-boost vaccination to inhibit influenza Virus infection in a subject, wherein the prime-boost vaccination comprises administering a primer vaccine and a booster vaccine to the subject, wherein: the primer vaccine comprises propagation-defective RNA virus vector or self-replicating RNA or non-self-replicating RNA; and the booster vaccine comprises a Live Attenuated Influenza Vaccine; thereby inhibiting the viral infection in the subject.

In a sixth aspect, the present invention provides a heterologous prime-boost vaccine for use in inducing an immune response in a mammal comprising: administering a primer vaccine and a booster vaccine to the subject, wherein: the primer vaccine comprises a propagation-defective RNA virus vector or self-replicating RNA or non-self-replicating RNA; and the booster vaccine comprises a Live Attenuated Influenza Vaccine; thereby inhibiting the viral infection in the subject.

In a seventh aspect, the present invention provides a heterologous prime-boost vaccine for use in immunization against influenza disease in a mammal comprising: administering a primer vaccine and a booster vaccine to the subject, wherein: the primer vaccine comprises a propagation-defective RNA virus vector or self-replicating RNA or non-self-replicating RNA; and the booster vaccine comprises a Live Attenuated Influenza Vaccine; thereby inhibiting the viral infection in the subject.

In an eight aspect, a kit for inhibiting influenza virus in a subject comprising a first container and a second container, wherein the first container comprises a primer vaccine comprising a propagation-defective RNA virus vector or self-replicating RNA or non-self-replicating RNA; and the second container comprises a Live Attenuated Influenza Vaccine; wherein the primer vaccine and the booster vaccine are formulated for use in a prime-booster vaccination to inhibit influenza virus in a subject; and instructions for using the kit.

### Brief Description of the Drawings

Exemplary arrangements of the disclosure shall now be described with reference to the drawings in which:

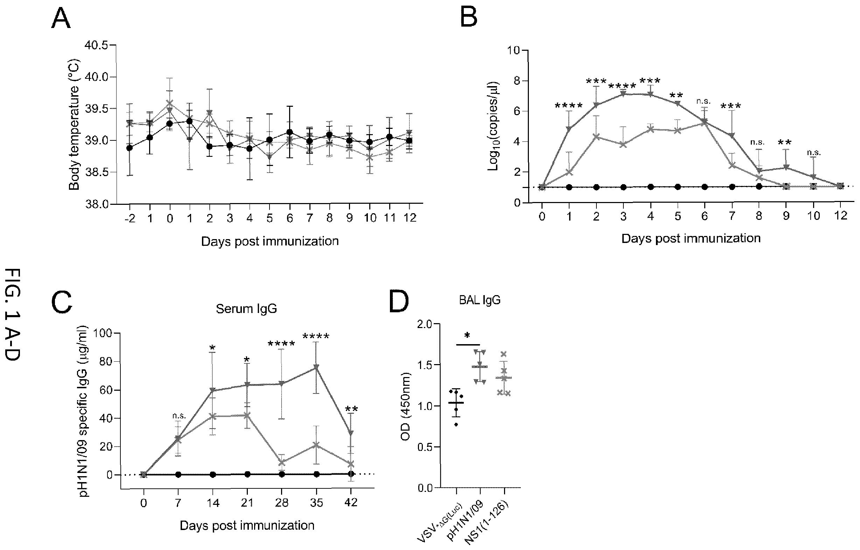

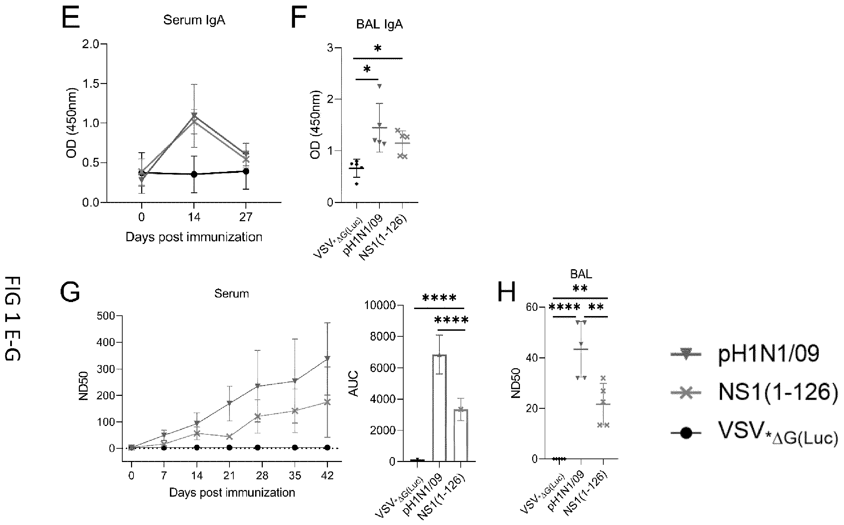

**Figure** 1. Replication and immunogenicity of pH1N1/09-NS1 (1-126) in pigs following intranasal application. Nasal swabs samples were collected daily and serum samples weekly. At day 42 the animals were euthanized, and BAL fluid was collected. (A) Daily rectal body temperatures. (B) Viral RNA quantities in oropharyngeal swab samples. (C, D) pH1N1/09-specific Ig in sera and (bonchoalveolar lavage) BAL fluids, respectively. (E, F) pH1N1/09-specific IgA in serum and in BAL fluids, respectively. (G) pH1N1/09-neutralizing antibody titres in the serum. The left panel depict average values at the individual days and the right panel the area under the curve (AUC) analyses encompassing all days. (H) pH1N1/09-neutralizing antibody titres in the BAL fluids. Statistical analyses employed two-way ANOVA tests for data in (B), (C) and (E); one-way ANOVA tests for (D), (F), (G, right panel) and (H). *p<0.05, **p<0.01, ***p<0.001, ****p<0.0001 indicate significant differences.

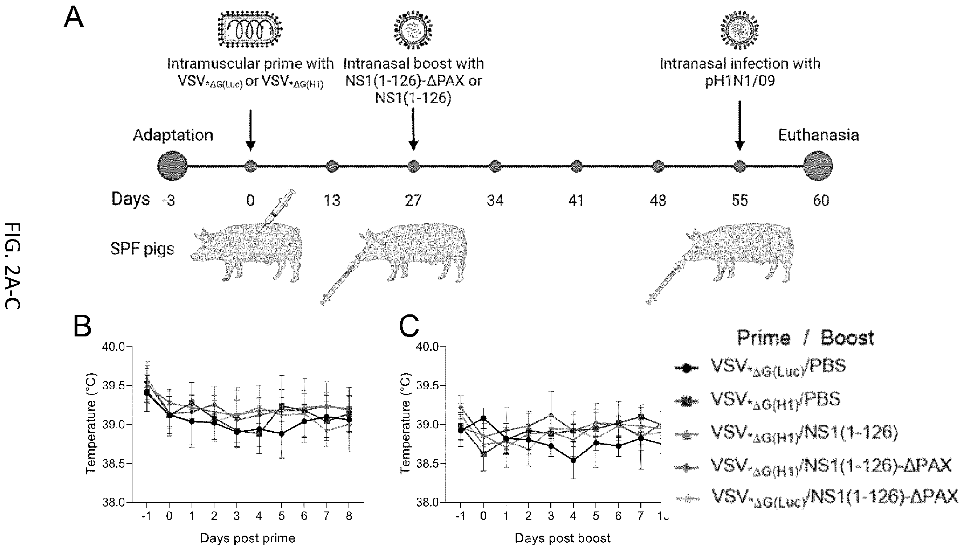

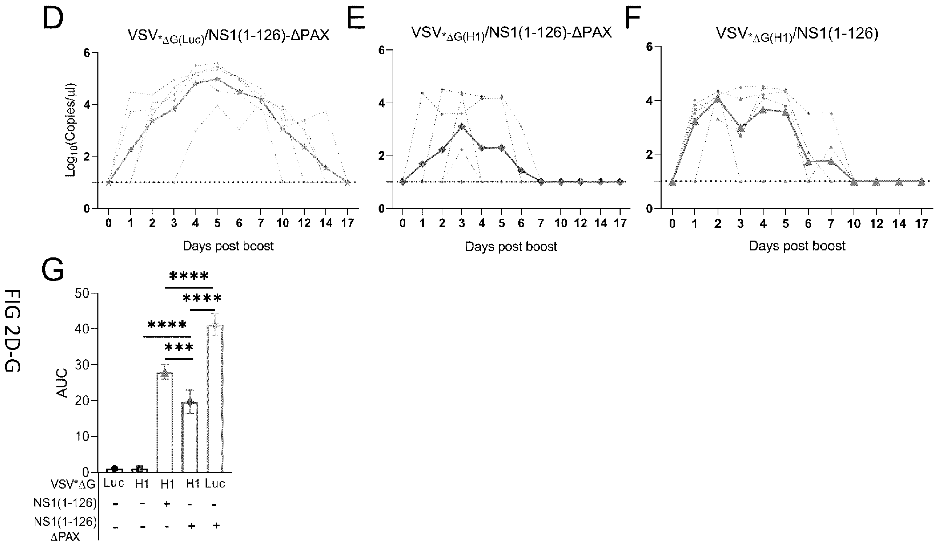

**Figure 2****.** Heterologous prime/boost vaccination protocols reduce LAIV shedding. (A) Schematic representation of the experiment design. Round points on the timeline indicate sampling days for blood (figure created on Biorender.com). (B) Body temperatures of pigs during the first eight days following primary immunization (i.m.). (C) Body temperature of pigs during the first 10 days after the subsequent intranasal LAIV immunization. (D-F) Viral RNA copies in oropharyngeal swab samples collected after the LAIV intranasal inoculation. Individual animals are represented by dashed lines and group mean values by continuous thick lines. In (D), the pigs were first immunized with negative control VSV*ΔG(Luc) followed by pH1N1/09-NS1(1-126)-ΔPAX; in (E) with VSV*ΔG(H1) followed by pH1N1/09-NS1(1-126)-ΔPAX, and in (F) with VSV*ΔG(H1) followed by pH1N1/09-NS1(1-126). (G) AUC analyses for viral RNA quantities from days 0 to 17 after LAIV application (calculated with data from D-F). Significant differences of the AUC values were determined using the one-way ANOVA test (*p<0.05, **p<0.01, ***p<0.001, ****p<0.0001).

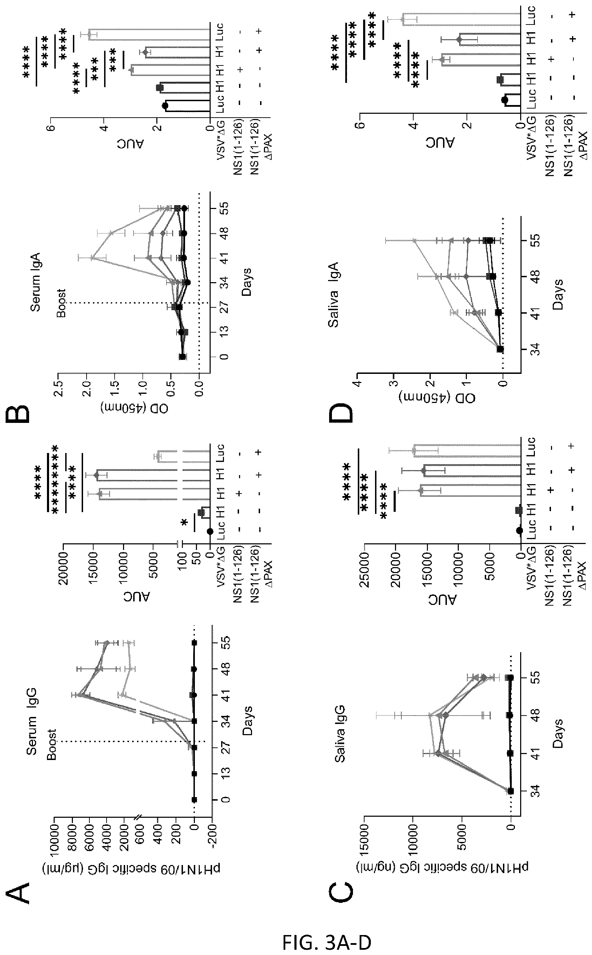

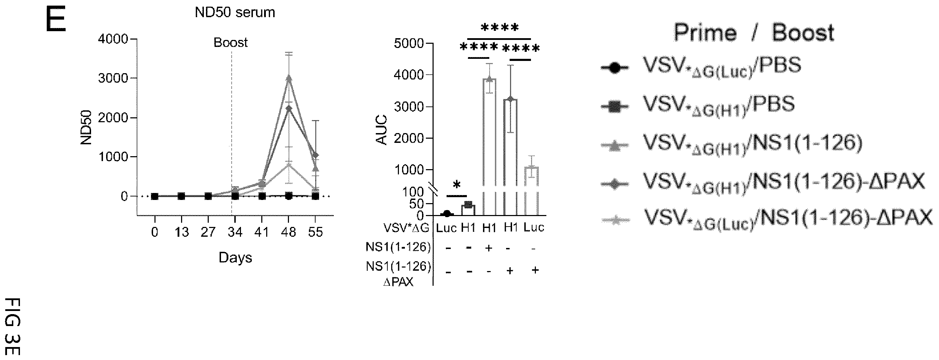

**Figure 3****.** Antibody responses following immunizations of pigs using heterologous prime/boost vaccination protocols. The experimental layout was as described in Fig 2A. In A-E the left panels show the kinetics of average antibody levels, whereas the right panels depict the AUC analyses of this data using all time points. (A, B) pH1N1/09-specific IgG or IgA in serum quantified by ELISA. (C, D) pH1N1/09-specific IgG or IgA in saliva quantified by ELISA. (E) Serum neutralizing antibody titres against homologous virus. Significant differences were determined using the one-way ANOVA test (*p<0.05, **p<0.01, ***p<0.001, ****p<0.0001).

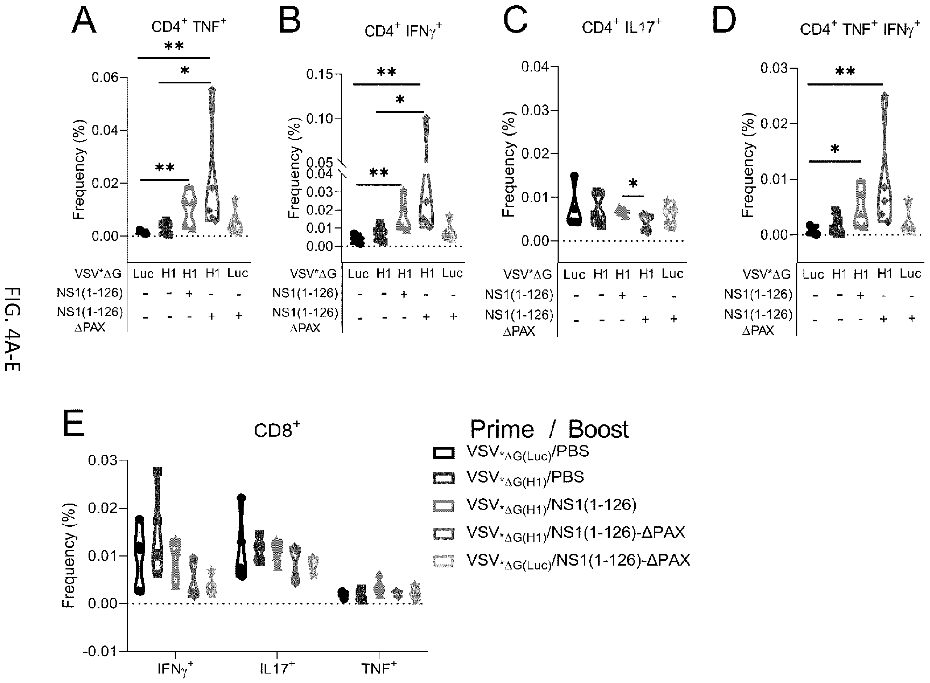

**Figure 4****.** Memory T-cell responses in the peripheral blood of pigs immunized using heterologous prime/boost protocols. PBMCs were isolated from immunized pigs at day 48 and restimulated with live pH1 N1/09 (see scheme in Fig. 2A). (A-D) Intracellular cytokine staining of CD4+ T cells for TNF (A), IFNγ (B), IL17 (C), and TNF/IFNγ double-positive cells (D). The frequency of cytokine-positive cells relative to the total number of CD4+ T cells is shown. (E) Frequency of reactivated CD8+ cells that were positive for the indicated cytokines. Significant differences were determined using Mann-Whitney tests (*p<0.05, **p<0.01, ***p<0.001, ****p<0.0001).

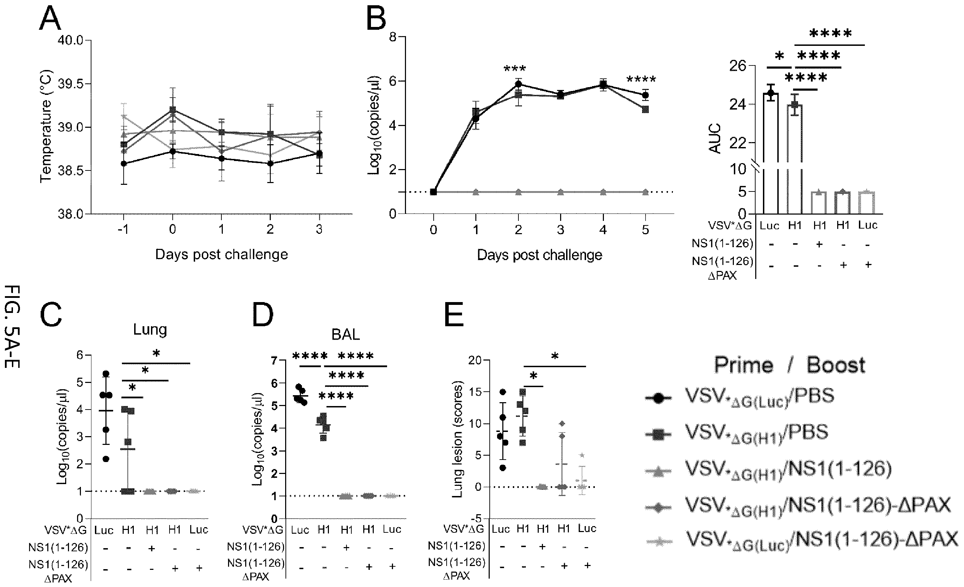

**Figure 5****.** Efficacy of vaccine candidates following challenge infection. Pigs were vaccinated and immunologically monitored as described in Fig. 2-4, and then challenged intranasally with pH1N1/09. (A) Body temperature after challenge infection. (B) Viral RNA loads in swab samples collected at all day's post infection (left panel), and AUC analyses of this data (right panel). (C, D) Viral RNA loads in lung tissue and BAL fluids collected at day 5 post infection. (E) Histopathological lung lesion scores. Significant differences were determined using the one-way ANOVA test (AUC values in B, C, and D), and Kruskal-Wallis test (E) *p<0.05, **p<0.01, ***p<0.001, ****p<0.0001 indicate significant differences.

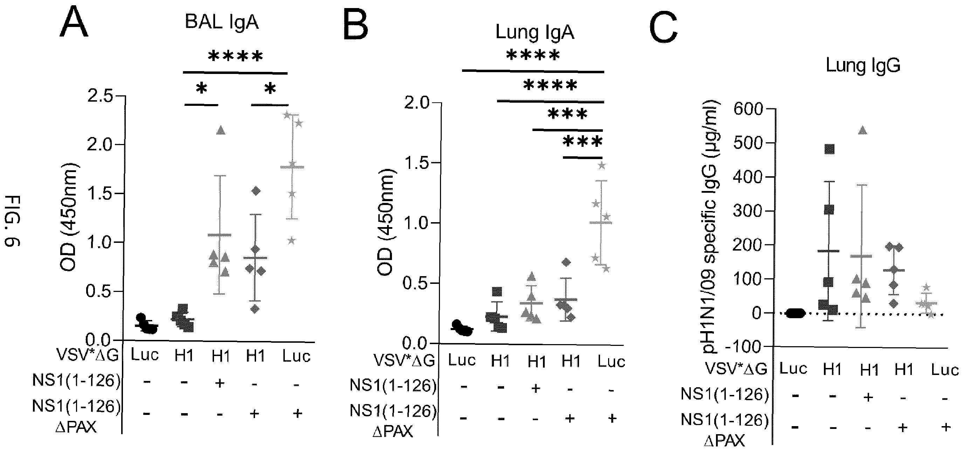

**Figure 6****.** Antibody responses of pigs after nasal challenge infection with pH1 N1/09. (A, B) pH1N1/09-specific IgA in BAL fluids and lung tissues 5 days post infection. (C) pH1N1/09-specific IgG in lung tissues. Significant differences were determined using the one-way ANOVA test. *p<0.05, **p<0.01, ***p<0.001, ****p<0.0001 indicate significant differences.

### Specific Description

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

For the avoidance of doubt, the information disclosed earlier in this specification under the heading "Background to the Invention" is relevant to the invention and is to be read as part of the disclosure of the invention.

Vaccination is one of the most effective methods for preventing infectious diseases. However, a single administration of an antigen is often not sufficient to confer optimal immunity and/or a long-lasting response. Approaches for establishing strong and lasting immunity to specific pathogens include repeated vaccination, i.e. boosting an immune response by administration of one or more further doses of antigen.

By "heterologous prime boost" is meant priming the immune response with an antigen and subsequent boosting of the immune response with the same antigen delivered by a different delivery system or route of administration. For example, heterologous prime boost regimens of the invention include priming by intramuscular administration and boosting with intranasal administration.

The term "primer composition", "primer vaccine," "priming immunization," or "primary immunization" refers to primary antigen stimulation by using a propagation-defective RNA virus vector or self-replicating RNA or non-self-replicating RNA according to the present invention. The mammal that receives the primer vaccine may or may not have already been exposed to the vector of the primer vaccine, and/or the pathogen against which the primer vaccine is designed, for instance by natural infection.

By "priming" is meant the administration of an immunogenic composition which induces a higher level of an immune response, when followed by a subsequent administration of the same or of a different immunogenic composition, than the immune response obtained by administration with a single immunogenic composition.

The term "booster composition", "booster vaccine," "boosting immunization," or "secondary immunization" refers to additional immunization administered to or effective in a mammal after the primary immunization. In various embodiments, the booster vaccine is administered at a dose higher than, lower than or equal to the effective dose that is normally administered when the booster vaccine is administered alone without priming. In certain advantageous embodiments, the booster vaccine is administered to the mammal at a dose lower than the effective dose that would be used when the immunization is administered to the mammal alone without priming. The booster vaccine refers to booster antigen stimulation by using a Live Attenuated Influenza Vaccine.

By "boosting" is meant the administration of a subsequent immunogenic composition after the administration of a priming immunogenic composition, wherein the subsequent administration produces a higher level of immune response than an immune response to a single administration of an immunogenic composition.

The prime and boost vaccine compositions may be administered via the same route or they may be administered via different routes. The boost vaccine composition may be administered one or several times at the same or different dosages. It is within the ability of one of ordinary skill in the art to optimize prime-boost combinations, including optimization of the timing and dose of vaccine administration.

Compositions disclosed herein will generally be administered directly to a subject. Direct delivery may be accomplished by parenteral administration, e.g. buccal, inhalation, intramuscular, intranasal, intraperitoneal, intrathecal, intravenous, oral, rectal, sublingual, transdermal, vaginal or to the interstitial space of a tissue. The priming immunogenic composition or the boosting immunogenic composition can be administered to the mammal by any known route of administration to one of ordinary skill in the field, including without limitation intratracheal, intramuscular and aerosol routes.

The term "vaccination" or "immunization" as used herein describes any kind of prophylactic or therapeutic immunization, whether administered after the disease has already been established to improve a clinical situation, or administered for the purpose of preventing a disease or infection from occurring. Therapeutic vaccination can prevent the development of a pathological condition and/or improve a clinical situation. When applied as a preventive agent, it will generally result in a protective immune response. A vaccine is an immunogenic composition that when administered to a subject, inhibits a disorder or disease, including prevention of the disease or disorder (such as a viral infection), or reduces the risk of the disease or disorder (such as the risk of contracting the viral infection). In one specific, non-limiting embodiment, a vaccine inhibits Influenza virus infection in a subject, by for example, preventing Influenza infection in the subject.

In a first aspect of the invention there is provided, a combination of a priming composition and a boosting composition for priming and boosting an immune response in a subject comprising (1) a priming composition comprised of a propagation-defective RNA virus vector or self-replicating RNA or non-self-replicating RNA, and (2) a boosting composition comprising a Live Attenuated Influenza Vaccine and, whereby the immune response resulting from administration of the priming composition to the subject is capable of being boosted.

In another aspect, the present invention provides a method for inhibiting a viral infection in a subject, comprising administering a prime-boost vaccination to the subject, wherein the prime-boost vaccination comprises administering a primer vaccine and a booster vaccine to the subject, wherein: the primer vaccine comprises a propagation-defective RNA virus vector or self-replicating RNA or non-self-replicating RNA and the booster vaccine comprises a Live Attenuated Influenza Vaccine; thereby inhibiting the viral infection in the subject.

In another aspect, the present invention provides a method of generating an immune response in a subject, comprising (a) parenterally administering a first immunogenic composition comprising a propagation-defective RNA virus vector or self-replicating RNA or non-self-replicating RNA and; (b) administering a second immunogenic composition by the respiratory route comprising a Live Attenuated Influenza Vaccine, thereby inducing an immune response in the subject.

In another aspect, the present invention provides a method for vaccinating a subject comprising a first step of administering an effective amount of a propagation-defective RNA virus vector or self-replicating RNA or non-self-replicating RNA and a second step of administering an effective amount of a Live Attenuated Influenza Vaccine to the subject.

In another aspect, the present invention provides the use of a prime-boost vaccination to inhibit influenza Virus infection in a subject, wherein the prime-boost vaccination comprises administering a primer vaccine and a booster vaccine to the subject, wherein: the primer vaccine comprises propagation-defective RNA virus vector or self-replicating RNA or non-self-replicating RNA; and the booster vaccine comprises a Live Attenuated Influenza Vaccine; thereby inhibiting the viral infection in the subject.

In another aspect, the present invention provides a heterologous prime-boost vaccine for use in inducing an immune response in a mammal comprising: administering a primer vaccine and a booster vaccine to the subject, wherein: the primer vaccine comprises a propagation-defective RNA virus vector or self-replicating RNA or non-self-replicating RNA; and the booster vaccine comprises a Live Attenuated Influenza Vaccine; thereby inhibiting the viral infection in the subject.

In another aspect, the present invention provides a heterologous prime-boost vaccine for use in immunization against influenza disease in a mammal comprising: administering a primer vaccine and a booster vaccine to the subject, wherein:
the primer vaccine comprises a propagation-defective RNA virus vector or self-replicating RNA or non-self-replicating RNA; and the booster vaccine comprises a Live Attenuated Influenza Vaccine; thereby inhibiting the viral infection in the subject.

In another aspect, the present invention provides a kit for inhibiting influenza virus in a subject comprising a first container and a second container, wherein the first container comprises a primer vaccine comprising a propagation-defective RNA virus vector or self-replicating RNA or non-self-replicating RNA; and the second container comprises a Live Attenuated Influenza Vaccine; wherein the primer vaccine and the booster vaccine are formulated for use in a prime-booster vaccination to inhibit influenza virus in a subject; and instructions for using the kit.

Prime boost compositions and methods of the invention generate strong and lasting immune responses without inducing significant, or in some cases, without inducing detectable anti-vector immunity in the recipient. Heterologous prime/boost compositions and methods of the invention provide a potent and effective vaccine strategy, with the possibility of re-administering the same vaccine antigen multiple times without inducing anti-vector immunity.

The immune response can confer protective immunity, in which the vaccinated subject is able to control an infection with the pathological organism against which the vaccination was performed. The subject that develops a protective immune response may develop only mild to moderate symptoms of the disease caused by the pathological organism or no symptoms at all. The immune response can also be therapeutic, alleviating or eliminating the subject's response to the pathological organism against which the vaccination was performed.

An "immune response" to an antigen or composition is the development in a subject of a humoral and/or a cellular immune response to an antigen present in the vaccine. For purposes of embodiments of the present invention, a "humoral immune response" refers to an immune response mediated by antibody molecules, including secretory (IgA) or IgG molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells. In addition, a chemokine response may be induced by various white blood or endothelial cells in response to an administered antigen.

Thus, an immunological response as used herein may be one that stimulates CTLs, and/or the production or activation of helper T-cells. The production of chemokines and/or cytokines may also be stimulated. The antigen of interest may also elicit an antibody-mediated immune response. Hence, an immunological response may include one or more of the following effects: the production of antibodies (e.g., IgA or IgG) by B-cells; and/or the activation of suppressor, cytotoxic, or helper T-cells and/or T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

In one embodiment, the invention may, further, serve the role of a prophylactic vaccine, wherein the host produces antibodies and/or CTL responses against influenza virus protein, which responses then preferably serve to neutralize influenza viruses by, for example, inhibiting influenza infection. Administration of the compounds of one embodiment of the invention as a prophylactic vaccine, therefore, would comprise administering to a host a concentration of compounds effective in raising an immune response which is sufficient to elicit antibody and/or CTL responses to influenza virus protein, and/or neutralize an influenza virus, by, for example, inhibiting the ability of the virus to infect cells. The exact concentration will depend upon the specific compound to be administered, but may be determined by using standard techniques for assaying the development of an immune response which are well known to those of ordinary skill in the art.

As used herein, the term "antigen" refers to a molecule containing one or more epitopes (e.g., linear, conformational or both) that will stimulate a host's immune system to make a humoral, i.e., B cell mediated antibody production, and/or cellular antigen-specific immunological response (i.e. T cell mediated immunity). An "epitope" is that portion of an antigen that determines its immunological specificity.

In one embodiment the propagation-defective RNA virus vector or self-replicating RNA or non-self-replicating RNA encodes influenza antigen. In a preferred embodiment, the influenza antigen includes HA and/or NA. In another embodiment, the propagation-defective RNA virus vector is propagation-defective vesicular stomatitis virus (VSV) vector. In another embodiment the propagation-defective RNA virus vector or self-replicating RNA or non-self-replicating RNA is a propagation-defective vesicular stomatitis virus (VSV) vector encoding either HA and/or NA antigen. In another embodiment the self-replicating RNA or non-self-replicating RNA is a synthetically produced RNA molecule encoding an antigen which is delivered to a mammal in a manner ensuring the production of the encoded antigen.

Self- replicating RNA or non-self-replicating RNA vaccines have been derived from genomic viral replicons that lack viral structural proteins and express a heterologous antigen in place of the viral structural proteins. These self-replicating, or self-amplifying, RNA molecules can be produced either synthetically or in packaging cell lines that permit the expression of a single-round of infectious particles carrying RNAs encoding the vaccine antigen. RNA amplification in the cytoplasm then produces multiple copies of antigen-encoding mRNAs and creates double stranded RNA intermediates, which are known to be potent stimulators of innate immunity, i.e., the non-specific defence mechanisms of the host that deploy rapidly against almost any microbe.

The term "RNA vaccine" encompasses all vaccines comprising the nucleic acid RNA and encode one or more nucleotide sequence encoding an antigen capable of inducing an immune response in a mammal.

In another embodiment the self-replicating RNA or non-self-replicating RNA is synthetically delivered, for example delivery using lipid nanoparticles (LNP), or using virus-like particles with viral envelope glycoproteins that have receptor-binding and membrane fusion activities.

"Self-amplifying RNA," "self-replicating RNA" and "RNA replicon" are used interchangeably to mean RNA with the ability to replicate itself. They can be derived from either negative-sense (-) RNA or plus-sense (+) RNA viruses. The term "self-replicating RNA" refers to a self-amplifying RNA encoding an RNA-dependent RNA polymerase capable of intracellular replication and transcription of the encoding RNA molecule . The self-amplifying RNA vectors of the invention comprise genes encoding one or more antigens. These genes can replace nucleic acid sequences encoding structural proteins required for the production of infectious virus. For example, the propagation-defective, G gene-deleted vesicular stomatitis virus (VSV) is a negative-sense RNA virus vector, which can be produced by transfection of VSV G protein-expressing cells with a set of DNA plasmids encoding the viral N, P, and L genes under control of the T7 promoter plus a plasmid encoding the modified viral genome (in plus-sense orientation, encoding one or more vaccine antigens) under control of the T7 promoter. These VSV-vectored vaccines can be produced on packaging cell lines providing the deleted G protein in trans. The resulting G protein complemented "RNA replicon particles" can efficiently infect a variety of cell types of different species by virtue of the G protein that has receptor-binding and membrane fusion activities. After immunization with the propagation-defective RNA virus vector of the invention, replication and amplification of the RNA molecule occur in the cytoplasm of the infected or transfected cell and the nucleic acid is not integrated into the genome. As the RNA does not integrate into the genome and transform the target cell, self-amplifying RNA vaccines do not pose the safety hurdles faced by some recombinant DNA vaccines. Due to the absence of the genetic information of the essential G protein, the infected cells are unable to produce infectious progeny viruses.

Synthetically delivered self-replicating RNA molecules or non-self-replicating RNA are known in the art and can be produced by using replication elements derived from, e.g., alphaviruses, and substituting structural viral proteins with a nucleotide sequence encoding a protein of interest. A synthetically delivered self-replicating RNA molecule typically is in plus-sense orientation and can be directly translated into protein after delivery to a cell. This translation provides an RNA-dependent RNA polymerase which then produces both antisense and sense transcripts from the delivered RNA. Thus, the delivered RNA leads to the production of multiple daughter RNAs. These daughter RNAs, as well as collinear subgenomic transcripts, may be translated themselves to provide in situ expression of an encoded antigen or may be transcribed to provide further transcripts with the same sense as the delivered RNA, which are then translated to provide in situ expression of the antigen.

A synthetically delivered self-replicating RNA molecule or non-self-replicating RNA useful with the invention may have one or more open reading frames, leading to expression of at least a replicase and the antigen of interest. Alternatively or additionally, the RNA may have one or more additional (e.g. downstream) open reading frames, e.g. to encode further antigen(s) or to encode accessory polypeptides.

The synthetically delivered self-replicating RNA or non-self-replicating RNA can conveniently be prepared by in vitro transcription (IVT). IVT can use a cDNA template created and propagated in plasmid form in bacteria, or created synthetically, for example by gene synthesis and/or polymerase chain-reaction (PCR) engineering methods.

The synthetically delivered self-replicating RNA or non-self-replicating RNA can include, alternatively or in addition to any 5' cap structure, one or more nucleotides having a modified nucleobase. An RNA used with the invention preferably includes only phosphodiester linkages between nucleosides, but in some embodiments, it can contain phosphoramidate, phosphorothioate, and/or methylphosphonate linkages.

The self-replicating RNA molecules or non-self-replicating RNA described herein may be engineered to express multiple nucleotide sequences, from one or more open reading frames, allowing co-expression of proteins, such as one, two or more antigens.

In an embodiment, a self-replicating RNA or non-self-replicating RNA is produced synthetically. In another embodiment, this self-replicating RNA or non-self-replicating RNA is produced by in vitro transcription.

The term "propagation-defective" or "replication-incompetent" RNA virus vector refers to a virus that is incapable of replication because it has been engineered to comprise at least a functional deletion (or "loss-of-function" mutation), i.e. a deletion or mutation which impairs the function of a gene without removing it entirely, e.g. introduction of artificial stop codons, deletion or mutation of active sites or interaction domains, mutation or deletion of a regulatory sequence of a gene etc., or a complete removal of a gene encoding a gene product that is essential for viral replication. Such a virus vector is able to infect a cell resulting in replication and translation of its RNA but cannot form new virus particles able to further spread and infect other cells.

The composition of the present invention may be a pharmaceutical composition, e.g., an immunogenic composition or a vaccine composition. Accordingly, the composition may also comprise a pharmaceutically acceptable carrier. A "pharmaceutically acceptable carrier" includes any carrier that does not itself induce the production of antibodies harmful to the individual receiving the composition. The compositions of the invention may also contain a pharmaceutically acceptable diluent, such as water, sterile pyrogen-free water, saline, phosphate-buffered physiologic saline, glycerol, etc. Additionally, auxiliary substances, such as wetting or emulsifying agents, pH buffering substances, and the like, may be present.

Pharmaceutical compositions may include the constructs, nucleic acid sequences, and/or polypeptide sequences described elsewhere herein in plain water (e.g. water for injection (w.f.i.)) or in a buffer e.g. a phosphate buffer, a Tris buffer, a borate buffer, a succinate buffer, a histidine buffer, or a citrate buffer. Buffer salts will typically be included in the 5-20 mM range. Pharmaceutical compositions may have a pH between 5.0 and 9.5. Compositions may include sodium salts, e.g. sodium chloride, to give tonicity. A concentration of 10±2 mg/ml NaCl is typical, e.g. about 9 mg/ml. Compositions may include metal ion chelators. These can prolong RNA stability by removing ions which can accelerate phosphodiester hydrolysis and contribute to adenovector vector stability. Thus, a composition may include one or more of EDTA, EGTA, BAPTA, pentetic acid, etc. Such chelators are typically present at between 10-500 µM, e.g., 0.1 mM. A citrate salt, such as sodium citrate, can also act as a chelator, while advantageously also providing buffering activity.

A composition of the invention may be administered without an adjuvant. A composition which in un-adjuvanted, i.e. does not contain any exogeneous adjuvant. Alternatively or additionally, the composition is administered with an adjuvant. By "adjuvant" is meant an agent that augments, stimulates, activates, potentiates or modulates the immune response to an active ingredient of the composition. The adjuvant effect may occur at the cellular or humoral level or both. Adjuvants stimulate the response of the immune system to the actual antigen but have no immunological effect themselves. Alternatively or additionally, adjuvanted compositions of the invention may comprise one or more immunostimulants. By "immunostimulant" it is meant an agent that induces a general, temporary increase in a subject's immune response, whether administered with the antigen or separately.

In another embodiment, there is no adjuvant used in the present invention.

In an embodiment, the one or more antigens from the same pathogenic organism are the same in the priming vaccine as in the boosting vaccine. In a yet further embodiment, at least one of the antigens from the same pathogenic organism are different in the priming vaccine and the boosting vaccine.

In any of the embodiments described herein, the immune response can be directed to an infectious organism, e.g., a virus, bacteria or fungus.

Methods are provided for inducing an immune response against a pathogenic organism in a subject in need thereof comprising a step of administering an immunologically effective amount of a construct or composition as disclosed herein. Some embodiments provide the use of the constructs or compositions disclosed herein for inducing an immune response to an antigen in a subject in need thereof. Some embodiments provide the use of the construct or composition as disclosed herein in the manufacture of a medicament inducing an immune response to an antigen in a subject.

By "subject" is meant a mammal, e.g. a human or a veterinary mammal. In some embodiments the subject is human. In another embodiment, the subject or mammal is a human or animal, wherein the animal is a pig, horses, dog, mink, or fox.

Preferably the target population is a population which is unprimed against influenza, either being naive (such as vis a vis a pandemic strain), or having failed to respond previously to influenza infection or vaccination. Preferably the target population is elderly persons suitably aged at least 50, typically at least 55, or at least 60, or 65 years and over, younger high-risk adults (i.e. between 18 and 64 years of age) such as people working in health institutions, or those young adults with a risk factor such as cardiovascular and pulmonary disease, or diabetes. Another target population is all children 6 months of age and over, especially children 6-23 months of age who experience a relatively high influenza-related hospitalization rate. Preferably the target population is elderly above 65 years of age.

The Live Attenuated Influenza Vaccine is an attenuated live vaccine, unlike other influenza vaccines, which are inactivated vaccines. The live attenuated vaccine is based on a flu strain that does not cause disease, because it lacks the ability to efficiently suppress the hosts innate immune response. In one embodiment the infection of a host with the live-attenuated vaccine results in the secretion of cytokines such as type I and type III interferons which induce an antiviral state in the host and thereby limit virus replication and dissemination. Influenza virus gene segments that code for surface proteins (targeted antigens) are combined with the modified viral genome backbone. The recombinant viruses are generated by transfection of human cell lines with the well-known 8-plasmid system driving the transcription of the eight viral negative-sense RNA segments and of the positive-sense messenger RNA encoding the influenza proteins. The resulting viruses are then propagated on established cell lines such as Madin-Darby canine kidney (MOCK) cells or 10-days old embryonated chicken eggs. To make the refrigerated version, the produced virus is purified by centrifugation through a sucrose gradient, then suspended in a buffered (pH-7.0 to pH-7.4) salt solutions containing or not containing stabilizing molecules, and stored frozen at -70°C or at lower temperatures.

In one embodiment the Live Attenuated Influenza Vaccine comprises at least one mutation in the nonstructural 1 (NS1) protein and/or PA-X protein. In another embodiment wherein the at least one mutation in the nonstructural 1 (NS1) protein and/or PA-X protein causes a loss of function of the NS1 and/or PA-X protein. In a preferred embodiment the at least one mutation comprises an amino acid substitution, insertion or deletion. In another embodiment the Live Attenuated Influenza Vaccine comprises NS1(1-126) encoded a truncated NS1 protein lacking 93 amino acids at the C-terminus, or NS1(1-126)-ΔPAX combining both NS1 truncation and PA-X deletion.

In an embodiment, at least one of the priming and boosting immunogenic compositions is administered by a route selected from buccal, inhalation, intramuscular, intranasal, intraperitoneal, intrathecal, intravenous, oral, rectal, sublingual, transdermal, vaginal or to the interstitial space of a tissue. In one embodiment, the administering a booster composition by the respiratory route is administered intranasally.

Influenza A viruses are classified into serologically-defined antigenic subtypes of the HA and NA major surface glycoproteins. In another embodiment, the influenza virus strain is selected from the group consisting of: H5N1, H9N2, H7N7, H2N2 and H1N1, and H5H2. In another embodiment, the influenza virus strain is selected from the group consisting of: H1, H2, H3, H5, H7 and H9. A seasonal influenza vaccine refers to the vaccine of the present invention that is developed for a flu season. A seasonal influenza vaccine may include a group 1 influenza A strain, a group 2 influenza A strain, and an influenza B strain. Typically, a seasonal influenza vaccine may include H1N1 influenza A strain, a H3N2 influenza A strain, and at least one influenza B strain. Pandemic influenza vaccines may contain other HA (H1-H16) and NA (N1-N9) subtypes which are derived from avian influenza viruses. Pandemic vaccines may also contain HA antigens (H1-H3) and NA antigens (N1-N2) that are derived from porcine influenza viruses. Group 1 influenza A strains include those strains having a H1, H2, H5, H7 or H9 HA subtype. Group 2 influenza A strains include those strains having a H3, H4, H6, H8, H10, H11, H12, H13, H14, H15 or H16 HA subtype. In one embodiment, nucleic acids encoding HA of subtype H1 and H3 and NA antigens of subtypes N1 and N2 for the generation of seasonal influenza vaccines are used. In another embodiment, the HA and/or NA antigens derived from avian or porcine influenza are used. The antigens will be selected according to the actual epidemiological situation.

The dose and perhaps prime-boost regimen, will also vary according to the age, weight, and response of the individual subject.

In preferred embodiment the primer vaccine comprises nucleic acid sequence ID NO: 1

In preferred embodiment the primer vaccine comprises amino acid sequence ID NO: 4.

In preferred embodiment the booster vaccine comprises nucleic acid sequence ID NO: 2 or 3.

In preferred embodiment the booster vaccine comprises amino acid sequence ID NO: 5 or 6.

In another embodiment the primer vaccine comprises an amino acid sequence or nucleic acid sequence according to any of SEQ ID Nos. 1 or 4 or a variant thereof. In a preferred embodiment the primer vaccine comprises greater than 90%, 85%, 75%, 70%, 65% sequence identity to an amino acid sequence or nucleic acid sequence according to any of SEQ ID Nos. 1 or 4 or a variant thereof. In a preferred embodiment the primer vaccine comprises having at least 80%, 85%, 90%, 95%, 97%, 99%, or 100% sequence identity to SEQ ID NO: 1 or 4 or variant thereof.

In another embodiment the booster vaccine comprises an amino acid sequence or nucleic acid sequence according to any of SEQ ID Nos. 2, 3, 5 or 6 or a variant thereof. In a preferred embodiment the booster vaccine comprises greater than 90%, 85%, 75%, 70%, 65% sequence identity to an amino acid sequence or nucleic acid sequence according to any of SEQ ID Nos. 2, 3, 5 or 6 or a variant thereof. In a preferred embodiment the booster vaccine comprises having at least 80%, 85%, 90%, 95%, 97%, 99%, or 100% sequence identity to SEQ ID NO: 2, 3, 5 or 6 or variant thereof.

In one embodiment, a nucleic acid molecule encoding any influenza A HA is used. Such an HA can be a known HA or an HA of an influenza virus that is evolving. In one embodiment, a nucleic acid molecule encoding a group 1 HA is used. In one embodiment, a nucleic acid molecule encoding a group 2 HA is used. In one embodiment, a nucleic acid molecule encoding a H1 HA is used. In one embodiment, a nucleic acid molecule encoding a H3 HA is used. In one embodiment, a nucleic acid molecule encoding a H5 HA is used. In one embodiment, a nucleic acid molecule encoding a H2 HA is used. In one embodiment, a nucleic acid encoding a H7 HA is used. In one embodiment, a nucleic acid molecule encoding a H9 HA is used.

In one embodiment a nucleic acid molecule encoding an influenza B HA is used. In one embodiment a nucleic acid molecule encoding an influenza C HA is used. The invention also includes the use of a nucleic acid molecule encoding one or more other influenza HAs.

The term "nucleic acid" means a polymeric form of nucleotides of any length, which contain deoxyribonucleotides, ribonucleotides, and/or their analogs. It includes DNA, RNA and DNA/RNA hybrids. It also includes DNA or RNA analogs, such as those containing modified backbones (e.g. peptide nucleic acids (PNAs) or phosphorothioates) or modified bases. Thus, the nucleic acid of the disclosure includes mRNA, DNA, cDNA, recombinant nucleic acids, branched nucleic acids, plasmids, vectors, etc. Where the nucleic acid takes the form of RNA, it may or may not have a 5' cap.

The present inventors disclose herein nucleic acids comprising one or more nucleic acid sequence which encodes an antigen. A nucleic acid, as disclosed herein, can take various forms (e.g. single-stranded, double-stranded, vector, etc.). Nucleic acids may be circular or branched, but will typically be linear.

The nucleic acids used herein are preferably provided in purified or substantially purified form i.e., substantially free from other nucleic acids (e.g. free from naturally-occurring nucleic acids), particularly from host cell nucleic acids, typically being at least about 50% pure (by weight), and usually at least about 90% pure.

Nucleic acids may be prepared in many ways e.g., by chemical synthesis in whole or in part, by digesting longer nucleic acids using nucleases (e.g., restriction enzymes), by joining shorter nucleic acids or nucleotides (e.g., using ligases or polymerases) and from genomic or cDNA libraries.

The nucleic acids herein comprise a sequence which encodes at least one antigen. Typically, the nucleic acids of the invention will be in recombinant form, i.e., a form which does not occur in nature. For example, the nucleic acid may comprise one or more heterologous nucleic acid sequences (e.g., a sequence encoding another antigen and/or a control sequence such as a promoter) in addition to the sequence encoding the antigen. The nucleic acid may be part of a vector, i.e., part of a nucleic acid construct designed for transduction/transfection of one or more cell types. Vectors may be, for example, expression vectors which are designed to express a nucleotide sequence in a host cell, or viral vectors which are designed to result in the production of a recombinant virus or virus-like particle.

Alternatively, or in addition, the sequence or chemical structure of the nucleic acid may be modified compared to a naturally-occurring sequence which encodes an antigen. The sequence of the nucleic acid molecule may be modified, e.g. to increase the efficacy of expression or replication of the nucleic acid, or to provide additional stability or resistance to degradation. Alternatively or additionally, a vaccine construct of the invention is resistant to RNAse digestion in an in vitro assay.

The nucleic acid encoding the polypeptides described above may be modified to increase or decrease translation efficacy and/or half-life. For example, the nucleic acid may be codon optimized or deoptimized.

The nucleic acids may comprise one or more nucleotide analogs or modified nucleotides. As used herein, "nucleotide analog" or "modified nucleotide" refers to a nucleotide that contains one or more chemical modifications (e.g., substitutions) in or on the nitrogenous base of the nucleoside (e.g., cytosine (C), thymine (T), uracil (U), adenine (A) or guanine (G)). A nucleotide analog can contain further chemical modifications in or on the sugar moiety of the nucleoside (e.g., ribose, deoxyribose, modified ribose, modified deoxyribose, six-membered sugar analog, or open-chain sugar analog), or in or on the phosphate moiety. Many modified nucleosides and modified nucleotides are commercially available.

Nucleic acids of the invention may, for example, be an RNA-based vaccine. The RNA-based vaccine may comprise a self-amplifying RNA molecule. The self-amplifying RNA molecule may be a propagation-defective vesicular stomatitis virus vector or an pestivirus alphavirus-derived synthetically-delivered RNA replicon.

By "polypeptide" is meant a plurality of covalently linked amino acid residues defining a sequence and linked by amide bonds. The term is used interchangeably with "peptide" and "protein" and is not limited to a minimum length of the polypeptide. The term polypeptide also embraces post-translational modifications introduced by chemical or enzyme-catalyzed reactions, as are known in the art. The term can refer to fragments of a polypeptide or variants of a polypeptide such as additions, deletions or substitutions.

Alternatively or additionally, a polypeptide herein is in a non-naturally occurring form (e.g. a recombinant or modified form). Polypeptides of the invention may have covalent modifications at the C-terminus and/or N-terminus. They can also take various forms (e.g. native, fusions, glycosylated, non-glycosylated, lipidated, non-lipidated, phosphorylated, non-phosphorylated, myristoylated, non-myristoylated, monomeric, multimeric, particulate, denatured, etc.). The polypeptides can be naturally or non-naturally glycosylated (i.e. the polypeptide may have a glycosylation pattern that differs from the glycosylation pattern found in the corresponding naturally occurring polypeptide).

Non-naturally occurring forms of polypeptides herein may comprise one or more heterologous amino acid sequences (e.g. another antigen sequence, another signal sequence, a detectable tag, or the like) in addition to an antigen sequence. For example, a polypeptide herein may be a fusion protein. Alternatively, or in addition, the amino acid sequence or chemical structure of the polypeptide may be modified (e.g. with one or more non-natural amino acids, by covalent modification, and/or or by having a different glycosylation pattern, for example, by the removal or addition of one or more glycosyl groups) compared to a naturally-occurring polypeptide sequence.

Identity with respect to a sequence is defined herein as the percentage of amino acid residues in the candidate sequence that are identical with the reference amino acid sequence after aligning the sequences and introducing gaps, if necessary, to achieve the maximum percent sequence identity, and not considering any conservative substitutions as part of the sequence identity.

Sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a pre-determined portion of one or both sequences).

The skilled person will recognise that individual substitutions, deletions or additions to a protein which alters, adds or deletes a single amino acid or a small percentage of amino acids is an "immunogenic derivative" where the alteration(s) results in the substitution of an amino acid with a functionally similar amino acid or the substitution/deletion/addition of residues which do not impact the immunogenic function.

Conservative substitution tables providing functionally similar amino acids are well known in the art. In general, such conservative substitutions will fall within one of the amino-acid groupings specified below, though in some circumstances other substitutions may be possible without substantially affecting the immunogenic properties of the antigen. The following eight groups each contain amino acids that are typically conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
Suitably such substitutions do not occur in the region of an epitope, and do not therefore have a significant impact on the immunogenic properties of the antigen.

Immunogenic derivatives may also include those wherein additional amino acids are inserted compared to the reference sequence. Suitably such insertions do not occur in the region of an epitope, and do not therefore have a significant impact on the immunogenic properties of the antigen.

Immunogenic derivatives include those wherein amino acids have been deleted compared to the reference sequence. Suitably such deletions do not occur in the region of an epitope, and do not therefore have a significant impact on the immunogenic properties of the antigen. The skilled person will recognise that a particular immunogenic derivative may comprise substitutions, deletions and additions (or any combination thereof).

A "variant" of a polypeptide sequence includes amino acid sequences having one or more amino acid additions, substitutions and/or deletions when compared to the reference sequence. The variant may comprise an amino acid sequence which is at least 70%, at least 75%, at least 80%, at least 85%, at least 90%, at least 95%, at least 96%, at least 97%, at least 98%, or at least 99% identical to a full-length wild-type polypeptide. Alternatively, or in addition to, a fragment of a polypeptide may comprise an immunogenic fragment (i.e. an epitope-containing fragment) of the full-length polypeptide which may comprise or consist of a contiguous amino acid sequence of at least 8, at least 9, at least 10, at least 11, at least 12, at least 13, at least 14, at least 15, at least 16, at least 17, at least 18, at least 20, or more amino acids which is identical to a contiguous amino acid sequence of the full-length polypeptide.

An infected subject is a subject that has been exposed to a virus such as influenza that causes a natural immune response in the subject. A vaccinated subject is a subject that has been administered a vaccine that is intended to provide a protective effect against a virus such as influenza.

In one embodiment, the immune response is a CD4+ T cell response. In a preferred embodiment the immune response is a CD8+ T cell response.

In another embodiment the booster vaccine is administered to the subject after the primer vaccine. In a preferred embodiment, the booster vaccine is administered 28 days after administration of the primer vaccine. In another embodiment, the booster vaccine is administered to the subject about 1 week, or 2 weeks, or 3 weeks, or 4 weeks, or 5 weeks, or 6 weeks, or 7 weeks, or 8 weeks, or 9 weeks, or 16 weeks, or 20 weeks, or 24 weeks, or 28 weeks, or 32 weeks after administration of the primer vaccine. Administration of the priming vaccine and the boosting vaccine can be separated by any suitable timeframe. In some embodiments, the booster vaccine can be administered at about 1 week, 2 weeks 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 24 weeks, 28 weeks, 35 weeks, 40 weeks, 50 weeks, or about 52 weeks, or a range defined by any two of the foregoing values, following administration of the first immunogenic compound. More than one dose of priming vaccine and/or boosting vaccine can be provided in any suitable timeframe. The dose of the priming vaccine and boosting vaccine administered to the subject depends on a number of factors, including the extent of any side-effects, the particular route of administration, and the like.

In one embodiment the booster vaccine is administered at least 6 months after the first vaccination(s), preferably 8 to 14 months after, more preferably at around 10 to 12 months after.

In another embodiment, the booster dose is about 10E5 infectious units per animal, preferably the dose is less than 10E5, preferably less than 20E5 unit per animal, or preferably less than 30E5.

In another embodiment, the present invention further comprises administering to the mammal a second boosting immunogenic composition.

In one embodiment, the primer vaccine is administered parentally. In another embodiment the booster vaccine is administered intranasally. For parenteral application, which includes intramuscular, intradermal, subcutaneous, intranasal, intracapsular, intraspinal, intrasternal, and intravenous injection, particularly suitable are injectable, sterile solutions, preferably oily or aqueous solutions, as well as suspensions, emulsions, or implants, including suppositories. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

Preferred devices for intranasal administration of the vaccines according to the invention are spray devices. Suitable commercially available nasal spray devices include AccusprayTM (Becton Dickinson). Nebulisers produce a very fine spray which can be easily inhaled. Nebulisers are therefore not preferred. Preferred spray devices for intranasal use are devices for which the performance of the device is not dependent upon the pressure applied by the user. These devices are known as pressure threshold devices. Liquid is released from the nozzle only when a threshold pressure is applied. These devices make it easier to achieve a spray with a regular droplet size. Pressure threshold devices suitable for use with the present invention are known in the art and are described for example in WO 91/13281 and EP 311 863 B and EP 516 636, Such devices are commercially available from Pfeiffer GmbH and are also described in Bommer, R. Pharmaceutical Technology Europe, Sept 1999.

Preferred intranasal devices produce droplets (measured using water as the liquid) in the range 1 to 200 p.m, preferably 10 to 120 pm. Below 10 p.m there is a risk of inhalation, therefore it is desirable to have no more than about 5% of droplets below 10 pm. Droplets above 120 pm do not spread as well as smaller droplets, so it is desirable to have no more than about 5% of droplets exceeding 120 pm. Bi-dose delivery is a further preferred feature of an intranasal delivery system for use with the vaccines according to the invention. Bi-dose devices contain two sub-doses of a single vaccine dose, one sub-dose for administration to each nostril. Generally, the two sub-doses are present in a single chamber and the construction of the device allows the efficient delivery of a single sub-dose at a time.

In one embodiment, the composition triggers a local (mucosal) and systemic immune response. In another embodiment the administration of the composition to a mammal can elicit humoral and/or cellular immune responses in the mammal. In certain embodiments, the immune response comprises a cellular immune response. In certain particular embodiments, the cellular immune response comprises a T cell-mediated immune response; in certain particular embodiments, the T cell-mediated immune response comprises a CD8+ T cell-mediated immune response.

In another embodiment, the composition prevents the shedding of the Live Attenuated Influenza Vaccine. In another embodiment, the composition prevents the transmission of the Live Attenuated Influenza Virus.

In other aspects kits are provided based on methods for the proteins of the present invention or their derivatives.

The invention contemplates combinations of any of the foregoing aspects and embodiments of the invention. Each and every embodiment described throughout the application can be combined, and can be applied to each and every aspect of the invention described herein. Further, the methods and reagents of the various aspects of the instant invention can be used prophylactically. Alternatively, they can be used therapeutically. The present invention is further illustrated by the figures and examples from which further features, embodiments and advantages may be taken.

### Sequence Listing

| Seq. ID NO: | |
|---|---|
| 1 (VSV HA) | ACGAAGACAAACAAACCATTATTATCATTAAAAGGCTCAGGAGAAACTTTAACAGTAATC AAAATGTCTGTTACAGTCAAGAGAATCATTGACAACACAGTCGTAGTTCCAAAACTTCCT GCAAATGAGGATCCAGTGGAATACCCGGCAGATTACTTCAGAAAATCAAAGGAGATTCCT CTTTACATCAATACTACAAAAAGTTTGTCAGATCTAAGAGGATATGTCTACCAAGGCCTC AAATCCGGAAATGTATCAATCATACATGTCAACAGCTACTTGTATGGAGCATTAAAGGAC ATCCGGGGTAAGTTGGATAAAGATTGGTCAAGTTTCGGAATAAACATCGGGAAAGCAGGG GATACAATCGGAATATTTGACCTTGTATCCTTGAAAGCCCTGGACGGCGTACTTCCAGAT GGAGTATCGGATGCTTCCAGAACCAGCGCAGATGACAAATGGTTGCCTTTGTATCTACTT GGCTTATACAGAGTGGGCAGAACACAAATGCCTGAATACAGAAAAAAGCTCATGGATGGG CTGACAAATCAATGCAAAATGATCAATGAACAGTTTGAACCTCTTGTGCCAGAAGGTCGT GACATTTTTGATGTGTGGGGAAATGACAGTAATTACACAAAAATTGTCGCTGCAGTGGAC ATGTTCTTCCACATGTTCAAAAAACATGAATGTGCCTCGTTCAGATACGGAACTATTGTT TCCAGATTCAAAGATTGTGCTGCATTGGCAACATTTGGACACCTCTGCAAAATAACCGGA ATGTCTACAGAAGATGTAACGACCTGGATCTTGAACCGAGAAGTTGCAGATGAAATGGTC CAAATGATGCTTCCAGGCCAAGAAATTGACAAGGCCGATTCATACATGCCTTATTTGATC GACTTTGGATTGTCTTCTAAGTCTCCATATTCTTCCGTCAAAAACCCTGCCTTCCACTTC TGGGGGCAATTGACAGCTCTTCTGCTCAGATCCACCAGAGCAAGGAATGCCCGACAGCCT GATGACATTGAGTATACATCTCTTACTACAGCAGGTTTGTTGTACGCTTATGCAGTAGGA TCCTCTGCCGACTTGGCACAACAGTTTTGTGTTGGAGATAACAAATACACTCCAGATGAT AGTACCGGAGGATTGACGACTAATGCACCGCCACAAGGCAGAGATGTGGTCGAATGGCTC GGATGGTTTGAAGATCAAAACAGAAAACCGACTCCTGATATGATGCAGTATGCGAAAAGA GCAGTCATGTCACTGCAAGGCCTAAGAGAGAAGACAATTGGCAAGTATGCTAAGTCAGAA TTTGACAAATGACCCTATAATTCTCAGATCACCTATTATATATTATGCTACATATGAAAA AAACTAACAGATATCATGGATAATCTCACAAAAGTTCGTGAGTATCTCAAGTCCTATTCT CGTCTGGATCAGGCGGTAGGAGAGATAGATGAGATCGAAGCACAACGAGCTGAAAAGTCC AATTATGAGTTGTTCCAAGAGGATGGAGTGGAAGAGCATACTAAGCCCTCTTATTTTCAG GCAGCAGATGATTCTGACACAGAATCTGAACCAGAAATTGAAGACAATCAAGGCTTGTAT GCACCAGATCCAGAAGCTGAGCAAGTTGAAGGCTTTATACAGGGGCCTTTAGATGACTAT GCAGATGAGGAAGTGGATGTTGTATTTACTTCGGACTGGAAACAGCCTGAGCTTGAATCT GACGAGCATGGAAAGACCTTACGGTTGACATCGCCAGAGGGTTTAAGTGGAGAGCAGAAA TCCCAGTGGCTTTCGACGATTAAAGCAGTCGTGCAAAGTGCCAAATACTGGAATCTGGCA GAGTGCACATTTGAAGCATCGGGAGAAGGGGTCATTATGAAGGAGCGCCAGATAACTCCG GATGTATATAAGGTCACTCCAGTGATGAACACACATCCGTCCCAATCAGAAGCAGTATCA GATGTTTGGTCTCTCTCAAAGACATCCATGACTTTCCAACCCAAGAAAGCAAGTCTTCAG CCTCTCACCATATCCTTGGATGAATTGTTCTCATCTAGAGGAGAGTTCATCTCTGTCGGA GGTGACGGACGAATGTCTCATAAAGAGGCCATCCTGCTCGGCCTGAGATACAAAAAGTTG TACAATCAGGCGAGAGTCAAATATTCTCTGTAGACTATGAAAAAAAGTAACAGATATCAC GATCTAAGTGTTATCCCAATCCATTCATCATGAGTTCCTTAAAGAAGATTCTCGGTCTGA AGGGGAAAGGTAAGAAATCTAAGAAATTAGGGATCGCACCACCCCCTTATGAAGAGGACA CTAGCATGGAGTATGCTCCGAGCGCTCCAATTGACAAATCCTATTTTGGAGTTGACGAGA TGGACACCTATGATCCGAATCAATTAAGATATGAGAAATTCTTCTTTACAGTGAAAATGA CGGTTAGATCTAATCGTCCGTTCAGAACATACTCAGATGTGGCAGCCGCTGTATCCCATT GGGATCACATGTACATCGGAATGGCAGGGAAACGTCCCTTCTACAAAATCTTGGCTTTTT TGGGTTCTTCTAATCTAAAGGCCACTCCAGCGGTATTGGCAGATCAAGGTCAACCAGAGT ATCACGCTCACTGCGAAGGCAGGGCTTATTTGCCACATAGGATGGGGAAGACCCCTCCCA TGCTCAATGTACCAGAGCACTTCAGAAGACCATTCAATATAGGTCTTTACAAGGGAACGA TTGAGCTCACAATGACCATCTACGATGATGAGTCACTGGAAGCAGCTCCTATGATCTGGG ATCATTTCAATTCTTCCAAATTTTCTGATTTCAGAGAGAAGGCCTTAATGTTTGGCCTGA TTGTCGAGAAAAAGGCATCTGGAGCGTGGGTCCTGGACTCTATCGGCCACTTCAAATGAG CTAGTCTAACTTCTAGCTTCTGAACAATCCCCGGTTTACTCAGTCTCCCCTAATTCCAGC CTCTCGAACAACTAATATCCTGTCTTTTCTATCCCTATGAAAAAAACTAACAGAGATCGA TCTGTTTACGCGTAACATGAAGGCAATACTAGTAGTTCTGCTATATACATTTACAACCGC AAATGCAGACACATTATGTATAGGTTATCATGCGAACAATTCAACAGACACTGTAGACAC AGTACTAGAAAAGAATGTAACAGTAACACACTCTGTTAACCTTCTAGAAGACAAGCATAA CGGGAAACTATGCAAACTAAGAGGGGTAGCCCCATTGCATTTGGGTAAATGTAACATTGC TGGCTGGATCCTGGGAAATCCAGAGTGTGAATCACTCTCCACAGCAAGCTCATGGTCCTA CATTGTGGAAACATCTAGTTCAGACAATGGAACGTGTTACCCAGGAGATTTCATCGATTA TGAGGAGCTAAGAGAGCAATTGAGCTCAGTGTCATCATTTGAAAGGTTTGAGATATTCCC CAAGACAAGTTCATGGCCCAATCATGACTCGAACAAAGGTGTAACGGCAGCATGTCCTCA TGCTGGAGCAAAAAGCTTCTACAAAAATTTAATATGGCTAGTTAAAAAAGGAAATTCATA CCCAAAGCTCAGCAAATCCTACATTAATGATAAAGGGAAAGAAGTCCTCGTGCTATGGGG |
| | CATTCACCATCCATCTACTAGTGCTGACCAACAAAGTCTCTATCAGAATGCAGATGCATA TGTTTTTGTGGGGTCATCAAGATACAGCAAGAAGTTCAAGCCGGAAATAGCAATAAGACC CAAAGTGAGGGATCAAGAAGGGAGAATGAACTATTACTGGACACTAGTAGAGCCGGGAGA CAAAATAACATTCGAAGCAACTGGAAATCTAGTGGTACCGAGATATGCATTCGCAATGGA AAGAAATGCTGGATCTGGTATTATCATTTCAGATACACCAGTCCACGATTGCAATACAAC TTGTCAGACACCCAAGGGTGCTATAAACACCAGCCTCCCATTTCAGAATATACATCCGAT CACAATTGGAAAATGTCCAAAATATGTAAAAAGCACAAAATTGAGACTGGCCACAGGATT GAGGAATGTCCCGTCTATTCAATCTAGAGGCCTATTTGGGGCCATTGCCGGTTTCATTGA AGGGGGGTGGACAGGGATGGTAGATGGATGGTACGGTTATCACCATCAAAATGAGCAGGG GTCAGGATATGCAGCCGACCTGAAGAGCACACAGAATGCCATTGACGAGATTACTAACAA AGTAAATTCTGTTATTGAAAAGATGAATACACAGTTCACAGCAGTAGGTAAAGAGTTCAA CCACCTGGAAAAAAGAATAGAGAATTTAAATAAAAAAGTTGATGATGGTTTCCTGGACAT TTGGACTTACAATGCCGAACTGTTGGTTCTATTGGAAAATGAAAGAACTTTGGACTACCA CGATTCAAATGTGAAGAACTTATATGAAAAGGTAAGAAGCCAGCTAAAAAACAATGCCAA GGAAATTGGAAACGGCTGCTTTGAATTTTACCACAAATGCGATAACACGTGCATGGAAAG TGTCAAAAATGGGACTTATGACTACCCAAAATACTCAGAGGAAGCAAAATTAAACAGAGA AGAAATAGATGGGGTAAAGCTGGAATCAACAAGGATTTACCAGATTTTGGCGATCTATTC AACTGTCGCCAGTTCATTGGTACTGGTAGTCTCCCTGGGGGCAATCAGTTTCTGGATGTG CTCTAATGGGTCTCTACAGTGTAGAATATGTATTTAAGCTAGCCAGATTCTTCATGTTTG GACCAAATCAACTTGTGATACCATGCTCAAAGAGGCCTCAATTATATTTGAGTTTTTAATT TTTATGAAAAAAACTAACAGCAATCATGGAAGTCCACGATTTTGAGACCGACGAGTTCA ATGATTTCAATGAAGATGACTATGCCACAAGAGAATTCCTGAATCCCGATGAGCGCATGA CGTACTTGAATCATGCTGATTACAACCTGAATTCTCCTCTAATTAGTGATGATATTGACA ATTTAATCAGGAAATTCAATTCTCTTCCAATTCCCTCGATGTGGGATAGTAAGAACTGGG ATGGAGTTCTTGAGATGTTAACGTCATGTCAAGCCAATCCCATCCCAACATCTCAGATGC ATAAATGGATGGGAAGTTGGTTAATGTCTGATAATCATGATGCCAGTCAAGGGTATAGTT TTTTACATGAAGTGGACAAAGAGGCAGAAATAACATTTGACGTGGTGGAGACCTTCATCC GCGGCTGGGGCAACAAACCAATTGAATACATCAAAAAGGAAAGATGGACTGACTCATTCA AAATTCTCGCTTATTTGTGTCAAAAGTTTTTGGACTTACACAAGTTGACATTAATCTTAA ATGCTGTCTCTGAGGTGGAATTGCTCAACTTGGCGAGGACTTTCAAAGGCAAAGTCAGAA GAAGTTCTCATGGAACGAACATATGCAGGATTAGGGTTCCCAGCTTGGGTCCTACTTTTA TTTCAGAAGGATGGGCTTACTTCAAGAAACTTGATATTCTAATGGACCGAAACTTTCTGT TAATGGTCAAAGATGTGATTATAGGGAGGATGCAAACGGTGCTATCCATGGTATGTAGAA TAGACAACCTGTTCTCAGAGCAAGACATCTTCTCCCTTCTAAATATCTACAGAATTGGAG ATAAAATTGTGGAGAGGCAGGGAAATTTTTCTTATGACTTGATTAAAATGGTGGAACCGA TATGCAACTTGAAGCTGATGAAATTAGCAAGAGAATCAAGGCCTTTAGTCCCACAATTCC CTCATTTTGAAAATCATATCAAGACTTCTGTTGATGAAGGGGCAAAAATTGACCGAGGTA TAAGATTCCTCCATGATCAGATAATGAGTGTGAAAACAGTGGATCTCACACTGGTGATTT ATGGATCGTTCAGACATTGGGGTCATCCTTTTATAGATTATTACACTGGACTAGAAAAAT TACATTCCCAAGTAACCATGAAGAAAGATATTGATGTGTCATATGCAAAAGCACTTGCAA GTGATTTAGCTCGGATTGTTCTATTTCAACAGTTCAATGATCATAAAAAGTGGTTCGTGA ATGGAGACTTGCTCCCTCATGATCATCCCTTTAAAAGTCATGTTAAAGAAAATACATGGC CCACAGCTGCTCAAGTTCAAGATTTTGGAGATAAATGGCATGAACTTCCGCTGATTAAAT GTTTTGAAATACCCGACTTACTAGACCCATCGATAATATACTCTGACAAAAGTCATTCAA TGAATAGGTCAGAGGTGTTGAAACATGTCCGAATGAATCCGAACACTCCTATCCCTAGTA AAAAGGTGTTGCAGACTATGTTGGACACAAAGGCTACCAATTGGAAAGAATTTCTTAAAG AGATTGATGAGAAGGGCTTAGATGATGATGATCTAATTATTGGTCTTAAAGGAAAGGAGA GGGAACTGAAGTTGGCAGGTAGATTTTTCTCCCTAATGTCTTGGAAATTGCGAGAATACT TTGTAATTACCGAATATTTGATAAAGACTCATTTCGTCCCTATGTTTAAAGGCCTGACAA TGGCGGACGATCTAACTGCAGTCATTAAAAAGATGTTAGATTCCTCATCCGGCCAAGGAT TGAAGTCATATGAGGCAATTTGCATAGCCAATCACATTGATTACGAAAAATGGAATAACC ACCAAAGGAAGTTATCAAACGGCCCAGTGTTCCGAGTTATGGGCCAGTTCTTAGGTTATC CATCCTTAATCGAGAGAACTCATGAATTTTTTGAGAAAAGTCTTATATACTACAATGGAA GACCAGACTTGATGCGTGTTCACAACAACACACTGATCAATTCAACCTCCCAACGAGTTT GTTGGCAAGGACAAGAGGGTGGACTGGAAGGTCTACGGCAAAAAGGATGGAGTATCCTCA ATCTACTGGTTATTCAAAGAGAGGCTAAAATCAGAAACACTGCTGTCAAAGTCTTGGCAC AAGGTGATAATCAAGTTATTTGCACACAGTATAAAACGAAGAAATCGAGAAACGTTGTAG AATTACAGGGTGCTCTCAATCAAATGGTTTCTAATAATGAGAAAATTATGACTGCAATCA AAATAGGGACAGGGAAGTTAGGACTTTTGATAAATGACGATGAGACTATGCAATCTGCAG ATTACTTGAATTATGGAAAAATACCGATTTTCCGTGGAGTGATTAGAGGGTTAGAGACCA AGAGATGGTCACGAGTGACTTGTGTCACCAATGACCAAATACCCACTTGTGCTAATATAA TGAGCTCAGTTTCCACAAATGCTCTCACCGTAGCTCATTTTGCTGAGAACCCAATCAATG CCATGATACAGTACAATTATTTTGGGACATTTGCTAGACTCTTGTTGATGATGCATGATC CTGCTCTTCGTCAATCATTGTATGAAGTTCAAGATAAGATACCGGGCTTGCACAGTTCTA CTTTCAAATACGCCATGTTGTATTTGGACCCTTCCATTGGAGGAGTGTCGGGCATGTCTT TGTCCAGGTTTTTGATTAGAGCCTTCCCAGATCCCGTAACAGAAAGTCTCTCATTCTGGA GATTCATCCATGTACATGCTCGAAGTGAGCATCTGAAGGAGATGAGTGCAGTATTTGGAA ACCCCGAGATAGCCAAGTTTCGAATAACTCACATAGACAAGCTAGTAGAAGATCCAACCT |
| | CTCTGAACATCGCTATGGGAATGAGTCCAGCGAACTTGTTAAAGACTGAGGTTAAAAAAT GCTTAATCGAATCAAGACAAACCATCAGGAACCAGGTGATTAAGGATGCAACCATATATT TGTATCATGAAGAGGATCGGCTCAGAAGTTTCTTATGGTCAATAAATCCTCTGTTCCCTA GATTTTTAAGTGAATTCAAATCAGGCACTTTTTTGGGAGTCGCAGACGGGCTCATCAGTC TATTTCAAAATTCTCGTACTATTCGGAACTCCTTTAAGAAAAAGTATCATAGGGAATTGG ATGATTTGATTGTGAGGAGTGAGGTATCCTCTTTGACACATTTAGGGAAACTTCATTTGA GAAGGGGATCATGTAAAATGTGGACATGTTCAGCTACTCATGCTGACACATTAAGATACA AATCCTGGGGCCGTACAGTTATTGGGACAACTGTACCCCATCCATTAGAAATGTTGGGTC CACAACATCGAAAAGAGACTCCTTGTGCACCATGTAACACATCAGGGTTCAATTATGTTT CTGTGCATTGTCCAGACGGGATCCATGACGTCTTTAGTTCACGGGGACCATTGCCTGCTT ATCTAGGGTCTAAAACATCTGAATCTACATCTATTTTGCAGCCTTGGGAAAGGGAAAGCA AAGTCCCACTGATTAAAAGAGCTACACGTCTTAGAGATGCTATCTCTTGGTTTGTTGAAC CCGACTCTAAACTAGCAATGACTATACTTTCTAACATCCACTCTTTAACAGGCGAAGAAT GGACCAAAAGGCAGCATGGGTTCAAAAGAACAGGGTCTGCCCTTCATAGGTTTTCGACAT CTCGGATGAGCCATGGTGGGTTCGCATCTCAGAGCACTGCAGCATTGACCAGGTTGATGG CAACTACAGACACCATGAGGGATCTGGGAGATCAGAATTTCGACTTTTTATTCCAAGCAA CGTTGCTCTATGCTCAAATTACCACCACTGTTGCAAGAGACGGATGGATCACCAGTTGTA CAGATCATTATCATATTGCCTGTAAGTCCTGTTTGAGACCCATAGAAGAGATCACCCTGG ACTCAAGTATGGACTACACGCCCCCAGATGTATCCCATGTGCTGAAGACATGGAGGAATG GGGAAGGTTCGTGGGGACAAGAGATAAAACAGATCTATCCTTTAGAAGGGAATTGGAAGA ATTTAGCACCTGCTGAGCAATCCTATCAAGTCGGCAGATGTATAGGTTTTCTATATGGAG ACTTGGCGTATAGAAAATCTACTCATGCCGAGGACAGTTCTCTATTTCCTCTATCTATAC AAGGTCGTATTAGAGGTCGAGGTTTCTTAAAAGGGTTGCTAGACGGATTAATGAGAGCAA GTTGCTGCCAAGTAATACACCGGAGAAGTCTGGCTCATTTGAAGAGGCCGGCCAACGCAG TGTACGGAGGTTTGATTTACTTGATTGATAAATTGAGTGTATCACCTCCATTCCTTTCTC TTACTAGATCAGGACCTATTAGAGACGAATTAGAAACGATTCCCCACAAGATCCCAACCT CCTATCCGACAAGCAACCGTGATATGGGGGTGATTGTCAGAAATTACTTCAAATACCAAT GCCGTCTAATTGAAAAGGGAAAATACAGATCACATTATTCACAATTATGGTTATTCTCAG ATGTCTTATCCATAGACTTCATTGGACCATTCTCTATTTCCACCACCCTCTTGCAAATCC TATACAAGCCATTTTTATCTGGGAAAGATAAGAATGAGTTGAGAGAGCTGGCAAATCTTT CTTCATTGCTAAGATCAGGAGAGGGGTGGGAAGACATACATGTGAAATTCTTCACCAAGG ACATATTATTGTGTCCAGAGGAAATCAGACATGCTTGCAAGTTCGGGATTGCTAAGGATA ATAATAAAGACATGAGCTATCCCCCTTGGGGAAGGGAATCCAGAGGGACAATTACAACAA TCCCTGTTTATTATACGACCACCCCTTACCCAAAGATGCTAGAGATGCCTCCAAGAATCC AAAATCCCCTGCTGTCCGGAATCAGGTTGGGCCAATTACCAACTGGCGCTCATTATAAAA TTCGGAGTATATTACATGGAATGGGAATCCATTACAGGGACTTCTTGAGTTGTGGAGACG GCTCCGGAGGGATGACTGCTGCATTACTACGAGAAAATGTGCATAGCAGAGGAATATTCA ATAGTCTGTTAGAATTATCAGGGTCAGTCATGCGAGGCGCCTCTCCTGAGCCCCCCAGTG CCCTAGAAACTTTAGGAGGAGATAAATCGAGATGTGTAAATGGTGAAACATGTTGGGAAT ATCCATCTGACTTATGTGACCCAAGGACTTGGGACTATTTCCTCCGACTCAAAGCAGGCT TGGGGCTTCAAATTGATTTAATTGTAATGGATATGGAAGTTCGGGATTCTTCTACTAGCC TGAAAATTGAGACGAATGTTAGAAATTATGTGCACCGGATTTTGGATGAGCAAGGAGTTT TAATCTACAAGACTTATGGAACATATATTTGTGAGAGCGAAAAGAATGCAGTAACAATCC TTGGTCCCATGTTCAAGACGGTCGACTTAGTTCAAACAGAATTTAGTAGTTCTCAAACGT CTGAAGTATATATGGTATGTAAAGGTTTGAAGAAATTAATCGATGAACCCAATCCCGATT GGTCTTCCATCAATGAATCCTGGAAAAACCTGTACGCATTCCAGTCATCAGAACAGGAAT TTGCCAGAGCAAAGAAGGTTAGTACATACTTTACCTTGACAGGTATTCCCTCCCAATTCA TTCCTGATCCTTTTGTAAACATTGAGACTATGCTACAAATATTCGGAGTACCCACGGGTG TGTCTCATGCGGCTGCCTTAAAATCATCTGATAGACCTGCAGATTTATTGACCATTAGCC TTTTTTATATGGCGATTATATCGTATTATAACATCAATCATATCAGAGTAGGACCGATAC CTCCGAACCCCCCATCAGATGGAATTGCACAAAATGTGGGGATCGCTATAACTGGTATAA GCTTTTGGCTGAGTTTGATGGAGAAAGACATTCCACTATATCAACAGTGTTTAGCAGTTA TCCAGCAATCATTCCCGATTAGGTGGGAGGCTGTTTCAGTAAAAGGAGGATACAAGCAGA AGTGGAGTACTAGAGGTGATGGGCTCCCAAAAGATACCCGAATTTCAGACTCCTTGGCCC CAATCGGGAACTGGATCAGATCTCTGGAATTGGTCCGAAACCAAGTTCGTCTAAATCCAT TCAATGAGATCTTGTTCAATCAGCTATGTCGTACAGTGGATAATCATTTGAAATGGTCAA ATTTGCGAAGAAACACAGGAATGATTGAATGGATCAATAGACGAATTTCAAAAGAAGACC GGTCTATACTGATGTTGAAGAGTGACCTACACGAGGAAAACTCTTGGAGAGATTAAAAAA TCATGAGGAGACTCCAAACTTTAAGTATGAAAAAAACTTTGATCCTTAAGACCCTCTTGT GGTTTTTATTTTTTATCTGGTTTTGTGGTCTTCGT |
| 2 (NS1(1-126) | Genome segment 1/PB2 AGCGAAAGCAGGTCAAATATATTCAATATGGAGAGAATAAAAGAACTGAGAGATCTAATG TCGCAGTCCCGCACTCGCGAGATACTCACTAAGACCACTGTGGACCATATGGCCATAATC AAAAAGTACACATCAGGAAGGCAAGAGAAGAACCCCGCACTCAGAATGAAGTGGATGATG GCAATGAGATACCCAATTACAGCAGACAAGAGAATAATGGACATGATTCCAGAGAGGAAT |
| | GAACAAGGACAAACCCTCTGGAGCAAAACAAACGATGCTGGATCAGACCGAGTGATGGTA TCACCTCTGGCCGTAACATGGTGGAATAGGAATGGCCCAACAACAAGTACAGTTCATTAC CCTAAGGTATATAAAACTTATTTCGAAAAGGTCGAAAGGTTGAAACATGGTACCTTCGGC CCTGTCCACTTCAGAAATCAAGTTAAAATAAGGAGGAGAGTTGATACAAACCCTGGCCAT GCAGATCTCAGTGCCAAGGAGGCACAGGATGTGATTATGGAAGTTGTTTTCCCAAATGAA GTGGGGGCAAGAATACTGACATCAGAGTCACAGCTGGCAATAACAAAAGAGAAGAAAGAA GAGCTCCAGGATTGTAAAATTGCTCCCTTGATGGTGGCGTACATGCTAGAAAGAGAATTG GTCCGTAAAACAAGGTTTCTCCCAGTAGCCGGCGGAACAGGCAGTGTTTATATTGAAGTG TTGCACTTAACCCAAGGGACGTGCTGGGAGCAGATGTACACTCCAGGAGGAGAAGTGAGA AATGATGATGTTGACCAAAGTTTGATTATCGCTGCTAGAAACATAGTAAGAAGAGCAGCA GTGTCAGCAGACCCATTAGCATCTCTCTTGGAAATGTGCCACAGCACACAGATTGGAGGA GTAAGGATGGTGGACATCCTTAGACAGAATCCAACTGAGGAACAAGCCGTAGACATATGC AAGGCAGCAATAGGGTTGAGGATTAGCTCATCTTTCAGTTTTGGTGGGTTCACTTTCAAA AGGACAAGCGGATCATCAGTCAAGAAAGAAGAAGAAGTGCTAACGGGCAACCTCCAAACA CTGAAAATAAGAGTACATGAAGGGTATGAAGAATTCACAATGGTTGGGAGAAGAGCAACA GCTATTCTCAGAAAGGCAACCAGGAGATTGATCCAGTTGATAGTAAGCGGGAGAGACGAG CAGTCAATTGCTGAGGCAATAATTGTGGCCATGGTATTCTCACAAGAGGATTGCATGATC AAGGCAGTTAGGGGCGATCTGAACTTTGTCAATAGGGCAAACCAGCGACTGAACCCCATG CACCAACTCTTGAGGCATTTCCAAAAAGATGCAAAAGTGCTTTTCCAGAACTGGGGAATT GAATCCATCGACAATGTGATGGGAATGATCGGAATACTGCCCGACATGACCCCAAGCACG GAGATGTCGCTGAGAGGGATAAGAGTCAGCAAAATGGGAGTAGATGAATACTCCAGCACG GAGAGAGTGGTAGTGAGTATTGACCGATTTTTAAGGGTTAGAGATCAAAGAGGGAACGTA CTATTGTCTCCCGAAGAAGTCAGTGAAACGCAAGGAACTGAGAGGTTGACAATAACTTAT TCGTCATCAATGATGTGGGAGATCAATGGCCCTGAGTCAGTGCTAGTCAACACTTATCAA TGGATAATCAGGAACTGGGAAATTGTGAAAATTCAATGGTCACAAGATCCCACAATGTTA TACAACAAAATGGAATTTGAACCATTTCAGTCTCTTGTCCCTAAGGCAACCAGAAGCCGG TACAGTGGATTCGTAAGGACACTGTTCCAGCAAATGCGGGATGTGCTTGGGACATTTGAC ACTGTCCAAATAATAAAACTTCTCCCCTTTGCTGCTGCTCCACCAGAACAGAGTAGGATG CAATTTTCCTCATTGACTGTGAATGTGAGAGGATCAGGGTTGAGGATACTGGTAAGAGGC AATTCTCCAGTATTCAATTACAACAAGGCAACCAAACGACTTACAGTTCTTGGAAAGGAT GCAGGTGCATTGACTGAAGATCCAGATGAAGGCACATCTGGGGTGGAGTCTGCTGTCCTG AGAGGATTTCTCATTTTGGGCAAAGAAGACAAGAGATATGGCCCAGCATTAAGCATCAAT GAACTGAGCAATCTTGCAAAAGGAGAGAAGGCTAATGTGCTAATTGGGCAAGGGGACGTA GTGTTGGTAATGAAACGAAAACGGGACTCTAGCATACTTACTGACAGCCAGACAGCGACC AAAAGAATTCGGATGGCCATCAATTAGTGTCGAATTGTTTAAAAACGACCTTGTTTCTAC T |
| | Genome segment 2/PB1 AGCGAAAGCAGGCAAACCATTTGAATGGATGTCAATCCGACTCTACTTTTCCTAAAAATT CCAGCGCAAAATGCCATAAGCACCACATTCCCTTATACTGGAGATCCTCCATACAGCCAT GGAACAGGAACAGGATACACCATGGACACAGTAAACAGAACACACCAATACTCAGAAAAG GGAAAGTGGACGACAAACACAGAGACTGGTGCACCCCAGCTCAACCCGATTGATGGACCA CTACCTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATGGAA GTTGTTCAACAAACAAGGGTAGATAAACTAACTCAAGGTCGCCAGACTTATGATTGGACA TTAAACAGAAATCAACCGGCAGCAACTGCATTGGCCAACACCATAGAAGTCTTTAGATCG AATGGCCTAACAGCTAATGAGTCAGGAAGGCTAATAGATTTCTTAAAGGATGTAATGGAA TCAATGAACAAAGAGGAAATAGAGATAACAACCCACTTTCAAAGAAAAAGGAGAGTAAGA GACAACATGACCAAGAAGATGGTCACGCAAAGAACAATAGGGAAGAAAAAACAAAGACTG AATAAGAGAGGCTATCTAATAAGAGCACTGACATTAAATACGATGACCAAAGATGCAGAG AGAGGCAAGTTAAAAAGAAGGGCTATCGCAACACCTGGGATGCAGATTAGAGGTTTCGTA TACTTTGTTGAAACTTTAGCTAGGAGCATTTGCGAAAAGCTTGAACAGTCTGGGCTCCCA GTAGGGGGCAATGAAAAGAAGGCCAAACTGGCAAATGTTGTGAGAAAGATGATGACTAAT TCACAAGACACAGAGATTTCTTTCACAATCACTGGGGACAACACTAAGTGGAATGAAAAT CAAAATCCTCGAGTGTTCCTGGCGATGATTACATATATCACCAGAAATCAACCCGAGTGG TTCAGAAACATCCTGAGCATGGCACCCATAATGTTCTCAAACAAAATGGCAAGACTAGGG AAAGGGTACATGTTCGAGAGTAAAAGAATGAAGATTCGAACACAAATACCAGCAGAAATG CTAGCAAGCATTGACCTGAAGTACTTCAATGAATCAACAAAGAAGAAAATTGAGAAAATA AGGCCTCTTCTAATAGATGGCACAGCATCACTGAGTCCTGGGATGATGATGGGCATGTTC AACATGCTAAGTACGGTCTTGGGAGTCTCGATACTGAATCTTGGACAAAAGAAATACACC AAGACAATATACTGGTGGGATGGGCTCCAATCATCCGACGATTTTGCTCTCATAGTGAAT GCACCAAACCATGAGGGAATACAAGCAGGAGTGGACAGATTCTACAGGACCTGCAAGTTA GTGGGAATCAACATGAGCAAAAAGAAGTCCTATATAAATAAGACAGGGACATTTGAATTC ACAAGCTTTTTTTATCGCTATGGATTTGTGGCTAATTTTAGCATGGAGCTACCCAGCTTT GGAGTGTCTGGAGTAAATGAATCAGCTGACATGAGTATTGGAGTAACAGTGATAAAGAAC AACATGATAAACAATGACCTTGGACCTGCAACGGCCCAGATGGCTCTTCAATTGTTCATC AAAGACTACAGATACACATATAGGTGCCATAGGGGAGACACACAAATTCAGACGAGAAGA TCATTTGAGTTAAAGAAGCTGTGGGATCAAACCCAATCAAAGGTAGGGCTATTAGTATCA |
| | GATGGAGGACCAAACTTATACAATATACGGAATCTTCACATTCCTGAAGTCTGCTTAAAA TGGGAGCTAATGGATGATGATTATCGGGGAAGACTTTGTAATCCCCTGAATCCCTTTGTC AGTCATAAAGAGATTGATTCTGTAAACAATGCTGTGGTAATGCCAGCCCATGGTCCAGCC AAAAGCATGGAATATGATGCCGTTGCAACTACACATTCCTGGATTCCCAAGAGGAATCGT TCTATTCTCAACACAAGCCAAAGGGGAATTCTTGAGGATGAACAGATGTACCAGAAGTGC TGCAATCTATTCGAGAAATTTTTCCCTAGCAGTTCATATAGGAGACCGGTTGGAATTTCT AGCATGGTGGAGGCCATGGTGTCTAGGGCCCGGATTGATGCCAGGGTCGACTTCGAGTCT GGACGGATCAAGAAAGAAGAGTTCTCTGAGATCATGAAGATCTGTTCCACCATTGAAGAA CTCAGACGGCAAAAATAATGAATTTAGCTTGTCCTTCATGAAAAAATGCCTTGTTTCTAC T |
| | Genome segment 3/PA AGCAAAAGCAGGTACTGATCCAAAATGGAAGACTTTGTGCGACAATGCTTCAATCCAATG ATCGTCGAGCTTGCGGAAAAGGCAATGAAAGAATATGGGGAAGATCCGAAAATCGAAACT AACAAGTTTGCTGCAATATGCACACATTTGGAAGTTTGTTTCATGTATTCGGATTTCCAT TTCATCGACGAACGGGGTGAATCAATAATTGTAGAATCTGGTGACCCGAATGCACTATTG AAGCACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGATTAC AAAGAGAACCGGTTCATTGAAATTGGAGTAACACGGAGGGAAGTCCACATATATTACCTA GAGAAAGCCAACAAAATAAAATCTGAGAAGACACACATTCACATCTTTTCATTCACTGGA GAGGAGATGGCCACCAAAGCGGACTACACCCTTGACGAAGAGAGCAGGGCAAGAATCAAA ACTAGGCTTTTCACTATAAGACAAGAAATGGCCAGTAGGAGTCTATGGGATTCCTTTCGT CAGTCCGAAAGAGGCGAAGAGACAATTGAAGAAAAATTTGAGATTACAGGAACTATGCGC AAGCTTGCCGACCAAAGTCTCCCACCGAACTTCTCCAGCCTTGAAAACTTTAGAGCCTAT GTAGATGGATTCGAGCCGAACGGCTGCATTGAGGGCAAGCTTTCCCAAATGTCAAAAGAA GTGAACGCCAAAATTGAACCATTCTTGAGGACGACACCACGCCCCCTCAGATTGCCTGAT GGGCCTCTTTGCCATCAGCGGTCAAAGTTCCTGCTGATGGATGCTCTGAAATTAAGTATT GAAGACCCGAGTCACGAGGGGGAGGCAATACCACTATATGATGCAATCAAATGCATGAAG ACATTCTTTGGCTGGAAAGAGCCTAACATAGTCAAACCACATGAGAAAGGCATAAATCCC AATTACCTCATGGCTTGGAAGCAGGTGCTAGCAGAGCTACAGGACATTGAAAATGAAGAG AAGATCCCAAGGACAAAGAACATGAAGAGAACAAGCCAATTGAAGTGGGCACTCGGTGAA AATATGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATTCAAT AAGGCATGTGAATTGACTGATTCAAGCTGGATAGAACTTGATGAAATAGGAGAAGATGTT GCCCCGATTGAACATATCGCAAGCATGAGGAGGAACTATTTTACAGCAGAAGTGTCCCAC TGCAGGGCTACTGAATACATAATGAAGGGAGTGTACATAAATACGGCCTTGCTCAATGCA TCCTGTGCAGCCATGGATGACTTTCAGCTGATCCCAATGATAAGCAAATGTAGGACCAAA GAAGGAAGACGGAAAACAAACCTGTATGGGTTCATTATAAAAGGAAGGTCTCATTTGAGA AATGATACTGATGTGGTGAACTTTGTAAGTATGGAGTTCTCACTCACTGACCCGAGACTG GAGCCACACAAATGGGAAAAATACTGTGTTCTTGAAATAGGAGACATGCTCTTGAGGACT GCGATAGGCCAAGTGTCGAGGCCCATGTTCCTATATGTGAGAACCAATGGAACCTCCAAG ATCAAGATGAAATGGGGCATGGAACTGAGGCGCTGCCTTCTTCAGTCTCTTCAGCAGATT GAGAGCATGATTGAGGCCGAGTCTTCTGTCAAAGAGAAAGACATGACCAAGGAATTCTTT GAAAACAAATCGGAAACATGGCCAATCGGAGAGTCACCCAGGGGAGTGGAGGAAGGCTCT ATTGGGAAAGTGTGCAGGACCTTACTGGCAAAATCTGTATTCAACAGTCTATATGCGTCT CCACAACTTGAGGGGTTTTCGGCTGAATCGAGAAAATTGCTTCTCATTGTTCAGGCACTT AGGGACAACCTGGAACCTGGAACCTTCGATCTTGGGGGGCTATATGAAGCAATCGAGGAG TGCCTGATTAATGATCCCTGGGTTTTGCTTAATGCATCTTGGTTCAACTCCTTCCTCACA CATGCACTGAAGTAGTTGTGGCAATGCTACTATTTGCTATCCATACTGTCCAAAAAAGTA CCTTGTTTCTACT |
| | Genome segment 4/HA AGCAAAAGCAGGGGAAAACAAAAGCAACAAAAATGAAGGCAATACTAGTAGTTCTGCTAT ATACATTTACAACCGCAAATGCAGACACATTATGTATAGGTTATCATGCGAACAATTCAA CAGACACTGTAGACACAGTACTAGAAAAGAATGTAACAGTAACACACTCTGTTAACCTTC TAGAAGACAAGCATAACGGGAAACTATGCAAACTAAGAGGGGTAGCCCCATTGCATTTGG GTAAATGTAACATTGCTGGCTGGATCCTGGGAAATCCAGAGTGTGAATCACTCTCCACAG CAAGCTCATGGTCCTACATTGTGGAAACATCTAGTTCAGACAATGGAACGTGTTACCCAG GAGATTTCATCGATTATGAGGAGCTAAGAGAGCAATTGAGCTCAGTGTCATCATTTGAAA GGTTTGAGATATTCCCCAAGACAAGTTCATGGCCCAATCATGACTCGAACAAAGGTGTAA CGGCAGCATGTCCTCATGCTGGAGCAAAAAGCTTCTACAAAAATTTAATATGGCTAGTTA AAAAAGGAAATTCATACCCAAAGCTCAGCAAATCCTACATTAATGATAAAGGGAAAGAAG TCCTCGTGCTATGGGGCATTCACCATCCATCTACTAGTGCTGACCAACAAAGTCTCTATC AGAATGCAGATGCATATGTTTTTGTGGGGTCATCAAGATACAGCAAGAAGTTCAAGCCGG AAATAGCAATAAGACCCAAAGTGAGGGATCAAGAAGGGAGAATGAACTATTACTGGACAC TAGTAGAGCCGGGAGACAAAATAACATTCGAAGCAACTGGAAATCTAGTGGTACCGAGAT ATGCATTCGCAATGGAAAGAAATGCTGGATCTGGTATTATCATTTCAGATACACCAGTCC ACGATTGCAATACAACTTGTCAGACACCCAAGGGTGCTATAAACACCAGCCTCCCATTTC |
| | AGAATATACATCCGATCACAATTGGAAAATGTCCAAAATATGTAAAAAGCACAAAATTGA GACTGGCCACAGGATTGAGGAATGTCCCGTCTATTCAATCTAGAGGCCTATTTGGGGCCA TTGCCGGTTTCATTGAAGGGGGGTGGACAGGGATGGTAGATGGATGGTACGGTTATCACC ATCAAAATGAGCAGGGGTCAGGATATGCAGCCGACCTGAAGAGCACACAGAATGCCATTG ACGAGATTACTAACAAAGTAAATTCTGTTATTGAAAAGATGAATACACAGTTCACAGCAG TAGGTAAAGAGTTCAACCACCTGGAAAAAAGAATAGAGAATTTAAATAAAAAAGTTGATG ATGGTTTCCTGGACATTTGGACTTACAATGCCGAACTGTTGGTTCTATTGGAAAATGAAA GAACTTTGGACTACCACGATTCAAATGTGAAGAACTTATATGAAAAGGTAAGAAGCCAGC TAAAAAACAATGCCAAGGAAATTGGAAACGGCTGCTTTGAATTTTACCACAAATGCGATA ACACGTGCATGGAAAGTGTCAAAAATGGGACTTATGACTACCCAAAATACTCAGAGGAAG CAAAATTAAACAGAGAAGAAATAGATGGGGTAAAGCTGGAATCAACAAGGATTTACCAGA TTTTGGCGATCTATTCAACTGTCGCCAGTTCATTGGTACTGGTAGTCTCCCTGGGGGCAA TCAGTTTCTGGATGTGCTCTAATGGGTCTCTACAGTGTAGAATATGTATTTAACATTAGG ATTTCAGAAGCATGAGAAAAACACCCTTGTTTCTACT |
| | Genome segment 5/NP AGCAAAAGCAGGGTAGATAATCACTCAATGGCGTCTCAAGGCACCAAACGATCATATGAA CAAATGGAGACTGGTGGGGAGCGCCAGGATGCCACAGAAATCAGAGCATCTGTCGGAAGA ATGATTGGTGGAATCGGGAGATTCTACATCCAAATGTGCACTGAACTCAAACTCAGTGAT TATGATGGACGACTAATCCAGAATAGCATAACAATAGAGAGGATGGTGCTTTCTGCTTTT GATGAGAGAAGAAATAAATACCTAGAAGAGCATCCCAGTGCTGGGAAGGACCCCAAGAAA ACAGGAGGACCCATATATAGAAGAGTAGACGGAAAGTGGATGAGAGAACTCATCCTTTAT GACAAAGAAGAAATAAGGAGAGTTTGGCGCCAAGCAAACAATGGCGAAGATGCAACAGCA GGTATTACTCATATCATGATTTGGCATTCCAACCTGAATGATGCCACATATCAGAGAACA AGAGCGCTTGTTCGCACCGGAATGGATCCCAGAATGTGCTCTCTAATGCAAGGTTCAACA CTTCCCAGAAGGTCTGGTGCCGCAGGTGCTGCGGTGAAAGGAGTTGGAACAATAGCAATG GAGTTAATCAGAATGATCAAACGTGGAATCAATGACCGAAATTTCTGGAGGGGTGAAAAT GGACGAAGGACAAGGGTTGCTTATGAAAGAATGTGCAATATCCTCAAAGGAAAATTTCAA ACAGCTGCCCAGAGGGCAATGATGGATCAAGTAAGAGAAAGTCGAAACCCAGGAAACGCT GAGATTGAAGACCTCATTTTCCTGGCACGGTCAGCACTCATTCTGAGGGGATCAGTTGCA CATAAATCCTGCCTGCCTGCTTGTGTGTATGGGCTTGCAGTAGCAAGTGGGCATGACTTT GAAAGGGAAGGGTACTCACTGGTCGGGATAGACCCATTCAAATTACTCCAAAACAGCCAA GTGGTCAGCCTGATGAGACCAAATGAAAACCCAGCTCACAAGAGTCAATTGGTGTGGATG GCATGCCACTCTGCTGCATTTGAAGATTTAAGAGTATCAAGTTTCATAAGAGGAAAGAAA GTGATTCCAAGAGGAAAGCTTTCCACAAGAGGGGTCCAGATTGCTTCAAATGAGAATGTG GAAATCATGGACTCCAATACCCTGGAACTGAGAAGCAGATACTGGGCCATAAGGACCAGG AGTGGAGGAAATACCAATCAACAAAAGGCATCCGCAGGCCAGATCAGTGTGCAGCCTACA TTCTCAGTGCAGCGGAATCTCCCTTTTGAAAGAGCAACCGTTATGGCAGCATTCAGCGGG AACAATGAAGGACGGACATCCGACATGCGAACAGAAGTTATAAGAATGATGGAAAGTGCA AAGCCAGAAGATTTGTCCTTCCAGGGGCGGGGAGTCTTCGAGCTCTCGGACGAAAAGGCA ACGAACCCGATCGTGCCTTCCTTTGACATGAGTAATGAAGGGTCTTATTTCTTCGGAGAC AATGCAGAGGAGTATGACAGTTGAGGAAAAATACCCTTGTTTCTACT |
| | Genome segment 6/NA AGCAAAAGCAGGAGTTTAAAATGAATCCAAACCAAAAGATAATAACCATTGGTTCGGTCT GTATGACAATTGGAATGGCTAACTTAATATTACAAATTGGAAACATAATCTCAACATGGA TTAGCCACTCAATTCAACTTGGGAATCAAAATCAGATTGAAACATGCAATCAAAGCGTCA TTACTTATGAAAACAACACTTGGGTAAATCAGACATATGTTAACATCAGCAACACCAACT TTGCTGCTGGACAGTCAGTGGTTTCCGTGAAATTAGCGGGCAATTCCTCTCTCTGCCCTG TTAGTGGATGGGCTATATACAGTAAAGACAACAGCGTAAGAATCGGTTCCAAGGGGGATG TGTTTGTCATAAGGGAACCATTCATATCATGCTCCCCCTTGGAATGCAGAACCTTCTTCT TGACTCAAGGGGCCTTGCTAAATGACAAACATTCCAATGGAACCATTAAAGACAGGAGCC CATATCGAACCCTAATGAGCTGTCCTATTGGTGAAGTTCCCTCTCCATACAACTCAAGAT TTGAGTCAGTCGCTTGGTCAGCAAGTGCTTGTCATGATGGCATCAATTGGCTAACAATTG GAATTTCTGGCCCAGACAATGGGGCAGTGGCTGTGTTAAAGTACAACGGCATAATAACAG ACACTATCAAGAGTTGGAGAAACAATATATTGAGAACACAAGAGTCTGAATGTGCATGTG TAAATGGTTCTTGCTTTACTGTAATGACCGATGGACCAAGTAATGGACAGGCCTCATACA AGATCTTCAGAATAGAAAAGGGAAAAATAGTCAAATCAGTCGAAATGAATGCCCCTAATT ATCACTATGAGGAATGCTCCTGTTATCCTGATTCTAGTGAAATCACATGTGTGTGCAGGG ATAACTGGCATGGCTCGAATCGACCGTGGGTGTCTTTCAACCAGAATCTGGAATATCAGA TAGGATACATATGCAGTGGGATTTTCGGAGACAATCCACGCCCTAATGATAAGACAGGCA GTTGTGGTCCAGTATCGTCTAATGGAGCAAATGGAGTAAAAGGATTTTCATTCAAATACG GCAATGGTGTTTGGATAGGGAGAACTAAAAGCATTAGTTCAAGAAACGGTTTTGAGATGA TTTGGGATCCGAACGGATGGACTGGGACAGACAATAACTTCTCAATAAAGCAAGATATCG TAGGAATAAATGAGTGGTCAGGATATAGCGGGAGTTTTGTTCAGCATCCAGAACTAACAG GGCTGGATTGTATAAGACCTTGCTTCTGGGTTGAACTAATCAGAGGGCGACCCAAAGAGA ACACAATCTGGACTAGCGGGAGCAGCATATCCTTTTGTGGTGTAAACAGTGACACTGTGG GTTGGTCTTGGCCAGACGGTGCTGAGTTGCCATTTACCATTGACAAGTAATTTGTTCAAA |
| | AAACTCCTTGTTTCTACT |
| | Genome segment 7/M AGCAAAAGCAGGTAGATATTTAAAGATGAGTCTTCTAACCGAGGTCGAAACGTACGTTCT TTCTATCATCCCGTCAGGCCCCCTCAAAGCCGAGATCGCGCAGAGACTGGAAAGTGTCTT TGCAGGAAAGAACACAGATCTTGAGGCTCTCATGGAATGGCTAAAGACAAGACCAATCTT GTCACCTCTGACTAAGGGAATTTTAGGATTTGTGTTCACGCTCACCGTGCCCAGTGAGCG AGGACTGCAGCGTAGACGCTTTGTCCAAAATGCCCTAAATGGGAATGGGGACCCGAACAA CATGGATAGAGCAGTTAAACTATACAAGAAGCTCAAAAGAGAAATAACGTTCCATGGGGC CAAGGAGGTGTCACTAAGCTATTCAACTGGTGCACTTGCCAGTTGCATGGGCCTCATATA CAACAGGATGGGAACAGTGACCACAGAAGCTGCTTTTGGTCTAGTGTGTGCCACTTGTGA ACAGATTGCTGATTCACAGCATCGGTCTCACAGACAGATGGCTACTACCACCAATCCACT AATCAGGCATGAAAACAGAATGGTGCTGGCTAGCACTACGGCAAAGGCTATGGAACAGAT GGCTGGATCGAGTGAACAGGCAGCGGAGGCCATGGAGGTTGCTAATCAGACTAGGCAGAT GGTACATGCAATGAGAACTATTGGGACTCATCCTAGCTCCAGTGCTGGTCTGAAAGATGA CCTTCTTGAAAATTTGCAGGCCTACCAGAAGCGAATGGGAGTGCAGATGCAGCGATTCAA GTGATCCTCTCGTCATTGCAGCAAATATCATTGGGATCTTGCACCTGATATTGTGGATTA CTGATCGTCTTTTTTTCAAATGTATTTATCGTCGCTTTAAATACGGTTTGAAAAGAGGGC CTTCTACGGAAGGAGTGCCTGAGTCCATGAGGGAAGAATATCAACAGGAACAGCAGAGTG CTGTGGATGTTGACGATGGTCATTTTGTCAACATAGAGCTGGAGTAAAAAACTACCTTGT TTCTACT |
| | genome segment 8/NS1(1-126) AGCAAAAGCAGGGTGACAAAAACATAATGGACTCCAACACCATGTCAAGCTTTCAGGTAG ACTGTTTCCTTTGGCATATCCGCAAGCGATTTGCAGACAATGGATTGGGTGATGCCCCAT TCCTTGATCGGCTCCGCCGAGATCAAAAGTCCTTAAAAGGAAGAGGCAACACCCTTGGCC TCGATATCGAAACAGCCACTCTTGTTGGGAAACAAATCGTGGAATGGATCTTGAAAGAGG AATCCAGCGAGACACTTAGAATGACAATTGCATCTGTACCTACTTCGCGCTACCTTTCTG ACATGACCCTCGAGGAAATGTCACGAGACTGGTTCATGCTCATGCCTAGGCAAAAGATAA TAGGCCCTCTTTGCGTGCGATTGGACCAGGCGATCATGGAAAAGTAGATCTTGATTAATT AAGAGGGAGCAATAGTTGGAGAAATTTCACCATTACCTTCTCTTCCAGGACATACTTATG AGGATGTCAAAAATGCAGTTGGGGTCCTCATCGGAGGACTTGAATGGAATGGTAACACGG TTCGAGTCTCTGAAAATATACAGAGATTCGCTTGGAGAAACTGTGATGAGAATGGGAGAC CTTCACTACCTCCAGAGCAGAAATGAAAAGTGGCGAGAGCAATTGGGACAGAAATTTGAG GAAATAAGGTGGTTAATTGAAGAAATGCGGCACAGATTGAAAGCGACAGAGAATAGTTTC GAACAAATAACATTTATGCAAGCCTTACAACTATTGCTTGAAGTAGAACAAGAGATAAGA GCTTTCTCGTTTCAGCTTATTTAATGATAAAAAACACCCTTGTTTCTACT |
| 3 (NS1(1-126)-ΔPAX) | Genome segment 1/PB2 AGCGAAAGCAGGTCAAATATATTCAATATGGAGAGAATAAAAGAACTGAGAGATCTAATG TCGCAGTCCCGCACTCGCGAGATACTCACTAAGACCACTGTGGACCATATGGCCATAATC AAAAAGTACACATCAGGAAGGCAAGAGAAGAACCCCGCACTCAGAATGAAGTGGATGATG GCAATGAGATACCCAATTACAGCAGACAAGAGAATAATGGACATGATTCCAGAGAGGAAT GAACAAGGACAAACCCTCTGGAGCAAAACAAACGATGCTGGATCAGACCGAGTGATGGTA TCACCTCTGGCCGTAACATGGTGGAATAGGAATGGCCCAACAACAAGTACAGTTCATTAC CCTAAGGTATATAAAACTTATTTCGAAAAGGTCGAAAGGTTGAAACATGGTACCTTCGGC CCTGTCCACTTCAGAAATCAAGTTAAAATAAGGAGGAGAGTTGATACAAACCCTGGCCAT GCAGATCTCAGTGCCAAGGAGGCACAGGATGTGATTATGGAAGTTGTTTTCCCAAATGAA GTGGGGGCAAGAATACTGACATCAGAGTCACAGCTGGCAATAACAAAAGAGAAGAAAGAA GAGCTCCAGGATTGTAAAATTGCTCCCTTGATGGTGGCGTACATGCTAGAAAGAGAATTG GTCCGTAAAACAAGGTTTCTCCCAGTAGCCGGCGGAACAGGCAGTGTTTATATTGAAGTG TTGCACTTAACCCAAGGGACGTGCTGGGAGCAGATGTACACTCCAGGAGGAGAAGTGAGA AATGATGATGTTGACCAAAGTTTGATTATCGCTGCTAGAAACATAGTAAGAAGAGCAGCA GTGTCAGCAGACCCATTAGCATCTCTCTTGGAAATGTGCCACAGCACACAGATTGGAGGA GTAAGGATGGTGGACATCCTTAGACAGAATCCAACTGAGGAACAAGCCGTAGACATATGC AAGGCAGCAATAGGGTTGAGGATTAGCTCATCTTTCAGTTTTGGTGGGTTCACTTTCAAA AGGACAAGCGGATCATCAGTCAAGAAAGAAGAAGAAGTGCTAACGGGCAACCTCCAAACA CTGAAAATAAGAGTACATGAAGGGTATGAAGAATTCACAATGGTTGGGAGAAGAGCAACA GCTATTCTCAGAAAGGCAACCAGGAGATTGATCCAGTTGATAGTAAGCGGGAGAGACGAG CAGTCAATTGCTGAGGCAATAATTGTGGCCATGGTATTCTCACAAGAGGATTGCATGATC AAGGCAGTTAGGGGCGATCTGAACTTTGTCAATAGGGCAAACCAGCGACTGAACCCCATG CACCAACTCTTGAGGCATTTCCAAAAAGATGCAAAAGTGCTTTTCCAGAACTGGGGAATT GAATCCATCGACAATGTGATGGGAATGATCGGAATACTGCCCGACATGACCCCAAGCACG GAGATGTCGCTGAGAGGGATAAGAGTCAGCAAAATGGGAGTAGATGAATACTCCAGCACG GAGAGAGTGGTAGTGAGTATTGACCGATTTTTAAGGGTTAGAGATCAAAGAGGGAACGTA CTATTGTCTCCCGAAGAAGTCAGTGAAACGCAAGGAACTGAGAGGTTGACAATAACTTAT TCGTCATCAATGATGTGGGAGATCAATGGCCCTGAGTCAGTGCTAGTCAACACTTATCAA TGGATAATCAGGAACTGGGAAATTGTGAAAATTCAATGGTCACAAGATCCCACAATGTTA |
| | TACAACAAAATGGAATTTGAACCATTTCAGTCTCTTGTCCCTAAGGCAACCAGAAGCCGG TACAGTGGATTCGTAAGGACACTGTTCCAGCAAATGCGGGATGTGCTTGGGACATTTGAC ACTGTCCAAATAATAAAACTTCTCCCCTTTGCTGCTGCTCCACCAGAACAGAGTAGGATG CAATTTTCCTCATTGACTGTGAATGTGAGAGGATCAGGGTTGAGGATACTGGTAAGAGGC AATTCTCCAGTATTCAATTACAACAAGGCAACCAAACGACTTACAGTTCTTGGAAAGGAT GCAGGTGCATTGACTGAAGATCCAGATGAAGGCACATCTGGGGTGGAGTCTGCTGTCCTG AGAGGATTTCTCATTTTGGGCAAAGAAGACAAGAGATATGGCCCAGCATTAAGCATCAAT GAACTGAGCAATCTTGCAAAAGGAGAGAAGGCTAATGTGCTAATTGGGCAAGGGGACGTA GTGTTGGTAATGAAACGAAAACGGGACTCTAGCATACTTACTGACAGCCAGACAGCGACC AAAAGAATTCGGATGGCCATCAATTAGTGTCGAATTGTTTAAAAACGACCTTGTTTCTAC T |
| | Genome segment 2/PB1 AGCGAAAGCAGGCAAACCATTTGAATGGATGTCAATCCGACTCTACTTTTCCTAAAAATT CCAGCGCAAAATGCCATAAGCACCACATTCCCTTATACTGGAGATCCTCCATACAGCCAT GGAACAGGAACAGGATACACCATGGACACAGTAAACAGAACACACCAATACTCAGAAAAG GGAAAGTGGACGACAAACACAGAGACTGGTGCACCCCAGCTCAACCCGATTGATGGACCA CTACCTGAGGATAATGAACCAAGTGGGTATGCACAAACAGACTGTGTTCTAGAGGCTATG GCTTTCCTTGAAGAATCCCACCCAGGAATATTTGAGAATTCATGCCTTGAAACAATGGAA GTTGTTCAACAAACAAGGGTAGATAAACTAACTCAAGGTCGCCAGACTTATGATTGGACA TTAAACAGAAATCAACCGGCAGCAACTGCATTGGCCAACACCATAGAAGTCTTTAGATCG AATGGCCTAACAGCTAATGAGTCAGGAAGGCTAATAGATTTCTTAAAGGATGTAATGGAA TCAATGAACAAAGAGGAAATAGAGATAACAACCCACTTTCAAAGAAAAAGGAGAGTAAGA GACAACATGACCAAGAAGATGGTCACGCAAAGAACAATAGGGAAGAAAAAACAAAGACTG AATAAGAGAGGCTATCTAATAAGAGCACTGACATTAAATACGATGACCAAAGATGCAGAG AGAGGCAAGTTAAAAAGAAGGGCTATCGCAACACCTGGGATGCAGATTAGAGGTTTCGTA TACTTTGTTGAAACTTTAGCTAGGAGCATTTGCGAAAAGCTTGAACAGTCTGGGCTCCCA GTAGGGGGCAATGAAAAGAAGGCCAAACTGGCAAATGTTGTGAGAAAGATGATGACTAAT TCACAAGACACAGAGATTTCTTTCACAATCACTGGGGACAACACTAAGTGGAATGAAAAT CAAAATCCTCGAGTGTTCCTGGCGATGATTACATATATCACCAGAAATCAACCCGAGTGG TTCAGAAACATCCTGAGCATGGCACCCATAATGTTCTCAAACAAAATGGCAAGACTAGGG AAAGGGTACATGTTCGAGAGTAAAAGAATGAAGATTCGAACACAAATACCAGCAGAAATG CTAGCAAGCATTGACCTGAAGTACTTCAATGAATCAACAAAGAAGAAAATTGAGAAAATA AGGCCTCTTCTAATAGATGGCACAGCATCACTGAGTCCTGGGATGATGATGGGCATGTTC AACATGCTAAGTACGGTCTTGGGAGTCTCGATACTGAATCTTGGACAAAAGAAATACACC AAGACAATATACTGGTGGGATGGGCTCCAATCATCCGACGATTTTGCTCTCATAGTGAAT GCACCAAACCATGAGGGAATACAAGCAGGAGTGGACAGATTCTACAGGACCTGCAAGTTA GTGGGAATCAACATGAGCAAAAAGAAGTCCTATATAAATAAGACAGGGACATTTGAATTC ACAAGCTTTTTTTATCGCTATGGATTTGTGGCTAATTTTAGCATGGAGCTACCCAGCTTT GGAGTGTCTGGAGTAAATGAATCAGCTGACATGAGTATTGGAGTAACAGTGATAAAGAAC AACATGATAAACAATGACCTTGGACCTGCAACGGCCCAGATGGCTCTTCAATTGTTCATC AAAGACTACAGATACACATATAGGTGCCATAGGGGAGACACACAAATTCAGACGAGAAGA TCATTTGAGTTAAAGAAGCTGTGGGATCAAACCCAATCAAAGGTAGGGCTATTAGTATCA GATGGAGGACCAAACTTATACAATATACGGAATCTTCACATTCCTGAAGTCTGCTTAAAA TGGGAGCTAATGGATGATGATTATCGGGGAAGACTTTGTAATCCCCTGAATCCCTTTGTC AGTCATAAAGAGATTGATTCTGTAAACAATGCTGTGGTAATGCCAGCCCATGGTCCAGCC AAAAGCATGGAATATGATGCCGTTGCAACTACACATTCCTGGATTCCCAAGAGGAATCGT TCTATTCTCAACACAAGCCAAAGGGGAATTCTTGAGGATGAACAGATGTACCAGAAGTGC TGCAATCTATTCGAGAAATTTTTCCCTAGCAGTTCATATAGGAGACCGGTTGGAATTTCT AGCATGGTGGAGGCCATGGTGTCTAGGGCCCGGATTGATGCCAGGGTCGACTTCGAGTCT GGACGGATCAAGAAAGAAGAGTTCTCTGAGATCATGAAGATCTGTTCCACCATTGAAGAA CTCAGACGGCAAAAATAATGAATTTAGCTTGTCCTTCATGAAAAAATGCCTTGTTTCTAC T |
| | Genome segment 3/PA-ΔPAX AGCAAAAGCAGGTACTGATCCAAAATGGAAGACTTTGTGCGACAATGCTTCAATCCAATG ATCGTCGAGCTTGCGGAAAAGGCAATGAAAGAATATGGGGAAGATCCGAAAATCGAAACT AACAAGTTTGCTGCAATATGCACACATTTGGAAGTTTGTTTCATGTATTCGGATTTCCAT TTCATCGACGAACGGGGTGAATCAATAATTGTAGAATCTGGTGACCCGAATGCACTATTG AAGCACCGATTTGAGATAATTGAAGGAAGAGACCGAATCATGGCCTGGACAGTGGTGAAC AGTATATGTAACACAACAGGGGTAGAGAAGCCTAAATTTCTTCCTGATTTGTATGATTAC AAAGAGAACCGGTTCATTGAAATTGGAGTAACACGGAGGGAAGTCCACATATATTACCTA GAGAAAGCCAACAAAATAAAATCTGAGAAGACACACATTCACATCTTTTCATTCACTGGA GAGGAGATGGCCACCAAAGCGGACTACACCCTTGACGAAGAGAGCAGGGCAAGAATCAAA ACTAGGCTTTTCACTATAAGACAAGAAATGGCCAGTAGGAGTCTATGGGATTCGTTCAGA CAGTCCGAAAGAGGCGAAGAGACAATTGAAGAAAAATTTGAGATTACAGGAACTATGCGC AAGCTTGCCGACCAAAGTCTCCCACCGAACTTCTCCAGCCTTGAAAACTTTAGAGCCTAT GTAGATGGATTCGAGCCGAACGGCTGCATTGAGGGCAAGCTTTCCCAAATGTCAAAAGAA |
| | GTGAACGCCAAAATTGAACCATTCTTGAGGACGACACCACGCCCCCTCAGATTGCCTGATG GGCCTCTTTGCCATCAGCGGTCAAAGTTCCTGCTGATGGATGCTCTGAAATTAAGTATT GAAGACCCGAGTCACGAGGGGGAGGGAATACCACTATATGATGCAATCAAATGCATGAAG ACATTCTTTGGCTGGAAAGAGCCTAACATAGTCAAACCACATGAGAAAGGCATAAATCCC AATTACCTCATGGCTTGGAAGCAGGTGCTAGCAGAGCTACAGGACATTGAAAATGAAGAG AAGATCCCAAGGACAAAGAACATGAAGAGAACAAGCCAATTGAAGTGGGCACTCGGTGAA AATATGGCACCAGAAAAAGTAGACTTTGATGACTGCAAAGATGTTGGAGACCTTAAACAG TATGACAGTGATGAGCCAGAGCCCAGATCTCTAGCAAGCTGGGTCCAAAATGAATTCAAT AAGGCATGTGAATTGACTGATTCAAGCTGGATAGAACTTGATGAAATAGGAGAAGATGTT GCCCCGATTGAACATATCGCAAGCATGAGGAGGAACTATTTTACAGCAGAAGTGTCCCAC TGCAGGGCTACTGAATACATAATGAAGGGAGTGTACATAAATACGGCCTTGCTCAATGCA TCCTGTGCAGCCATGGATGACTTTCAGCTGATCCCAATGATAAGCAAATGTAGGACCAAA GAAGGAAGACGGAAAACAAACCTGTATGGGTTCATTATAAAAGGAAGGTCTCATTTGAGA AATGATACTGATGTGGTGAACTTTGTAAGTATGGAGTTCTCACTCACTGACCCGAGACTG GAGCCACACAAATGGGAAAAATACTGTGTTCTTGAAATAGGAGACATGCTCTTGAGGACT GCGATAGGCCAAGTGTCGAGGCCCATGTTCCTATATGTGAGAACCAATGGAACCTCCAAG ATCAAGATGAAATGGGGCATGGAACTGAGGCGCTGCCTTCTTCAGTCTCTTCAGCAGATT GAGAGCATGATTGAGGCCGAGTCTTCTGTCAAAGAGAAAGACATGACCAAGGAATTCTTT GAAAACAAATCGGAAACATGGCCAATCGGAGAGTCACCCAGGGGAGTGGAGGAAGGCTCT ATTGGGAAAGTGTGCAGGACCTTACTGGCAAAATCTGTATTCAACAGTCTATATGCGTCT CCACAACTTGAGGGGTTTTCGGCTGAATCGAGAAAATTGCTTCTCATTGTTCAGGCACTT AGGGACAACCTGGAACCTGGAACCTTCGATCTTGGGGGGCTATATGAAGCAATCGAGGAG TGCCTGATTAATGATCCCTGGGTTTTGCTTAATGCATCTTGGTTCAACTCCTTCCTCACA CATGCACTGAAGTAGTTGTGGCAATGCTACTATTTGCTATCCATACTGTCCAAAAAAGTA CCTTGTTTCTACT |
| | Genome segment 4/HA AGCAAAAGCAGGGGAAAACAAAAGCAACAAAAATGAAGGCAATACTAGTAGTTCTGCTAT ATACATTTACAACCGCAAATGCAGACACATTATGTATAGGTTATCATGCGAACAATTCAA CAGACACTGTAGACACAGTACTAGAAAAGAATGTAACAGTAACACACTCTGTTAACCTTC TAGAAGACAAGCATAACGGGAAACTATGCAAACTAAGAGGGGTAGCCCCATTGCATTTGG GTAAATGTAACATTGCTGGCTGGATCCTGGGAAATCCAGAGTGTGAATCACTCTCCACAG CAAGCTCATGGTCCTACATTGTGGAAACATCTAGTTCAGACAATGGAACGTGTTACCCAG GAGATTTCATCGATTATGAGGAGCTAAGAGAGCAATTGAGCTCAGTGTCATCATTTGAAA GGTTTGAGATATTCCCCAAGACAAGTTCATGGCCCAATCATGACTCGAACAAAGGTGTAA CGGCAGCATGTCCTCATGCTGGAGCAAAAAGCTTCTACAAAAATTTAATATGGCTAGTTA AAAAAGGAAATTCATACCCAAAGCTCAGCAAATCCTACATTAATGATAAAGGGAAAGAAG TCCTCGTGCTATGGGGCATTCACCATCCATCTACTAGTGCTGACCAACAAAGTCTCTATC AGAATGCAGATGCATATGTTTTTGTGGGGTCATCAAGATACAGCAAGAAGTTCAAGCCGG AAATAGCAATAAGACCCAAAGTGAGGGATCAAGAAGGGAGAATGAACTATTACTGGACAC TAGTAGAGCCGGGAGACAAAATAACATTCGAAGCAACTGGAAATCTAGTGGTACCGAGAT ATGCATTCGCAATGGAAAGAAATGCTGGATCTGGTATTATCATTTCAGATACACCAGTCC ACGATTGCAATACAACTTGTCAGACACCCAAGGGTGCTATAAACACCAGCCTCCCATTTC AGAATATACATCCGATCACAATTGGAAAATGTCCAAAATATGTAAAAAGCACAAAATTGA GACTGGCCACAGGATTGAGGAATGTCCCGTCTATTCAATCTAGAGGCCTATTTGGGGCCA TTGCCGGTTTCATTGAAGGGGGGTGGACAGGGATGGTAGATGGATGGTACGGTTATCACC ATCAAAATGAGCAGGGGTCAGGATATGCAGCCGACCTGAAGAGCACACAGAATGCCATTG ACGAGATTACTAACAAAGTAAATTCTGTTATTGAAAAGATGAATACACAGTTCACAGCAG TAGGTAAAGAGTTCAACCACCTGGAAAAAAGAATAGAGAATTTAAATAAAAAAGTTGATG ATGGTTTCCTGGACATTTGGACTTACAATGCCGAACTGTTGGTTCTATTGGAAAATGAAA GAACTTTGGACTACCACGATTCAAATGTGAAGAACTTATATGAAAAGGTAAGAAGCCAGC TAAAAAACAATGCCAAGGAAATTGGAAACGGCTGCTTTGAATTTTACCACAAATGCGATA ACACGTGCATGGAAAGTGTCAAAAATGGGACTTATGACTACCCAAAATACTCAGAGGAAG CAAAATTAAACAGAGAAGAAATAGATGGGGTAAAGCTGGAATCAACAAGGATTTACCAGA TTTTGGCGATCTATTCAACTGTCGCCAGTTCATTGGTACTGGTAGTCTCCCTGGGGGCAA TCAGTTTCTGGATGTGCTCTAATGGGTCTCTACAGTGTAGAATATGTATTTAACATTAGG ATTTCAGAAGCATGAGAAAAACACCCTTGTTTCTACT |
| | Genome segment 5/NP AGCAAAAGCAGGGTAGATAATCACTCAATGGCGTCTCAAGGCACCAAACGATCATATGAA CAAATGGAGACTGGTGGGGAGCGCCAGGATGCCACAGAAATCAGAGCATCTGTCGGAAGA ATGATTGGTGGAATCGGGAGATTCTACATCCAAATGTGCACTGAACTCAAACTCAGTGAT TATGATGGACGACTAATCCAGAATAGCATAACAATAGAGAGGATGGTGCTTTCTGCTTTT GATGAGAGAAGAAATAAATACCTAGAAGAGCATCCCAGTGCTGGGAAGGACCCCAAGAAA ACAGGAGGACCCATATATAGAAGAGTAGACGGAAAGTGGATGAGAGAACTCATCCTTTAT GACAAAGAAGAAATAAGGAGAGTTTGGCGCCAAGCAAACAATGGCGAAGATGCAACAGCA GGTATTACTCATATCATGATTTGGCATTCCAACCTGAATGATGCCACATATCAGAGAACA AGAGCGCTTGTTCGCACCGGAATGGATCCCAGAATGTGCTCTCTAATGCAAGGTTCAACA |
| | CTTCCCAGAAGGTCTGGTGCCGCAGGTGCTGCGGTGAAAGGAGTTGGAACAATAGCAATG GAGTTAATCAGAATGATCAAACGTGGAATCAATGACCGAAATTTCTGGAGGGGTGAAAAT GGACGAAGGACAAGGGTTGCTTATGAAAGAATGTGCAATATCCTCAAAGGAAAATTTCAA ACAGCTGCCCAGAGGGCAATGATGGATCAAGTAAGAGAAAGTCGAAACCCAGGAAACGCT GAGATTGAAGACCTCATTTTCCTGGCACGGTCAGCACTCATTCTGAGGGGATCAGTTGCA CATAAATCCTGCCTGCCTGCTTGTGTGTATGGGCTTGCAGTAGCAAGTGGGCATGACTTT GAAAGGGAAGGGTACTCACTGGTCGGGATAGACCCATTCAAATTACTCCAAAACAGCCAA GTGGTCAGCCTGATGAGACCAAATGAAAACCCAGCTCACAAGAGTCAATTGGTGTGGATG GCATGCCACTCTGCTGCATTTGAAGATTTAAGAGTATCAAGTTTCATAAGAGGAAAGAAA GTGATTCCAAGAGGAAAGCTTTCCACAAGAGGGGTCCAGATTGCTTCAAATGAGAATGTG GAAATCATGGACTCCAATACCCTGGAACTGAGAAGCAGATACTGGGCCATAAGGACCAGG AGTGGAGGAAATACCAATCAACAAAAGGCATCCGCAGGCCAGATCAGTGTGCAGCCTACA TTCTCAGTGCAGCGGAATCTCCCTTTTGAAAGAGCAACCGTTATGGCAGCATTCAGCGGG AACAATGAAGGACGGACATCCGACATGCGAACAGAAGTTATAAGAATGATGGAAAGTGCA AAGCCAGAAGATTTGTCCTTCCAGGGGCGGGGAGTCTTCGAGCTCTCGGACGAAAAGGCA ACGAACCCGATCGTGCCTTCCTTTGACATGAGTAATGAAGGGTCTTATTTCTTCGGAGAC AATGCAGAGGAGTATGACAGTTGAGGAAAAATACCCTTGTTTCTACT |
| | Genome segment 6/NA AGCAAAAGCAGGAGTTTAAAATGAATCCAAACCAAAAGATAATAACCATTGGTTCGGTCT GTATGACAATTGGAATGGCTAACTTAATATTACAAATTGGAAACATAATCTCAACATGGA TTAGCCACTCAATTCAACTTGGGAATCAAAATCAGATTGAAACATGCAATCAAAGCGTCA TTACTTATGAAAACAACACTTGGGTAAATCAGACATATGTTAACATCAGCAACACCAACT TTGCTGCTGGACAGTCAGTGGTTTCCGTGAAATTAGCGGGCAATTCCTCTCTCTGCCCTG TTAGTGGATGGGCTATATACAGTAAAGACAACAGCGTAAGAATCGGTTCCAAGGGGGATG TGTTTGTCATAAGGGAACCATTCATATCATGCTCCCCCTTGGAATGCAGAACCTTCTTCT TGACTCAAGGGGCCTTGCTAAATGACAAACATTCCAATGGAACCATTAAAGACAGGAGCC CATATCGAACCCTAATGAGCTGTCCTATTGGTGAAGTTCCCTCTCCATACAACTCAAGAT TTGAGTCAGTCGCTTGGTCAGCAAGTGCTTGTCATGATGGCATCAATTGGCTAACAATTG GAATTTCTGGCCCAGACAATGGGGCAGTGGCTGTGTTAAAGTACAACGGCATAATAACAG ACACTATCAAGAGTTGGAGAAACAATATATTGAGAACACAAGAGTCTGAATGTGCATGTG TAAATGGTTCTTGCTTTACTGTAATGACCGATGGACCAAGTAATGGACAGGCCTCATACA AGATCTTCAGAATAGAAAAGGGAAAAATAGTCAAATCAGTCGAAATGAATGCCCCTAATT ATCACTATGAGGAATGCTCCTGTTATCCTGATTCTAGTGAAATCACATGTGTGTGCAGGG ATAACTGGCATGGCTCGAATCGACCGTGGGTGTCTTTCAACCAGAATCTGGAATATCAGA TAGGATACATATGCAGTGGGATTTTCGGAGACAATCCACGCCCTAATGATAAGACAGGCA GTTGTGGTCCAGTATCGTCTAATGGAGCAAATGGAGTAAAAGGATTTTCATTCAAATACG GCAATGGTGTTTGGATAGGGAGAACTAAAAGCATTAGTTCAAGAAACGGTTTTGAGATGA TTTGGGATCCGAACGGATGGACTGGGACAGACAATAACTTCTCAATAAAGCAAGATATCG TAGGAATAAATGAGTGGTCAGGATATAGCGGGAGTTTTGTTCAGCATCCAGAACTAACAG GGCTGGATTGTATAAGACCTTGCTTCTGGGTTGAACTAATCAGAGGGCGACCCAAAGAGA ACACAATCTGGACTAGCGGGAGCAGCATATCCTTTTGTGGTGTAAACAGTGACACTGTGG GTTGGTCTTGGCCAGACGGTGCTGAGTTGCCATTTACCATTGACAAGTAATTTGTTCAAA AAACTCCTTGTTTCTACT |
| | Genome segment 7/M AGCAAAAGCAGGTAGATATTTAAAGATGAGTCTTCTAACCGAGGTCGAAACGTACGTTCT TTCTATCATCCCGTCAGGCCCCCTCAAAGCCGAGATCGCGCAGAGACTGGAAAGTGTCTT TGCAGGAAAGAACACAGATCTTGAGGCTCTCATGGAATGGCTAAAGACAAGACCAATCTT GTCACCTCTGACTAAGGGAATTTTAGGATTTGTGTTCACGCTCACCGTGCCCAGTGAGCG AGGACTGCAGCGTAGACGCTTTGTCCAAAATGCCCTAAATGGGAATGGGGACCCGAACAA CATGGATAGAGCAGTTAAACTATACAAGAAGCTCAAAAGAGAAATAACGTTCCATGGGGC CAAGGAGGTGTCACTAAGCTATTCAACTGGTGCACTTGCCAGTTGCATGGGCCTCATATA CAACAGGATGGGAACAGTGACCACAGAAGCTGCTTTTGGTCTAGTGTGTGCCACTTGTGA ACAGATTGCTGATTCACAGCATCGGTCTCACAGACAGATGGCTACTACCACCAATCCACT AATCAGGCATGAAAACAGAATGGTGCTGGCTAGCACTACGGCAAAGGCTATGGAACAGAT GGCTGGATCGAGTGAACAGGCAGCGGAGGCCATGGAGGTTGCTAATCAGACTAGGCAGAT GGTACATGCAATGAGAACTATTGGGACTCATCCTAGCTCCAGTGCTGGTCTGAAAGATGA CCTTCTTGAAAATTTGCAGGCCTACCAGAAGCGAATGGGAGTGCAGATGCAGCGATTCAA GTGATCCTCTCGTCATTGCAGCAAATATCATTGGGATCTTGCACCTGATATTGTGGATTA CTGATCGTCTTTTTTTCAAATGTATTTATCGTCGCTTTAAATACGGTTTGAAAAGAGGGC CTTCTACGGAAGGAGTGCCTGAGTCCATGAGGGAAGAATATCAACAGGAACAGCAGAGTG CTGTGGATGTTGACGATGGTCATTTTGTCAACATAGAGCTGGAGTAAAAAACTACCTTGT TTCTACT |
| | genome segment 8/NS1(1-126) AGCAAAAGCAGGGTGACAAAAACATAATGGACTCCAACACCATGTCAAGCTTTCAGGTAG ACTGTTTCCTTTGGCATATCCGCAAGCGATTTGCAGACAATGGATTGGGTGATGCCCCAT |
| | TCCTTGATCGGCTCCGCCGAGATCAAAAGTCCTTAAAAGGAAGAGGCAACACCCTTGGCC TCGATATCGAAACAGCCACTCTTGTTGGGAAACAAATCGTGGAATGGATCTTGAAAGAGG AATCCAGCGAGACACTTAGAATGACAATTGCATCTGTACCTACTTCGCGCTACCTTTCTG ACATGACCCTCGAGGAAATGTCACGAGACTGGTTCATGCTCATGCCTAGGCAAAAGATAA TAGGCCCTCTTTGCGTGCGATTGGACCAGGCGATCATGGAAAAGTAGATCTTGATTAATT AAGAGGGAGCAATAGTTGGAGAAATTTCACCATTACCTTCTCTTCCAGGACATACTTATG AGGATGTCAAAAATGCAGTTGGGGTCCTCATCGGAGGACTTGAATGGAATGGTAACACGG TTCGAGTCTCTGAAAATATACAGAGATTCGCTTGGAGAAACTGTGATGAGAATGGGAGAC CTTCACTACCTCCAGAGCAGAAATGAAAAGTGGCGAGAGCAATTGGGACAGAAATTTGAG GAAATAAGGTGGTTAATTGAAGAAATGCGGCACAGATTGAAAGCGACAGAGAATAGTTTC GAACAAATAACATTTATGCAAGCCTTACAACTATTGCTTGAAGTAGAACAAGAGATAAGA GCTTTCTCGTTTCAGCTTATTTAATGATAAAAAACACCCTTGTTTCTACT |
| | |

| Seq. ID NO: | Amino Acid |
|---|---|
| 4 (VSV HA) | Nucleoprotein (N): MSVTVKRIIDNTVIVPKLPANEDPVEYPADYFRKSKEIPLYINTTKSLSDLRGYVYQGLKSGNVSIIHVN SYLYGALKDIRGKLDKDWSSFGINIGKAGDTIGIFDLVSLKALDGVLPDGVSDASRTSADDKWLPLYLLG LYRVGRTQMPEYRKKLMDGLTNQCKMINEQFEPLVPEGRDIFDVWGNDSNYTKIVAAVDMFFHMFKKHEC ASFRYGTIVSRFKDCAALATFGHLCKITGMSTEDVTTWILNREVADEMVQMMLPGQEIDKADSYMPYLID FGLSSKSPYSSVKNPAFHFWGQLTALLLRSTRARNARQPDDIEYTSLTTAGLLYAYAVGSSADLAQQFCV GDNKYTPDDSTGGLTTNAPPQGRDVVEWLGWFEDQNRKPTPDMMQYAKRAVMSLQGLREKTIGKYAKSEF DK |
| | Phosphoprotein (P): MDNLTKVREYLKSYSRLDQAVGEIDEIEAQRAEKSNYELFQEDGVEEHTKPSYFQAADDSDTESEPEIED NQGLYAQDPEAEQVEGFIQGPLDDYADEEVDVVFTSDWKPPELESDEHGKTLRLTSPEGLSGEQKSQWLS TIKAVVQSAKYWNLAECTFEASGEGVIMKERQITPDVYKVTPVMNTHPSQSEAVSDVWSLSKTSMTFQPK KASLQPLTISLDELFSSRGEFISVGGDGRMSHKEAILLGLRYKKLYNQARVKYSL |
| | Matrix protein (M): MSSLKKILGLKGKGKKSKKLGIAPPPYEEDTSMEYAPSAPIDKSYFGVDEMDTYDPNQLRYEKFFFTVKM TVRSNRPFRTYSDVAAAVSHWDHMYIGMAGKRPFYKILAFLGSSNLKATPAVLADQGQPEYHTHCEGRAY LPHRMGKTPPMLNVPEHFRRPFNIGLYKGTIELTMTIYDDESLEAAPMIWDHFNSSKFSDFREKALMFGL IVEKKASGAWVLDSISHFK |
| | HA antigen A/Hamburg/4/2009 (H1N1): MKAILVVLLYTFTTANADTLCIGYHANNSTDTVDTVLEKNVTVTHSVNLLEDKHNGKLCK LRGVAPLHLGKCNIAGWILGNPECESLSTASSWSYIVETSSSDNGTCYPGDFIDYEELRE QLSSVSSFERFEIFPKTSSWPNHDSNKGVTAACPHAGAKSFYKNLIWLVKKGNSYPKLSK SYINDKGKEVLVLWGIHHPSTSADQQSLYQNADAYVFVGSSRYSKKFKPEIAIRPKVRDQ EGRMNYYWTLVEPGDKITFEATGNLVVPRYAFAMERNAGSGIIISDTPVHDCNTTCQTPK GAINTSLPFQNIHPITIGKCPKYVKSTKLRLATGLRNVPSIQSRGLFGAIAGFIEGGWTG MVDGWYGYHHQNEQGSGYAADLKSTQNAIDEITNKVNSVIEKMNTQFTAVGKEFNHLEKR IENLNKKVDDGFLDIWTYNAELLVLLENERTLDYHDSNVKNLYEKVRSQLKNNAKEIGNG CFEFYHKCDNTCMESVKNGTYDYPKYSEEAKLNREEIDGVKLESTRIYQILAIYSTVASS LVLVVSLGAISFWMCSNGSLQCRICI |
| | Large protein (L): MEVHDFETDEFNDFNEDDYATREFLNPDERMTYLNHADYNLNSPLISDDIDNLIRKFNSLPIPSMWDSKN WDGVLEMLTSCQANPISTSQMHKWMGSWLMSDNHDASQGYSFLHEVDKEAEITFDVVETFIRGWGNKPIE YIKKERWTDSFKILAYLCQKFLDLHKLTLILNAVSEVELLNLARTFKGKVRRSSHGTNICRIRVPSLGPT FISEGWAYFKKLDILMDRNFLLMVKDVIIGRMQTVLSMVCRIDNLFSEQDIFSLLNIYRIGDKIVERQGN FSYDLIKMVEPICNLKLMKLARESRPLVPQFPHFENHIKTSVDEGAKIDRGIRFLHDQIMSVKTVDLTLV IYGSFRHWGHPFIDYYTGLEKLHSQVTMKKDIDVSYAKALASDLARIVLFQQFNDHKKWFVNGDLLPHDH PFKSHVKENTWPTAAQVQDFGDKWHELPLIKCFEIPDLLDPSIIYSDKSHSMNRSEVLKHVRMNPNTPIP SKKVLQTMLDTKATNWKEFLKEIDEKGLDDDDLIIGLKGKERELKLAGRFFSLMSWKLREYFVITEYLIK |
| | THFVPMFKGLTMADDLTAVIKKMLDSSSGQGLKSYEAICIANHIDYEKWNNHQRKLSNGPVFRVMGQFLG YPSLIERTHEFFEKSLIYYNGRPDLMRVHNNTLINSTSQRVCWQGQEGGLEGLRQKGWTILNLLVIQREA KIRNTAVKVLAQGDNQVICTQYKTKKSRNVVELQGALNQMVSNNEKIMTAIKIGTGKLGLLINDDETMQS ADYLNYGKIPIFRGVIRGLETKRWSRVTCVTNDQIPTCANIMSSVSTNALTVAHFAENPINAMIQYNYFG TFARLLLMMHDPALRQSLYEVQDKIPGLHSSTFKYAMLYLDPSIGGVSGMSLSRFLIRAFPDPVTESLSF WRFIHVHARSEHLKEMSAVFGNPEIAKFRITHIDKLVEDPTSLNIAMGMSPANLLKTEVKKCLIESRQTI RNQVIKDATIYLYHEEDRLRSFLWSINPLFPRFLSEFKSGTFLGVADGLISLFQNSRTIRNSFKKKYHRE LDDLIVRSEVSSLTHLGKLHLRRGSCKMWTCSATHADTLRYKSWGRTVIGTTVPHPLEMLGPQHRKETPC APCNTSGFNYVSVHCPDGIHDVFSSRGPLPAYLGSKTSESTSILQPWERESKVPLIKRATRLRDAISWFV EPDSKLAMTILSNIHSLTGEEWTKRQHGFKRTGSALHRFSTSRMSHGGFASQSTAALTRLMATTDTMRDL GDQNFDFLFQATLLYAQITTTVARDGWITSCTDHYHIACKSCLRPIEEITLDSSMDYTPPDVSHVLKTWR NGEGSWGQEIKQIYPLEGNWKNLAPAEQSYQVGRCIGFLYGDLAYRKSTHAEDSSLFPLSIQGRIRGRGF LKGLLDGLMRASCCQVIHRRSLAHLKRPANAVYGGLIYLIDKLSVSPPFLSLTRSGPIRDELETIPHKIP TSYPTSNRDMGVIVRNYFKYQCRLIEKGKYRSHYSQLWLFSDVLSIDFIGPFSISTTLLQILYKPFLSGK DKNELRELANLSSLLRSGEGWEDIHVKFFTKDILLCPEEIRHACKFGIAKDNNKDMSYPPWGRESRGTIT TIPVYYTTTPYPKMLEMPPRIQNPLLSGIRLGQLPTGAHYKIRSILHGMGIHYRDFLSCGDGSGGMTAAL LRENVHSRGIFNSLLELSGSVMRGASPEPPSALETLGGDKSRCVNGETCWEYPSDLCDPRTWDYFLRLKA GLGLQIDLIVMDMEVRDSSTSLKIETNVRNYVHRILDEQGVLIYKTYGTYICESEKNAVTILGPMFKTVD LVQTEFSSSQTSEVYMVCKGLKKLIDEPNPDWSSINESWKNLYAFQSSEQEFARAKKVSTYFTLTGIPSQ FIPDPFVNIETMLQIFGVPTGVSHAAALKSSDRPADLLTISLFYMAIISYYNINHIRVGPIPPNPPSDGI AQNVGIAITGISFWLSLMEKDIPLYQQCLAVIQQSFPIRWEAVSVKGGYKQKWSTRGDGLPKDTRTSDSL APIGNWIRSLELVRNQVRLNPFNEILFNQLCRTVDNHLKWSNLRRNTGMIEWINRRISKEDRSILMLKSD LHEENSWRD |
| 5 (NS1(1-126) | PB2 protein: MERIKELRDLMSQSRTREILTKTTVDHMAIIKKYTSGRQEKNPALRMKWMMAMRYPITAD KRIMDMIPERNEQGQTLWSKTNDAGSDRVMVSPLAVTWWNRNGPTTSTVHYPKVYKTYFE KVERLKHGTFGPVHFRNQVKIRRRVDTNPGHADLSAKEAQDVIMEVVFPNEVGARILTSE SQLAITKEKKEELQDCKIAPLMVAYMLERELVRKTRFLPVAGGTGSVYIEVLHLTQGTCW EQMYTPGGEVRNDDVDQSLIIAARNIVRRAAVSADPLASLLEMCHSTQIGGVRMVDILRQ NPTEEQAVDICKAAIGLRISSSFSFGGFTFKRTSGSSVKKEEEVLTGNLQTLKIRVHEGY EEFTMVGRRATAILRKATRRLIQLIVSGRDEQSIAEAIIVAMVFSQEDCMIKAVRGDLNF VNRANQRLNPMHQLLRHFQKDAKVLFQNWGIESIDNVMGMIGILPDMTPSTEMSLRGIRV SKMGVDEYSSTERVVVSIDRFLRVRDQRGNVLLSPEEVSETQGTERLTITYSSSMMWEIN GPESVLVNTYQWIIRNWEIVKIQWSQDPTMLYNKMEFEPFQSLVPKATRSRYSGFVRTLF QQMRDVLGTFDTVQIIKLLPFAAAPPEQSRMQFSSLTVNVRGSGLRILVRGNSPVFNYNK ATKRLTVLGKDAGALTEDPDEGTSGVESAVLRGFLILGKEDKRYGPALSINELSNLAKGE KANVLIGQGDVVLVMKRKRDSSILTDSQTATKRIRMAIN |
| | PB1 protein: MDVNPTLLFLKIPAQNAISTTFPYTGDPPYSHGTGTGYTMDTVNRTHQYSEKGKWTTNTE TGAPQLNPIDGPLPEDNEPSGYAQTDCVLEAMAFLEESHPGIFENSCLETMEVVQQTRVD KLTQGRQTYDWTLNRNQPAATALANTIEVFRSNGLTANESGRLIDFLKDVMESMNKEEIE ITTHFQRKRRVRDNMTKKMVTQRTIGKKKQRLNKRGYLIRALTLNTMTKDAERGKLKRRA IATPGMQIRGFVYFVETLARSICEKLEQSGLPVGGNEKKAKLANVVRKMMTNSQDTEISF TITGDNTKWNENQNPRVFLAMITYITRNQPEWFRNILSMAPIMFSNKMARLGKGYMFESK RMKIRTQIPAEMLASIDLKYFNESTKKKIEKIRPLLIDGTASLSPGMMMGMFNMLSTVLG VSILNLGQKKYTKTIYWWDGLQSSDDFALIVNAPNHEGIQAGVDRFYRTCKLVGINMSKK KSYINKTGTFEFTSFFYRYGFVANFSMELPSFGVSGVNESADMSIGVTVIKNNMINNDLG PATAQMALQLFIKDYRYTYRCHRGDTQIQTRRSFELKKLWDQTQSKVGLLVSDGGPNLYN IRNLHIPEVCLKWELMDDDYRGRLCNPLNPFVSHKEIDSVNNAVVMPAHGPAKSMEYDAV ATTHSWIPKRNRSILNTSQRGILEDEQMYQKCCNLFEKFFPSSSYRRPVGISSMVEAMVS RARIDARVDFESGRIKKEEFSEIMKICSTIEELRRQK |
| | PA protein: MEDFVRQCFNPMIVELAEKAMKEYGEDPKIETNKFAAICTHLEVCFMYSDFHFIDERGES IIVESGDPNALLKHRFEIIEGRDRIMAWTVVNSICNTTGVEKPKFLPDLYDYKENRFIEI GVTRREVHIYYLEKANKIKSEKTHIHIFSFTGEEMATKADYTLDEESRARIKTRLFTIRQ EMASRSLWDSFRQSERGEETIEEKFEITGTMRKLADQSLPPNFSSLENFRAYVDGFEPNG CIEGKLSQMSKEVNAKIEPFLRTTPRPLRLPDGPLCHQRSKFLLMDALKLSIEDPSHEGE |
| | GIPLYDAIKCMKTFFGWKEPNIVKPHEKGINPNYLMAWKQVLAELQDIENEEKIPRTKNM KRTSQLKWALGENMAPEKVDFDDCKDVGDLKQYDSDEPEPRSLASWVQNEFNKACELTDS SWIELDEIGEDVAPIEHIASMRRNYFTAEVSHCRATEYIMKGVYINTALLNASCAAMDDF QLIPMISKCRTKEGRRKTNLYGFIIKGRSHLRNDTDVVNFVSMEFSLTDPRLEPHKWEKY CVLEIGDMLLRTAIGQVSRPMFLYVRTNGTSKIKMKWGMELRRCLLQSLQQIESMIEAES SVKEKDMTKEFFENKSETWPIGESPRGVEEGSIGKVCRTLLAKSVFNSLYASPQLEGFSA ESRKLLLIVQALRDNLEPGTFDLGGLYEAIEECLINDPWVLLNASWFNSFLTHALK |
| | PA-X protein: MEDFVRQCFNPMIVELAEKAMKEYGEDPKIETNKFAAICTHLEVCFMYSDFHFIDERGES IIVESGDPNALLKHRFEIIEGRDRIMAWTVVNSICNTTGVEKPKFLPDLYDYKENRFIEI GVTRREVHIYYLEKANKIKSEKTHIHIFSFTGEEMATKADYTLDEESRARIKTRLFTIRQ EMASRSLWDSFVSPKEAKRQLKKNLRLQELCASLPTKVSHRTSPALKTLEPM |
| | HA protein: MKAILVVLLYTFTTANADTLCIGYHANNSTDTVDTVLEKNVTVTHSVNLLEDKHNGKLCK LRGVAPLHLGKCNIAGWILGNPECESLSTASSWSYIVETSSSDNGTCYPGDFIDYEELRE QLSSVSSFERFEIFPKTSSWPNHDSNKGVTAACPHAGAKSFYKNLIWLVKKGNSYPKLSK SYINDKGKEVLVLWGIHHPSTSADQQSLYQNADAYVFVGSSRYSKKFKPEIAIRPKVRDQ EGRMNYYWTLVEPGDKITFEATGNLVVPRYAFAMERNAGSGIIISDTPVHDCNTTCQTPK GAINTSLPFQNIHPITIGKCPKYVKSTKLRLATGLRNVPSIQSRGLFGAIAGFIEGGWTG MVDGWYGYHHQNEQGSGYAADLKSTQNAIDEITNKVNSVIEKMNTQFTAVGKEFNHLEKR IENLNKKVDDGFLDIWTYNAELLVLLENERTLDYHDSNVKNLYEKVRSQLKNNAKEIGNG CFEFYHKCDNTCMESVKNGTYDYPKYSEEAKLNREEIDGVKLESTRIYQILAIYSTVASS LVLVVSLGAISFWMCSNGSLQCRICI |
| | Nucleoprotein (NP): MASQGTKRSYEQMETGGERQDATEIRASVGRMIGGIGRFYIQMCTELKLSDYDGRLIQNS ITIERMVLSAFDERRNKYLEEHPSAGKDPKKTGGPIYRRVDGKWMRELILYDKEEIRRVW RQANNGEDATAGITHIMIWHSNLNDATYQRTRALVRTGMDPRMCSLMQGSTLPRRSGAAG AAVKGVGTIAMELIRMIKRGINDRNFWRGENGRRTRVAYERMCNILKGKFQTAAQRAMMD QVRESRNPGNAEIEDLIFLARSALILRGSVAHKSCLPACVYGLAVASGHDFEREGYSLVG IDPFKLLQNSQVVSLMRPNENPAHKSQLVWMACHSAAFEDLRVSSFIRGKKVIPRGKLST RGVQIASNENVEIMDSNTLELRSRYWAIRTRSGGNTNQQKASAGQISVQPTFSVQRNLPF ERATVMAAFSGNNEGRTSDMRTEVIRMMESAKPEDLSFQGRGVFELSDEKATNPIVPSFD MSNEGSYFFGDNAEEYDS |
| | Neuraminidase (NA): MNPNQKIITIGSVCMTIGMANLILQIGNIISTWISHSIQLGNQNQIETCNQSVITYENNT WVNQTYVNISNTNFAAGQSVVSVKLAGNSSLCPVSGWAIYSKDNSVRIGSKGDVFVIREP FISCSPLECRTFFLTQGALLNDKHSNGTIKDRSPYRTLMSCPIGEVPSPYNSRFESVAWS ASACHDGINWLTIGISGPDNGAVAVLKYNGIITDTIKSWRNNILRTQESECACVNGSCFT VMTDGPSNGQASYKIFRIEKGKIVKSVEMNAPNYHYEECSCYPDSSEITCVCRDNWHGSN RPWVSFNQNLEYQIGYICSGIFGDNPRPNDKTGSCGPVSSNGANGVKGFSFKYGNGVWIG RTKSISSRNGFEMIWDPNGWTGTDNNFSIKQDIVGINEWSGYSGSFVQHPELTGLDCIRP CFWVELIRGRPKENTIWTSGSSISFCGVNSDTVGWSWPDGAELPFTIDK |
| | M1 protein: MSLLTEVETYVLSIIPSGPLKAEIAQRLESVFAGKNTDLEALMEWLKTRPILSPLTKGIL GFVFTLTVPSERGLQRRRFVQNALNGNGDPNNMDRAVKLYKKLKREITFHGAKEVSLSYS TGALASCMGLIYNRMGTVTTEAAFGLVCATCEQIADSQHRSHRQMATTTNPLIRHENRMV LASTTAKAMEQMAGSSEQAAEAMEVANQTRQMVHAMRTIGTHPSSSAGLKDDLLENLQAY QKRMGVQMQRFK |
| | M2 protein: MSLLTEVETPTRSEWECRCSDSSDPLVIAANIIGILHLILWITDRLFFKCIYRRFKYGLK RGPSTEGVPESMREEYQQEQQSAVDVDDGHFVNIELE |
| | NS1(1-126) MDSNTMSSFQVDCFLWHIRKRFADNGLGDAPFLDRLRRDQKSLKGRGNTLGLDIETATLV GKQIVEWILKEESSETLRMTIASVPTSRYLSDMTLEEMSRDWFMLMPRQKIIGPLCVRLD |
| | QAIMEK Nuclear export protein (NEP): MDSNTMSSFQDILMRMSKMQLGSSSEDLNGMVTRFESLKIYRDSLGETVMRMGDLHYLQS RNEKWREQLGQKFEEIRWLIEEMRHRLKATENSFEQITFMQALQLLLEVEQEIRAFSFQL I |
| 6 (NS1(1-126)-ΔPAX | PB2 protein: MERIKELRDLMSQSRTREILTKTTVDHMAIIKKYTSGRQEKNPALRMKWMMAMRYPITAD KRIMDMIPERNEQGQTLWSKTNDAGSDRVMVSPLAVTWWNRNGPTTSTVHYPKVYKTYFE KVERLKHGTFGPVHFRNQVKIRRRVDTNPGHADLSAKEAQDVIMEVVFPNEVGARILTSE SQLAITKEKKEELQDCKIAPLMVAYMLERELVRKTRFLPVAGGTGSVYIEVLHLTQGTCW EQMYTPGGEVRNDDVDQSLIIAARNIVRRAAVSADPLASLLEMCHSTQIGGVRMVDILRQ NPTEEQAVDICKAAIGLRISSSFSFGGFTFKRTSGSSVKKEEEVLTGNLQTLKIRVHEGY EEFTMVGRRATAILRKATRRLIQLIVSGRDEQSIAEAIIVAMVFSQEDCMIKAVRGDLNF VNRANQRLNPMHQLLRHFQKDAKVLFQNWGIESIDNVMGMIGILPDMTPSTEMSLRGIRV SKMGVDEYSSTERVVVSIDRFLRVRDQRGNVLLSPEEVSETQGTERLTITYSSSMMWEIN GPESVLVNTYQWIIRNWEIVKIQWSQDPTMLYNKMEFEPFQSLVPKATRSRYSGFVRTLF QQMRDVLGTFDTVQIIKLLPFAAAPPEQSRMQFSSLTVNVRGSGLRILVRGNSPVFNYNK ATKRLTVLGKDAGALTEDPDEGTSGVESAVLRGFLILGKEDKRYGPALSINELSNLAKGE KANVLIGQGDVVLVMKRKRDSSILTDSQTATKRIRMAIN |
| | PB1 protein: MDVNPTLLFLKIPAQNAISTTFPYTGDPPYSHGTGTGYTMDTVNRTHQYSEKGKWTTNTE TGAPQLNPIDGPLPEDNEPSGYAQTDCVLEAMAFLEESHPGIFENSCLETMEVVQQTRVD KLTQGRQTYDWTLNRNQPAATALANTIEVFRSNGLTANESGRLIDFLKDVMESMNKEEIE ITTHFQRKRRVRDNMTKKMVTQRTIGKKKQRLNKRGYLIRALTLNTMTKDAERGKLKRRA IATPGMQIRGFVYFVETLARSICEKLEQSGLPVGGNEKKAKLANVVRKMMTNSQDTEISF TITGDNTKWNENQNPRVFLAMITYITRNQPEWFRNILSMAPIMFSNKMARLGKGYMFESK RMKIRTQIPAEMLASIDLKYFNESTKKKIEKIRPLLIDGTASLSPGMMMGMFNMLSTVLG VSILNLGQKKYTKTIYWWDGLQSSDDFALIVNAPNHEGIQAGVDRFYRTCKLVGINMSKK KSYINKTGTFEFTSFFYRYGFVANFSMELPSFGVSGVNESADMSIGVTVIKNNMINNDLG PATAQMALQLFIKDYRYTYRCHRGDTQIQTRRSFELKKLWDQTQSKVGLLVSDGGPNLYN IRNLHIPEVCLKWELMDDDYRGRLCNPLNPFVSHKEIDSVNNAVVMPAHGPAKSMEYDAV ATTHSWIPKRNRSILNTSQRGILEDEQMYQKCCNLFEKFFPSSSYRRPVGISSMVEAMVS RARIDARVDFESGRIKKEEFSEIMKICSTIEELRRQK |
| | PA protein: MEDFVRQCFNPMIVELAEKAMKEYGEDPKIETNKFAAICTHLEVCFMYSDFHFIDERGES IIVESGDPNALLKHRFEIIEGRDRIMAWTVVNSICNTTGVEKPKFLPDLYDYKENRFIEI GVTRREVHIYYLEKANKIKSEKTHIHIFSFTGEEMATKADYTLDEESRARIKTRLFTIRQ EMASRSLWDSFRQSERGEETIEEKFEITGTMRKLADQSLPPNFSSLENFRAYVDGFEPNG CIEGKLSQMSKEVNAKIEPFLRTTPRPLRLPDGPLCHQRSKFLLMDALKLSIEDPSHEGE GIPLYDAIKCMKTFFGWKEPNIVKPHEKGINPNYLMAWKQVLAELQDIENEEKIPRTKNM KRTSQLKWALGENMAPEKVDFDDCKDVGDLKQYDSDEPEPRSLASWVQNEFNKACELTDS SWIELDEIGEDVAPIEHIASMRRNYFTAEVSHCRATEYIMKGVYINTALLNASCAAMDDF QLIPMISKCRTKEGRRKTNLYGFIIKGRSHLRNDTDVVNFVSMEFSLTDPRLEPHKWEKY CVLEIGDMLLRTAIGQVSRPMFLYVRTNGTSKIKMKWGMELRRCLLQSLQQIESMIEAES SVKEKDMTKEFFENKSETWPIGESPRGVEEGSIGKVCRTLLAKSVFNSLYASPQLEGFSA ESRKLLLIVQALRDNLEPGTFDLGGLYEAIEECLINDPWVLLNASWFNSFLTHALK |
| | HA protein: MKAILVVLLYTFTTANADTLCIGYHANNSTDTVDTVLEKNVTVTHSVNLLEDKHNGKLCK LRGVAPLHLGKCNIAGWILGNPECESLSTASSWSYIVETSSSDNGTCYPGDFIDYEELRE QLSSVSSFERFEIFPKTSSWPNHDSNKGVTAACPHAGAKSFYKNLIWLVKKGNSYPKLSK SYINDKGKEVLVLWGIHHPSTSADQQSLYQNADAYVFVGSSRYSKKFKPEIAIRPKVRDQ EGRMNYYWTLVEPGDKITFEATGNLVVPRYAFAMERNAGSGIIISDTPVHDCNTTCQTPK GAINTSLPFQNIHPITIGKCPKYVKSTKLRLATGLRNVPSIQSRGLFGAIAGFIEGGWTG MVDGWYGYHHQNEQGSGYAADLKSTQNAIDEITNKVNSVIEKMNTQFTAVGKEFNHLEKR |
| | IENLNKKVDDGFLDIWTYNAELLVLLENERTLDYHDSNVKNLYEKVRSQLKNNAKEIGNG CFEFYHKCDNTCMESVKNGTYDYPKYSEEAKLNREEIDGVKLESTRIYQILAIYSTVASS LVLVVSLGAISFWMCSNGSLQCRICI |
| | Nucleoprotein (NP): MASQGTKRSYEQMETGGERQDATEIRASVGRMIGGIGRFYIQMCTELKLSDYDGRLIQNS ITIERMVLSAFDERRNKYLEEHPSAGKDPKKTGGPIYRRVDGKWMRELILYDKEEIRRVW RQANNGEDATAGITHIMIWHSNLNDATYQRTRALVRTGMDPRMCSLMQGSTLPRRSGAAG AAVKGVGTIAMELIRMIKRGINDRNFWRGENGRRTRVAYERMCNILKGKFQTAAQRAMMD QVRESRNPGNAEIEDLIFLARSALILRGSVAHKSCLPACVYGLAVASGHDFEREGYSLVG IDPFKLLQNSQVVSLMRPNENPAHKSQLVWMACHSAAFEDLRVSSFIRGKKVIPRGKLST RGVQIASNENVEIMDSNTLELRSRYWAIRTRSGGNTNQQKASAGQISVQPTFSVQRNLPF ERATVMAAFSGNNEGRTSDMRTEVIRMMESAKPEDLSFQGRGVFELSDEKATNPIVPSFD MSNEGSYFFGDNAEEYDS |
| | Neuraminidase (NA): MNPNQKIITIGSVCMTIGMANLILQIGNIISTWISHSIQLGNQNQIETCNQSVITYENNT WVNQTYVNISNTNFAAGQSVVSVKLAGNSSLCPVSGWAIYSKDNSVRIGSKGDVFVIREP FISCSPLECRTFFLTQGALLNDKHSNGTIKDRSPYRTLMSCPIGEVPSPYNSRFESVAWS ASACHDGINWLTIGISGPDNGAVAVLKYNGIITDTIKSWRNNILRTQESECACVNGSCFT VMTDGPSNGQASYKIFRIEKGKIVKSVEMNAPNYHYEECSCYPDSSEITCVCRDNWHGSN RPWVSFNQNLEYQIGYICSGIFGDNPRPNDKTGSCGPVSSNGANGVKGFSFKYGNGVWIG RTKSISSRNGFEMIWDPNGWTGTDNNFSIKQDIVGINEWSGYSGSFVQHPELTGLDCIRP CFWVELIRGRPKENTIWTSGSSISFCGVNSDTVGWSWPDGAELPFTIDK |
| | M1 protein: MSLLTEVETYVLSIIPSGPLKAEIAQRLESVFAGKNTDLEALMEWLKTRPILSPLTKGIL GFVFTLTVPSERGLQRRRFVQNALNGNGDPNNMDRAVKLYKKLKREITFHGAKEVSLSYS TGALASCMGLIYNRMGTVTTEAAFGLVCATCEQIADSQHRSHRQMATTTNPLIRHENRMV LASTTAKAMEQMAGSSEQAAEAMEVANQTRQMVHAMRTIGTHPSSSAGLKDDLLENLQAY QKRMGVQMQRFK |
| | M2 protein: MSLLTEVETPTRSEWECRCSDSSDPLVIAANIIGILHLILWITDRLFFKCIYRRFKYGLK RGPSTEGVPESMREEYQQEQQSAVDVDDGHFVNIELE |
| | NS1(1-126)protein: MDSNTMSSFQVDCFLWHIRKRFADNGLGDAPFLDRLRRDQKSLKGRGNTLGLDIETATLV GKQIVEWILKEESSETLRMTIASVPTSRYLSDMTLEEMSRDWFMLMPRQKIIGPLCVRLD QAIMEK |
| | Nuclear export protein (NEP): MDSNTMSSFQDILMRMSKMQLGSSSEDLNGMVTRFESLKIYRDSLGETVMRMGDLHYLQS RNEKWREQLGQKFEEIRWLIEEMRHRLKATENSFEQITFMQALQLLLEVEQEIRAFSFQL I |

### Examples

Solvents, reagents and starting materials were purchased from commercial vendors and used as received unless otherwise described. All reactions were performed at room temperature unless otherwise stated. Starting materials were purchased from commercial sources or synthesised according to the methods described herein or using literature procedures.

### Material and methods

### Cells

Madin-Darby canine kidney type II cells (MDCK-II) were kindly provided by Georg Herrler (University of Veterinary Medicine, Hannover, Germany) and maintained with minimum essential medium (MEM, Thermo Fisher Scientific, Basel, Switzerland; cat. no. 31095-029) supplemented with 5% of fetal bovine serum (FBS; Pan Biotech, Aidenbach, Germany; cat. no. P30-3033). Human embryonic kidney (HEK) 293T cells (American Type Culture Collection (ATCC), Manassas, USA; cat. no. CRL-3216) were maintained with Dulbecco's Modified Eagle Medium (DMEM, Thermo Fisher Scientific; cat. no. 32430-027) supplemented with 10% FBS. Baby hamster kidney 21 (BHK-21) fibroblasts were obtained from ATCC (cat. no. CCL-10) and maintained in Glasgow's minimal essential medium (GMEM, Thermo Fisher Scientific, cat. no. 21710-025) supplemented with 5% FBS. BHK-G43 cells, a transgenic BHK-21 cell clone expressing VSV glycoprotein G in a regulated manner, was maintained in GMEM supplemented with 5% FBS (Hanika et al., 2005). All cells were grown at 37°C in a 5% CO2 atmosphere.

### Generation of recombinant influenza virus

The pHW2000 plasmids encoding the 8 segments of A/Hamburg/4/2009 (H1N1) (GenBank accession nos.: GQ166207, GQ166209, GQ166211, GQ166213, GQ166215, GQ166217, GQ166219, GQ166221) were originally provided by Hans-Dieter Klenk (University of Marburg, Marburg, Germany). The pHW2000 plasmids encoding RNA segments 3 (PA) and 8 (NS1, NEP) were modified as previously described (Avanthay et al., 2023). Briefly, four stop codon were inserted at position 126 of the NS1 open reading frame leading to a truncated protein lacking the last 93 amino acids. This virus is termed "NS1(1-126)". To eliminate PA-X expression, four nucleotides were changed at position 191 without modifying the amino acid sequence of the PA protein. This virus was termed "NS1(1-126)-ΔPAX". Recombinant viruses were generated using the eight-plasmid system as previously described (Avanthay et al., 2023; Hoffmann et al., 2000) and passaged two times on MDCK-II cells with FBS-deficient medium containing 1% penicillin/streptomycin and 1 µg/ml of N-tosyl-L-phenylalanine chloromethyl ketone-treated trypsin (TPCK-trypsin, Merck KGgA, Darmstadt, Germany, cat. no. 4370285). Infectious virus titers were determined on MDCK-II cells as previously described (Ocaña-Macchi et al., 2009), using a monoclonal antibody directed to the influenza virus nucleoprotein clone (H16-L10-4R5; ATCC, HB-65) for detection of infected cells by indirect immunofluorescence.

### Generation of recombinant VSV vector vaccine

The HA gene of A/Hamburg/4/2009 (H1N1) (GenBank acc. no. GQ166213) was amplified by PCR using the Phusion DNA polymerase and inserted into the *Mlul* and BstEll sites of the pVSV*ΔG(HA_{H5-HP}) plasmid (Halbherr et al., 2013), resulting in the pVSV*ΔG(H1) plasmid. Recombinant vesicular stomatitis virus (VSV) replicon particles were generated as previously described (Berger Rentsch and Zimmer, 2011; Kalhoro et al., 2009; Ricklin et al., 2016). Briefly, BHK-G43 cells were first infected with modified vaccinia virus Ankara encoding the T7 phage RNA polymerase (MVA-T7, kindly provided by Gerd Sutter from Ludwig-Maximilians-Universität, München, Germany) using a multiplicity of infection (moi) of 3 focus-forming units (ffu) per cell. Subsequently, the cells were transfected with pVSV*ΔG(H1) along with three plasmids encoding the VSV N, P, and L proteins; all under the control of the T7 promoter. The cells were incubated for 24h at 37°C and 5% CO₂ in the presence of 10⁻⁹ M of mifepristone (Merck KGaA, Darmstadt, Germany) to allow expression of the G protein. Then, the cells were trypsinized and seeded along with fresh BHK-G43 cells into a T75 flask and incubated with GMEM containing 5% FBS and 10⁻⁹ M mifepristone for 24h at 37°C and 5% CO₂. The supernatant was harvested, and cells debris removed by low-speed centrifugation, and the supernatant passed through a 0.2 µm pore-size filter. The recombinant VSV*ΔG(H1) (VSV_{*ΔG(H1)}) vector was propagated on mifepristone-treated BHK-G43 cells and stored in aliquots at -70°C. Infectious virus titers were determined on BHK-21 cells. The recombinant VSV*ΔG(Luc) (VSV_{*ΔG(LUC)}) vector has been generated previously and was propagated accordingly (Berger Rentsch and Zimmer, 2011).

### Animal experiments

For the first animal experiment, 15 healthy 10-weeks old large white conventional pigs were purchased from Agroscope (competence center of the Swiss confederation in the field of agricultural and agri-food research). The animals were randomly allocated into three groups each containing five pigs of mixed sex. The animals were intranasally immunized with 106 ffu/animal of either pH1N1/09, pH1N1/09-NS1(1-126), or VSV*ΔG(Luc) as control. Body temperature and clinical symptoms of disease were monitored for the following 12 days. Oro-nasal swab samples were taken daily for the first 12 days post immunization to monitor virus shedding. Serum and saliva samples were taken once a week for a total period of six weeks after immunization to monitor for systemic and local antibody responses. Six weeks after immunization, the animals were euthanized by electroshock and subsequent exsanguination. Bronchoalveolar lavage (BAL) was prepared immediately after exsanguination.

For the second animal experiment, 25 healthy 10-weeks old specific pathogen-free (SPF) Swiss large white pigs from the Institute of Virology and Immunology (IVI, Mittelhäusern) breeding facility were used. The animals were tested seronegative for the influenza A virus nucleoprotein by using a commercial ELISA (ID Screen^{®} Influenza A Antibody Competition Multi-species ELISA, ID-Vet, Montpellier, France, cat. no, FLUACA). The animals were divided randomly into five groups each containing five pigs of mixed sex and immunized via the intramuscular route using a dose of 108 ffu/pig of either VSV*ΔG(H1) or the control vector VSV*ΔG(Luc) (Table 1). Temperature and clinical symptoms were monitored daily for seven days, and serum samples were prepared at days 13 and 27 after primary immunization (Fig. 2A). At day 28 after primary immunization, the animals were immunized via the nasal route with LAIV (see Table 1) using a dose of 105 ffu/pig. To this end, an MAD NasalTM intranasal mucosal atomization device (Teleflex Medical Europe Ltd., Ireland, cat. no, MAD300) plugged to a 10-ml syringe was employed. Body temperature and clinical symptoms were monitored for one week following the second (boost) immunization. Swabs samples were taken daily, suspended in 2 ml of MEM, and stored at -70°C prior to use. Serum and saliva samples were taken every week for four weeks and stored at -20°C prior to serological testing. Blood samples were collected at day 49 (three weeks post boost) for isolation of PBMCs and T cell reactivation experiments. At day 56, the animals were infected with wild-type pH1N1/09 via the nasal route using 106 ffu/pig. Body temperature and clinical symptoms were monitored, and serum and saliva samples were collected daily. Five days after challenge infection, the animals were euthanized by electroshock and subsequent exsanguination. Immediately after exsanguination, the lung's right cranial lobe was prepared for viral RNA detection and lung samples were taken in formalin as indicated below for histological analysis. Bronchoalveolar lavage was prepared for detection of virus-specific antibodies and viral RNA.

### Ethics statement

The study was performed according to Swiss laws (the Animal Welfare Act TSchG SR 455, the Animal Welfare Ordinance TSchV SR 455.1, and the Animal Experimentation Ordinance TVV SR 455.163). All experiments were reviewed by the committee on animal experiments of the canton of Bern and approved by the cantonal veterinary authority under the license number BE128/19.

### ELISA

For detection of antibodies directed against pH1 N1/09, 96-well plates (NuncTM MaxiSorpTM) were coated overnight at room temperature with 1 µg/well of heat-inactivated A/H1 N1/09 ( 56°C, 30 min). Plates were blocked for 1h at 37°C with 200 µl/well of 1% bovine serum albumin (BSA) in PBS containing 0.05% (v/v) Tween 20. Serum, saliva, and BAL samples were added in duplicates at the right dilution and incubated for 1h at 37°C. Ten-fold serial dilutions of pH1 N1/09 HA-specific antibody (Thermo Fisher, cat. no. PA5-81645) were used as standard. Plates were washed with PBS (without Ca2+ and Mg2+) containing 0.05% (v/v) of Tween 20 and incubated with horse radish peroxidase (HRP)-conjugated anti-swine IgG (Abcam, cat. no. ab6915) or anti-swine IgA (Bethyl, cat. no. A100-102P) diluted 1/2000 and 1/50'000, respectively. The cells were washed three times with 200 µl/well of PBS/Tween and incubated for 10 min at room temperature with 50 µl/well of 3,3',5',5'-tetramethylbenzidine (TMB)/H2O2 peroxidase substrate (Merck KGaA, cat. no. T4444). The reaction was stopped by addition of 50 µl/well of 1 M HCl and absorbance read at 450 nm using a GloMax microplate reader (Promega, Dübendorf, Switzerland).

### Virus neutralization test

Porcine sera were serially diluted in MEM medium (two-fold or five-fold dilution steps) and added in quadruplicates to 96-well microtiter plates (50 µl/well). To each well 50 µl of pH1N1/09 (2000 ffu/ml) were added and incubated for 1h at 37°C. The antibody/virus mix was then added to MDCK-II cells that were grown to confluence in 96-well cell culture plates and incubated for 1h at 37°C and 5% CO2. The cells were washed once and incubated with fresh medium (100 µl/well) for 24h at 37°C and 5% CO2. The cells were fixed with 4% formaldehyde solution and immuno-stained as previously described (Ocaña-Macchi et al., 2009). The 50% neutralizing dose (ND50) was calculated using the Spearman-Kärber method (Ramakrishnan, 2016).

### RNA extraction and RT-qPCR

All samples were directly transferred to 700 µl of RA1 lysis buffer (Macherey-Nagel, Düren, Germany, cat. no. 740961) containing 1% β-mercaptoethanol. Organs were homogenized in RA1 lysis buffer using a tissue bullet blender (Next Advanced Inc., Troy, NY, USA). Total RNA was extracted from the lysates using the NucleoMag Vet kit (Macherey-Nagel, cat.no. 744200) according to the manufacturer's protocol. Reverse transcription from RNA to cDNA and real-time quantitative PCR (qPCR) were performed on a QuantStudio 5 real-time PCR system (Thermo Fisher Scientific) using the AgPath-ID^{™} One-Step RT-PCR kit (Life Technologies, Zug, Switzerland, cat. no. AM1005) with vRNA segment 7-specific oligonucleotide primers and probe (Hofmann et al., 2008; Spackman et al., 2002). Data were acquired and analyzed using the Design and Analysis Software v1.5.2 (Thermo Fisher Scientific). Quantification was performed using an internal standard based on the IAV RNA segment 7.

### T cell assay

Peripheral blood mononuclear cells (PBMC) were isolated from 50 ml of blood in EDTA per pig. For each animal, five million PBMC were seeded per well (12-well plate format) in triplicates and stimulated for 14h at 39°C and 5% CO2 with pH1N1/09 (moi of 0.1 ffu/cell). Thereafter, brefeldin A at 3µg/ml (Invitrogen, cat. no. 00-4506-51) was added to the cells and incubated for another 4 h. The cells were washed twice and stained with the fixable Aqua Dead Cell Stain kit (ThermoFisher, cat. no. L34957). The CD4+ and CD8+ T lymphocyte cells were labelled by incubation with anti-CD4 IgG2b (clone 74-12-4, hybridoma kindly obtained from Dr. Joan Lunney, USDA Beltsville, MD, USA; Pescovitz et al., 1984) and anti-CD8β IgG2a (PG164A, BioTechne, Basel, cat. no. NBP2-60955), followed by isotype-specific AlexaFluor-488 (Thermo Fisher, cat. no. A21141) and PE-Cy7 conjugates (Abcam, cat.no. ab130787). After fixation and permeabilization, anti-IFNγ-PE diluted 1/200 (P2G10, BD Biosciences, cat. no. 561481), anti-TNF-AF647 diluted 1/20 (Mab11, Biolegend, cat. no. 502916) and anti-IL17 diluted 1/5 (SCPL1362, BD Bioscience, cat. no. 560436) were added. Data were acquired on FACS Canto-II (BD Bioscience) and analysed using FlowJo software, version 10 (BD Bioscience).

### Histopathology

Three lung samples of approximately 1 cm in diameter from the same location of the right lung: apical lobe, medial lobe, and caudo-dorsal area of the diaphragmatic lobe, were systematically taken and placed in 4 % formalin. In addition, when macroscopic lesions compatible with broncho interstitial pneumonia were observed, up to three additional samples from the affected lobes were taken for histological examination. The samples were trimmed and placed in two different cassettes per pig separately, with one cassette containing the three systematically collected lung sections and the other containing up to three lung sections with macroscopic lesions.

The microscopic evaluation was made based on the previously reported Morgan score (McNee et al., 2020; Morgan et al., 2016). Necrosis of the bronchiolar epithelium, airway inflammation, perivascular/bronchiolar cuffing, alveolar exudates, and septal inflammation, were scored from 0-4 points each (0: none, 1: minimal, 2: mild, 3: moderate, and 4: severe). All the parameters were added to obtain the scoring per slide (0-20 points), per animal (sum of slide 1 and 2, 0-40 points) and per group (mean score per animal for each group, 0-40 points).

### Statistical analysis

Data analysis and figures were done using GraphPad Prism 8 Software (GraphPad Software). One-way and two-way ANOVA tests with multiple comparisons, Kruskal-Wallis test, and Mann-Whitney tests were used to determine statistical significance in experimental data (detection of viral RNA by RT-qPCR, antibody titers, and T cell recall responses). P values lower than 0.05 was considered as statistically significant.

### Results

### LAIV NS1(1-126) is still excreted from the respiratory tract

In a recent work, we demonstrated that a recombinant pH1N1/09 encoding a modified NS1 protein that was truncated by 93 amino acids at the C-terminus replicated in a porcine bronchiolar cell line as efficiently as wild-type virus despite the induction of type I and III interferon (IFN-I and III) (Avanthay et al., 2023). To determine whether this LAIV candidate would be more attenuated in vivo, we infected pigs via the nasal route with either wild-type pH1N1/09 or the NS1(1-126) mutant. Monitoring of rectal temperature for 12 days revealed no fever in any of the animal groups (Fig. 1A). Analysis of oropharyngeal swab samples by RT-qPCR showed that pH1N1/09 RNA was shed for up to 10 days post infection (Fig. 1B). Compared to wild-type virus, the NS1(1-126) mutant was excreted at significantly lower levels with a reduction of viral RNA by almost two log10 between days 2 and 5, and a shortening of the overall excretion time by two days. Analysis of serum IgG by ELISA revealed that the NS1(1-126) mutant induced significantly lower levels of pH1N1/09-specific antibodies than wild-type virus (Fig. 1C). Over time the serum IgG levels induced by the LAIV candidate dropped faster than those induced by wild-type virus. Nevertheless, in the bronchoalveolar lavage (BAL), the levels of virus-specific IgG were similar in both groups (Fig. 1D). Likewise, virus-specific serum and BAL IgA levels did not differ significantly between the pH1N1/09- and NS1(1-126) mutant-infected animals (Fig. 1E, F). We also measured the levels of neutralizing antibodies in serum and BAL fluids and found lower levels with the NS1 (1-126) mutant when compared to pH1 N1/09 group (Fig. 1G, H). With respect to serum samples, the differences between the animal groups were only significant when the area under the curve (AUC) was analyzed (Fig. 1G). Altogether, these data indicate that the LAIV candidate NS1 (1-126) is still shed from the respiratory tract for a considerable time, albeit at reduced levels when compared to wild-type virus. Additionally, the systemic antibody response to the LAIV candidate was reduced when compared to that induced by wild-type virus infection.

### A heterologous prime/boost vaccination protocol reduces LAIV shedding

To improve the performance of LAIV in terms of immunogenicity and reduced virus shedding, we developed a heterologous prime/boost immunization protocol (Fig. 2A). The animals were first immunized via the intramuscular route with VSV*ΔG(H1), a propagation-incompetent VSV vector encoding the pH1 N1/09 HA in place of the VSV glycoprotein (G). Control animals were immunized with VSV*ΔG(Luc) encoding firefly luciferase. Immunization of pigs with either VSV*ΔG(H1) or VSV*ΔG(Luc) did not cause an increase of body temperature (Fig. 2B) or any other signs of disease. Four weeks after the primary vaccination, the pigs were immunized via the nasal route with the LAIV vaccine candidates (Table 1).

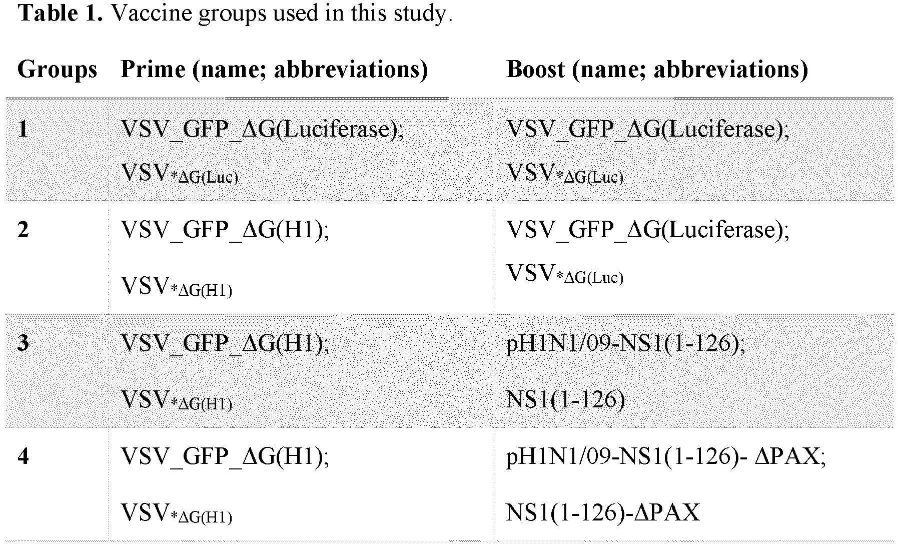

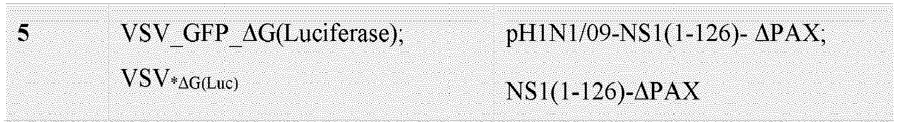

The LAIV NS1(1-126) encoded a truncated NS1 protein lacking 93 amino acids at the C-terminus, and NS1(1-126)- ΔPAX combined both NS1 truncation and PA-X deletion (Avanthay et al., 2023). No fever was recorded for the first 10 days following infection with any of the LAIV (Fig. 2C). Detection of viral RNA in oropharyngeal swab samples by RT-qPCR showed that animals which were first primed with the control vector VSV*ΔG(Luc) and subsequently immunized via the nasal route with NS1(1-126)-ΔPAX shed virus at relatively high levels and for prolonged time (Fig. 2D). In contrast, if the pigs were primed with VSV*ΔG(H1) and then boosted with NS1(1-126)-ΔPAX, virus shedding was significantly reduced with no viral RNA detectable after day 6 (Fig. 2E). Two out of five pigs showed virus shedding for only 1 day and one pig did not shed any virus (Fig. 2E). Interestingly, if VSV*ΔG(H1)-primed animals were boosted with NS1(1-126), shedding was increased when compared to the VSV*ΔG(H1)/NS1(1-126)-ΔPAX booster vaccine (Fig. 2F). Together, these data demonstrate that shedding of LAIV can be efficiently reduced by a heterologous prime/boost vaccination regimen as supported by analysis of their respective shedding AUC (Fig. 2G).

### VSV*ΔG(H1)/NS1(1-126)-ΔPAX prime/boost vaccination promotes systemic antibody responses

To assess the immune response following vaccination, serum and saliva were collected at different time points after primary and secondary immunization and analyzed by indirect ELISA. After the primary immunization with VSV*ΔG(H1), virus-specific IgG were not detected (Fig. 3A, B), but were strongly induced following the intranasal boost with NS1(1-126)-ΔPAX or NS1(1-126). In contrast, significantly lower levels of specific serum IgG were detected if the animals were first immunized with the control vector VSV*ΔG(Luc) and subsequently intranasally boosted with NS1(1-126)-ΔPAX (Fig. 3A, right panel). With respect to serum IgA, the VSV*ΔG(Luc)/NS1(1-126)-ΔPAX vaccination protocol resulted in higher virus-specific IgA levels compared to the VSV*ΔG(H1)/NS1(1-126) and VSV*ΔG(H1)/NS1(1-126)-ΔPAX vaccination protocols (Fig. 3B). No significant differences in the immune response following the boost immunization with NS1(1-126) or NS1 (1-126)-ΔPAX LAIV were observed (Fig. 3B). Interestingly, the serum IgA response was relatively short lasting with a peak at two weeks followed by a sharp decline. In contrast, serum IgG levels reached a plateau two weeks after the boost and thereafter decreased only slowly (Fig. 3A).

In addition to serum antibodies, we also analyzed the mucosal immune response by measuring the pH1N1/09-specific IgG and IgA antibody levels in saliva by indirect ELISA. Intramuscular immunization with VSV*ΔG(H1) followed by mock-vaccination with PBS via the nasal route did not result in the induction of detectable levels of IgG in saliva (Fig. 3C). In contrast, when the VSV*ΔG(H1)- or VSV*ΔG(Luc)-primed animals were boosted with either NS1(1-126) or NS1(1-126)-ΔPAX mutants, a strong increase in virus-specific secreted IgG was observed between days 34 and 41 post primary vaccination. These antibody levels stayed high until day 48 and then dropped (Fig. 3C). No significant differences between the LAIVs were observed. With respect to virus-specific IgA in saliva, an increase in ELISA reactivity was observed for the animals which were primed with either VSV*ΔG(Luc) or VSV*ΔG(H1) and boosted with PBS (Fig. 3D). Considering that this reaction was also found in absence of H1 expression, it can be considered as a non-specific reaction. In contrast, the animals primed with VSV*ΔG(H1) and subsequently boosted with either NS1(1-126) or NS1(1-126)-ΔPAX showed increased IgA responses. The highest IgA levels were induced by the VSV*ΔG(Luc)/NS1(1-126)-ΔPAX vaccination regimen (Fig. 3D). Next, we analysed the induction of virus-neutralizing antibodies. Priming with VSV*ΔG(H1) resulted in only very low levels of neutralizing antibodies (Fig. 3E, right panel). However, if the H1-primed animals were boosted via the nasal route with either the NS1(1-126)-ΔPAX or the NS1(1-126) mutants, a significant increase in virus-neutralizing serum antibodies was detected. The ND50 values reached a peak of approximately 3000 at day 48 and dropped to an ND50 value of approximately 1000 at day 55 post primary immunization. In contrast, immunization with NS1(1-126)-ΔPAX of pigs that were injected with the control vector VSV*ΔG(Luc) resulted in significantly lower neutralizing antibody levels compared to VSV*ΔG(H1)- primed and NS1(1-126)-ΔPAX-boosted animals.

*The prime*/*boost vaccination protocol enhances CD4+ T-cell memory responses* The cellular arm of the immune system is essential to help in the activation of the B cell through the production of cytokines and effector molecules (CD4+ T cell), and to clear virus-infected cells (CD8+ T cell) (L'Huillier et al., 2020). To assess the level of CD4+ and CD8+ T-cell memory induction by the tested vaccinations, PBMCs were collected at day 48, restimulated with viral antigen and analyzed by flow cytometry (FCM) for intracellular expression of IFNγ, TNF and IL-17 in CD4+ or CD8+ T cells (Fig. 4). Pigs vaccinated only once with VSV*ΔG(H1) showed no significant activation of CD4+ and CD8+ T cells when compared to the control vector group (VSV*ΔG(Luc)). Animals that were primed with VSV*ΔG(H1) and boosted with either NS1 (1-126) or NS1 (1-126)-ΔPAX LAIV showed significantly increased numbers of CD4+ T cells expressing TNF, IFNγ and both TNF and IFNγ but not IL-17s (Fig. 4A-D). Importantly, this was not observed in the group that was primed with the control vector VSV*ΔG(Luc) followed by immunization with NS1(1-126)-ΔPAX (Fig. 4, A, B, D), demonstrating improved priming of CD4 Th1 cells by the heterologous prime/boost vaccination protocol. No recall responses were observed for any of the vaccine groups in the CD8+ T cell subset (Fig. 4E).

### LAIV vaccine candidates induce sterilizing immunity

To assess whether the heterologous prime/boost vaccination regimen would provide protection against infection with the homologous IAV strain, pigs were intranasally inoculated with pH1N1/09 using a dose of 106 TCID50 per animal. Independent of the treatments, none of the infected animals developed clinical signs of disease or fever (Fig. 5A). For the vaccine groups VSV*ΔG(H1)/NS1(1-126)-ΔPAX, VSV*ΔG(H1)/NS1(1-126), and VSV*ΔG(Luc)/NS1(1-126)-ΔPAX, no viral RNA was detected in the swab samples (Fig. 5B), indicating the absence of any virus replication in the respiratory tract of the immunized animals. In contrast, in the control group and the VSV*ΔG(H1)-primed only group, high level of virus shedding was observed which reached 106 genome equivalents/µl at day two post infection and stayed at this level until euthanasia at day five (Fig. 5B). When the VSV*ΔG(H1)-primed only group was compared to the control group a minor but significant reduction of viral RNA was found at days two and five post infection, and in the AUC analyses (Fig. 5B).

Following euthanasia of the pigs at day 5 post infection, viral RNA was also measured in lung homogenates. The highest levels were detected in animals of the control vaccine group, while the VSV*ΔG(H1)-primed only group displayed lower levels of viral RNA (Fig. 5C). However, this difference was not significant due to high sample variability. Importantly, as observed in the swab samples, no viral RNA was detected in the lungs of animals immunized with all protocols utilizing LAIVs (Fig. 5C). When viral RNA was measured in BAL fluids, the animals of the VSV*ΔG(H1) - primed only group showed a significantly reduced level of viral RNA compared to the control group.

In contrast, as with the lung tissue, viral RNA was completely absent from the BAL fluids of all three vaccine groups that received the LAIV candidates (Fig. 5D). In agreement with the lack of clinical signs, the histopathological examination of the lungs identified only mild lesions typical of interstitial pneumonia. The histological lesions scoring was significantly higher in the VSV*ΔG(H1)-primed only than in LAIV groups (Fig. 5E). However, the macroscopic lesions, when present, were only mild and focal or multifocal. This may be a reason why there were no more differences between groups.

Postmortem analysis of mucosal antibody titers in BAL fluid revealed that pigs, which were first primed with VSV*ΔG(H1) and then boosted with NS1(1-126)-ΔPAX LAIV, had significantly higher IgA levels compared to the VSV*ΔG(H1) -prime only group (Fig. 6A). In lung homogenates, only the VSV*ΔG(Luc)/NS1(1-126)-ΔPAX induced significantly higher IgA levels when compared to the negative control and the VSV*ΔG(H1)/PBS groups (Fig. 6B). With respect to lung IgG levels, no significant enhancement was found even though animals that have been primed with VSV*ΔG(H1) and boosted with either NS1(1-126) or NS1(1-126)-ΔPAX present an increased tendency (Fig. 6C).

Taken together, these results demonstrate that our novel heterologous prime/boost vaccine induces enhanced systemic IAV-specific immunity in terms of higher IgG, higher neutralizing antibodies titers and enhanced memory Th1 cells. These systemic responses combined with the induction of local mucosal immunity in the respiratory tract provided sterilizing immunity against challenge infection. Importantly, both the priming with the VSV*ΔG(H1) and the use of the NS1(1-126)-ΔPAX for the intranasal LAIV vaccine component enhanced the safety by strongly reducing LAIV shedding following vaccination and completely preventing shedding of challenge virus.

### Discussion

LAIV vaccines offer several advantages compared to inactivated IAV vaccines (Ullah and Ross, 2022). First, LAIV infection results in the synthesis of all influenza virus antigens in their native conformation and thereby stimulate a broader antibody and T-cell response, that will improve protection against heterologous field viruses. Second, such vaccines also induce mucosal immunity when applied via the intranasal route and are expected to more efficiently stop transmission (Lavelle and Ward, 2022). Third, LAIV vaccines do not require adjuvants that can cause local and systemic side effects (Carter and Curran, 2011). Fourth, LAIV may have an improved duration of immunity due to prolonged antigenic stimulation and improved T-cell help which is required to generate long-lived plasma cells (Mohn et al., 2018, 2015). On the negative side, LAIV vaccines can be problematic in terms of safety. If administered to patients with a weakened immune system, they might cause disease symptoms and be released from the upper respiratory tract and transmitted to other individuals (Rubin et al., 2014). In addition, enhanced replication of LAIV may also favor the emergence of revertant virus that have regained virulence, or may enhance the risk of formation of reassortant viruses in the case of co-infection with field virus. On the other hand, if replication of LAIV is too restricted due to over-attenuation, the antigen levels will be too low to trigger a strong immune response. Therefore, finding the balance between safety and efficacy remains challenging.

An example for a LAIV failure is the NS1-truncated LAIV pig vaccine that had to be withdrawn from the market after emergence a reassortant virus with gene segments from field IAV and the vaccine strains (Sharma et al., 2020). In support of this, the present study shows that the shedding of this virus (NS1(1-126)) was lower than pH1N1/09 but still detectable for at least 8 days, and similar observations have been made by others using H3N2 swine IAV (Vandoorn et al., 2022). All this demonstrates the importance of novel or additional attenuation strategies.

Our invention combines the high capacity of VSV-based vectors to induce systemic antibody and T cell responses after i.m. injection (Ricklin et al., 2016) with the LAIV approach. To this end, we employed the highly safe propagation-defective VSV*ΔG(H1) replicon particles expressing HA for intramuscular priming and NS1- and/or PA-X-modified LAIV for nasal boosting to induce local protection. This approach strongly reduced LAIV shedding in particular when the double mutant NS1(1-126)- ΔPAX was employed. In addition to the reduced shedding, the heterologous VSV/LAIV vaccination protocol was more immunogenic in terms of the induction of systemic IgG and neutralizing antibodies as well as memory Th1 cells. While mucosal antibody levels were below those induced by LAIV alone, the immunity induced resulted in complete protection from challenge infection as indicated by the absence of lung lesions and the lack of viral RNA the respiratory tract. The achievement of such a sterilizing immunity would stop transmission chains and therefore have a major impact on slowing down epidemics. This novel prime/boost vaccination protocol will help to control seasonal influenza epidemics or pandemics more efficiently both in terms of improved effectiveness at preventing disease and better efficacy at the population level.

### Numbered Embodiments of the Inventions

1. A combination of a priming composition and a boosting composition for priming and boosting an immune response in a subject comprising (1) a priming composition comprised of a propagation-defective RNA virus vector or self-replicating RNA or non-self-replicating RNA, and (2) a boosting composition comprising a Live Attenuated Influenza Vaccine and, whereby the immune response resulting from administration of the priming composition to the subject is capable of being boosted.
2. A method for inhibiting a viral infection in a subject, comprising administering a prime-boost vaccination to the subject, wherein the prime-boost vaccination comprises administering a primer vaccine and a booster vaccine to the subject, wherein:
   the primer vaccine comprises a propagation-defective RNA virus vector or
   self-replicating RNA or non-self-replicating RNA and
   the booster vaccine comprises a Live Attenuated Influenza Vaccine;
   thereby inhibiting the viral infection in the subject.
3. A method of generating an immune response in a subject, comprising (a) parenterally administering a first immunogenic composition comprising a propagation-defective RNA virus vector or self-replicating RNA or non-self-replicating RNA and; (b) administering a second immunogenic composition by the respiratory route comprising a Live Attenuated Influenza Vaccine, thereby inducing an immune response in the subject.
4. A method for vaccinating a subject comprising a first step of administering an effective amount of a propagation-defective RNA virus vector or self-replicating RNA or non-self-replicating RNA and a second step of administering an effective amount of a Live Attenuated Influenza Vaccine to the subject.
5. Use of a prime-boost vaccination to inhibit influenza Virus infection in a subject, wherein the prime-boost vaccination comprises administering a primer vaccine and a booster vaccine to the subject, wherein:
   the primer vaccine comprises propagation-defective RNA virus vector or self-replicating RNA or non-self-replicating RNA; and the booster vaccine comprises a Live Attenuated Influenza Vaccine; thereby inhibiting the viral infection in the subject.
6. A heterologous prime-boost vaccine for use in inducing an immune response in a mammal comprising: administering a primer vaccine and a booster vaccine to the subject, wherein:
   the primer vaccine comprises a propagation-defective RNA virus vector or self-replicating RNA or non-self-replicating RNA; and the booster vaccine comprises a Live Attenuated Influenza Vaccine;
   thereby inhibiting the viral infection in the subject.
7. A heterologous prime-boost vaccine for use in immunization against influenza disease in a mammal comprising:
   administering a primer vaccine and a booster vaccine to the subject, wherein:
   the primer vaccine comprises a propagation-defective RNA virus vector or
   self-replicating RNA or non-self-replicating RNA; and
   the booster vaccine comprises a Live Attenuated Influenza Vaccine;
   thereby inhibiting the viral infection in the subject.
8. A kit for inhibiting influenza virus in a subject comprising a first container and a second container, wherein
   the first container comprises a primer vaccine comprising a propagation-defective RNA virus vector or self-replicating RNA or non-self-replicating RNA; and the second container comprises a Live Attenuated Influenza Vaccine;
   wherein the primer vaccine and the booster vaccine are formulated for use in a prime-booster vaccination to inhibit influenza virus in a subject; and instructions for using the kit.
9. The combination of embodiment 1, the method of embodiments 2-4, the heterologous prime-boost vaccine for use according to anyone of embodiments 5-7 or the kit according to embodiment 8, wherein the propagation-defective RNA virus vector or self-replicating RNA or non-self-replicating RNA encodes influenza antigens.
10. The combination of embodiment 1, the method of embodiments 2-4, the heterologous prime-boost vaccine for use according to anyone of embodiments 5-7 or the kit according to embodiment 8, wherein influenza antigens include HA and/or NA.
11. The combination of embodiment 1, the method of embodiments 2-4, the heterologous prime-boost vaccine for use according to anyone of embodiments 5-7 or the kit according to embodiment 8, wherein the propagation-defective RNA virus vector or self-replicating RNA or non-self-replicating RNA is propagation-defective vesicular stomatitis virus (VSV) vector.
12. The combination of embodiment 1, the method of embodiments 2-4, the heterologous prime-boost vaccine for use according to anyone of embodiments 5-7 or the kit according to embodiment 8, wherein the self-replicating RNA or non-self-replicating RNA is derived from a pestivirus replicon.
13. The combination of embodiment 1, the method of embodiments 2-4, the heterologous prime-boost vaccine for use according to anyone of embodiments 5-7 or the kit according to embodiment 8, wherein the Live Attenuated Influenza Vaccine comprises at least one mutation in the nonstructural 1 (NS1) protein and/or PA-X protein.
14. The combination of embodiment 1, the method of embodiments 2-4, the heterologous prime-boost vaccine for use according to anyone of embodiments 5-7 or the kit according to embodiment 8, wherein the at least one mutation in the nonstructural 1 (NS1) protein and/or PA-X protein causes a loss of function of the NS1 and/or PA-X protein.
15. The combination of embodiment 1, the method of embodiments 2-4, the heterologous prime-boost vaccine for use according to anyone of embodiments 5-7 or the kit according to embodiment 8, wherein the at least one mutation comprises an amino acid substitution, insertion or deletion.
16. The method of embodiments 2-4 or the heterologous prime-boost vaccine for use according to anyone of embodiments 5-7, wherein the administering a second immunogenic composition by the respiratory route is administered intranasally.
17. The combination of embodiment 1, the method of embodiments 2-4, the heterologous prime-boost vaccine for use according to anyone of embodiments 5-7 or the kit according to embodiment 8, wherein no adjuvant is used.
18. The combination of embodiment 1, the method of embodiments 2-4, the heterologous prime-boost vaccine for use according to anyone of embodiments 5-7 or the kit according to embodiment 8, wherein the influenza virus strain is selected from the group consisting of: H5N1, H9N2, H7N7, H2N2 and H1N1, and H5H2.
19. The combination of embodiment 1, the method of embodiments 2-4, the heterologous prime-boost vaccine for use according to anyone of embodiments 5-7 or the kit according to embodiment 8, wherein the primer vaccine comprises sequence ID NO:1 or 4.
20. The combination of embodiment 1, the method of embodiments 2-4, the heterologous prime-boost vaccine for use according to anyone of embodiments 5-7 or the kit according to embodiment 8, wherein the booster vaccine comprises sequence ID NO: 2, 3, 5 or 6.
21. The combination of embodiment 1, the method of embodiments 2-4, the heterologous prime-boost vaccine for use according to anyone of embodiments 5-7 or the kit according to embodiment 8, wherein the immune response is a CD4+ T cell response.
22. The combination of embodiment 1, the method of embodiments 2-4, the heterologous prime-boost vaccine for use according to anyone of embodiments 5-7 or the kit according to embodiment 8, wherein the immune response is a CD8+ T cell response.
23. The method of embodiments 2-4 or the heterologous prime-boost vaccine for use according to anyone of embodiments 5-7 wherein the booster vaccine is administered to the subject about 1 week, or 2 weeks, or 3 weeks, or 4 weeks, or 5 weeks, or 6 weeks, or 7 weeks, or 8 weeks, or 9 weeks, or 16 weeks, or 20 weeks, or 24 weeks, or 28 weeks, or 32 weeks after administration of the primer vaccine.
24. The method of embodiments 2-4 or the heterologous prime-boost vaccine for use according to anyone of embodiments 5-7, wherein the booster dose is about 10E5 infectious units per animal, preferably the dose is less than 10E5, preferably less than 20E5 unit per animal, or preferably less than 30E5.
25. The method of embodiments 2-4 or the heterologous prime-boost vaccine for use according to anyone of embodiments 5-7, comprising further administering to the mammal a second boosting immunogenic composition.
26. The method of embodiments 2-4 or the heterologous prime-boost vaccine for use according to anyone of embodiments 5-7, wherein the subject or mammal is a human or animal, wherein the animal is a pig, horses, mink, or fox.
27. The method of embodiments 2-4 or the heterologous prime-boost vaccine for use according to anyone of embodiments 5-7, wherein the primer vaccine is administered parentally.
28. The method of embodiments 2-4 or the heterologous prime-boost vaccine for use according to anyone of embodiments 5-7, wherein the booster vaccine is administered intranasally.
29. The method of embodiments 2-4 or the heterologous prime-boost vaccine for use according to anyone of embodiments 5-7, wherein the composition triggers a local (mucosal) and systemic immune response.
30. The method of embodiments 2-4 or the heterologous prime-boost vaccine for use according to anyone of embodiments 5-7, wherein the composition prevents the shedding of the Live Attenuated Influenza Vaccine.
31. The method of embodiments 2-4 or the heterologous prime-boost vaccine for use according to anyone of embodiments 5-7, wherein the composition prevents the transmission of the Live Attenuated Influenza Virus.

## Claims

1. A combination of a priming composition and a boosting composition for priming and boosting an immune response in a subject comprising (1) a priming composition comprised of a propagation-defective RNA virus vector or self-replicating RNA or non-self-replicating RNA, and (2) a boosting composition comprising a Live Attenuated Influenza Vaccine and, whereby the immune response resulting from administration of the priming composition to the subject is capable of being boosted.

2. A method for vaccinating a subject comprising a first step of administering an effective amount of a propagation-defective RNA virus vector or self-replicating RNA or non-self-replicating RNA and a second step of administering an effective amount of a Live Attenuated Influenza Vaccine to the subject.

3. Use of a prime-boost vaccination to inhibit influenza Virus infection in a subject, wherein the prime-boost vaccination comprises administering a primer vaccine and a booster vaccine to the subject, wherein:
the primer vaccine comprises propagation-defective RNA virus vector or self-replicating RNA or non-self-replicating RNA; and the booster vaccine comprises a Live Attenuated Influenza Vaccine; thereby inhibiting the viral infection in the subject.

4. A heterologous prime-boost vaccine for use in immunization against influenza disease in a mammal comprising:
administering a primer vaccine and a booster vaccine to the subject, wherein:
the primer vaccine comprises a propagation-defective RNA virus vector or self-replicating RNA or non-self-replicating RNA; and
the booster vaccine comprises a Live Attenuated Influenza Vaccine;
thereby inhibiting the viral infection in the subject.

5. A kit for inhibiting influenza virus in a subject comprising a first container and
a second container, wherein
the first container comprises a primer vaccine comprising a propagation-defective RNA virus vector or self-replicating RNA or non-self-replicating RNA; and the second container comprises a Live Attenuated Influenza Vaccine;
wherein the primer vaccine and the booster vaccine are formulated for use in a prime-booster vaccination to inhibit influenza virus in a subject; and instructions for using the kit.

6. The combination of claim 1, the method of claim 2, the heterologous prime-boost vaccine for use according to anyone of claims 3-4 or the kit according to claim 5, wherein the propagation-defective RNA virus vector or self-replicating RNA or non-self-replicating RNA encodes influenza antigens, preferably wherein influenza antigens include HA and/or NA.

7. The combination of claim 1, the method of claim 2, the heterologous prime-boost vaccine for use according to anyone of claims 3-4 or the kit according to claim 5, wherein the propagation-defective RNA virus vector or self-replicating RNA or non-self-replicating RNA is propagation-defective vesicular stomatitis virus (VSV) vector.

8. The combination of claim 1, the method of claim 2, the heterologous prime-boost vaccine for use according to anyone of claims 3-4 or the kit according to claim 5, wherein the Live Attenuated Influenza Vaccine comprises at least one mutation in the nonstructural 1 (NS1) protein and/or PA-X protein, preferably wherein the at least one mutation in the nonstructural 1 (NS1) protein and/or PA-X protein causes a loss of function of the NS1 and/or PA-X protein.

9. The method of claim 2 or the heterologous prime-boost vaccine for use according to anyone of claims 3-4, wherein the administering a second immunogenic composition by the respiratory route is administered intranasally.

10. The combination of claim 1, the method of claim 2, the heterologous prime-boost vaccine for use according to anyone of claims 3-4 or the kit according to claim 5, wherein no adjuvant is used.

11. The combination of claim 1, the method of claim 2, the heterologous prime-boost vaccine for use according to anyone of claims 3-4 or the kit according to claim 5, wherein the primer vaccine comprises sequence ID NO:1 or 4.

12. The combination of claim 1, the method of claim 2, the heterologous prime-boost vaccine for use according to anyone of claims 3-4 or the kit according to claim 5, wherein the booster vaccine comprises sequence ID NO: 2, 3, 5 or 6.

13. The method of claim 2 or the heterologous prime-boost vaccine for use according to anyone of claims 3-4, wherein the primer vaccine is administered parentally.

14. The method of claim 2 or the heterologous prime-boost vaccine for use according to anyone of claims 3-4, wherein the booster vaccine is administered intranasally.

15. The method of claim 2 or the heterologous prime-boost vaccine for use according to anyone of claims 3-4, wherein the composition prevents the shedding of the Live Attenuated Influenza Vaccine and/or prevents the transmission of the Live Attenuated Influenza Virus.
